(19) **Europäisches Patentamt / European Patent Office / Office européen des brevets**

(11) **EP 4 640 700 A1**

(12) **EUROPEAN PATENT APPLICATION**
published in accordance with Art. 153(4) EPC

(43) Date of publication:
**29.10.2025 Bulletin 2025/44**

(21) Application number: **23905894.4**

(22) Date of filing: **18.12.2023**

(51) International Patent Classification (IPC):
**C07K 16/00** $^{(2006.01)}$

(52) Cooperative Patent Classification (CPC):
**C07K 16/00**

(86) International application number:
**PCT/CN2023/139544**

(87) International publication number:
**WO 2024/131731 (27.06.2024 Gazette 2024/26)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **19.12.2022 CN 202211633462**

(71) Applicant: **Harbour Biomed (Shanghai) Co., Ltd Shanghai 201203 (CN)**

(72) Inventors:
• **HE, Yun**
 **Shanghai 201203 (CN)**
• **YANG, Yunxing**
 **Shanghai 201203 (CN)**

• **WU, Xiaodong**
 **Shanghai 201203 (CN)**
• **CHEN, Fei**
 **Shanghai 201203 (CN)**
• **XIE, Jinli**
 **Shanghai 201203 (CN)**
• **WANG, Ling**
 **Shanghai 201203 (CN)**

(74) Representative: **Maiwald GmbH**
**Elisenhof**
**Elisenstraße 3**
**80335 München (DE)**

Remarks:
The complete document including Reference Table(s) and the Sequence Listing(s) can be downloaded from the EPO website

(54) **"KAPPA/LAMBDA" FAB-FAB SERIES-CONNECTION MULTI-SPECIFIC BINDING PROTEIN, PREPARATION THEREOF, AND USE THEREOF**

(57) Provided are an antigen-binding protein referred to as a "kappa/lambda" Fab-Fab series connection structure, preparation thereof, and use thereof, as well as a universal technical solution for preparing any two conventional IgG antibodies into a bispecific antigen-binding protein molecule having the structure.

**Description**

**Cross-Reference to Related Applications**

This application claims the benefits of Chinese Patent Application No. 202211633462.8 filed on December 19, 2022, the entire contents of which are incorporated herein by reference.

**Field of the Disclosure**

The present disclosure relates to the field of biomedicine, and in particular, to a multispecific binding protein of a "$\kappa/\lambda$" Fab-Fab series-connection structure, as well as preparation and use thereof.

**Background**

An antibody is an immunoglobulin (Immunoglobulin, Ig) produced by B cells in the immune system under the stimulation of antigens, which is capable of specifically binding to the corresponding antigen. The basic structure of antibodies in most species is in the form of a "Y"-shaped tetramer, which contains two identical heavy chains (H chains) and two identical light chains (L chains), also known as "H2L2". The heavy chain comprises a heavy chain variable region (VH) proximal to the N-terminus and a heavy chain constant region (CH) proximal to the C-terminus; and the light chain comprises a light chain variable region (VL) proximal to the N-terminus and a light chain constant region (CL) proximal to the C-terminus. The heavy chain constant region of IgG antibodies has three domains, namely CH1, CH2, and CH3, as well as a hinge between CH1 and CH2. The variable region of an antibody is the main portion for its recognition and binding to antigens. An antigen-binding fragment (Fab) is formed by the variable region domains VH and VL together with the constant region domains CH1 and CL of the antibody. CH2 and CH3 constitute the fragment crystallizable (Fc), which is the main portion that exerts the effector functions of antibodies and affects the serum half-life of antibodies.

Bispecific antibodies and multispecific antibodies are a type of artificial antibodies with two or more different specific antigen-binding sites prepared through protein engineering technology based on natural monoclonal antibodies. The structural design of bispecific antibodies is very important. A naturally occurring bivalent H2L2 (IgG) antibody consists of two identical heavy chains and two identical light chains, which contains two identical antigen-binding sites. A bispecific antibody requires the introduction of two different antigen-binding sites through structural design by means of protein engineering technology and other methods. The resulting molecules involve two different heavy chains and two different light chains. Therefore, one of the most significant challenges in the development of bispecific antibodies is how to obtain functional bispecific antibody molecules with the correct chain combination from over 10 different combinations of heavy and light chains, i.e., how to solve the chain-mismatching problem.

Currently, the molecular structures of bispecific antibodies are mainly divided into two categories: antibody fragment structures without Fc, and IgG-like antibody structures containing Fc. These two categories of structures each have their own advantages, drawbacks, and application scenarios. Micromet's BiTE® bispecific antibody structure is a class of single-chain fusion protein structures without Fc. It fuses the variable regions VH and VL of an IgG antibody into a single-chain variable fragment (scFv), and links scFvs recognizing different targets into a single protein polypeptide chain to form a scFv series-connection structure. This BiTE® structure, consisting of only one polypeptide chain, exhibits a simple structure and no chain-mismatching, and due to its small molecular weight, it also demonstrates better tissue penetration. However, its drawbacks are evident. The conversion from the Fab of IgG to scFv often significantly reduces the binding activity, and the scFv structure is often unstable and prone to aggregation. Meanwhile, due to the lack of Fc, its serum half-life is relatively short, making it unsuitable for application scenarios that require a longer half-life. The IgG-like antibody structures containing Fc are mainly classified into symmetric structures and asymmetric structures, and both need to solve the chain-mismatching problem. IgG-like bispecific antibodies with asymmetric structure usually adopt a bivalent bispecific asymmetric "1+1" structure, often containing at least three different polypeptide chains. Thus, it is required to solve the problems of both heavy chain-mismatching and light chain-mismatching, which increases the complexity of the molecule and makes the production of such molecules a greater challenge. IgG-like bispecific antibodies with symmetric structure typically adopt a tetravalent bispecific symmetric "2+2" structure. Due to the potential differences in the structures, orientations, and positions of their antigen-binding domains, these molecules may vary significantly in molecular size and pharmaceutical properties. Nevertheless, the problem of light chain-mismatching still exists in symmetric structures. Currently, multiple technologies have been developed to address the chain-mismatching problem of IgG-like bispecific antibody structures. For example, the knobs-into-holes (KiH) technology may be used to promote the heterodimerization of heavy chains. Alternatively, the "common light chain" or "common heavy chain" technology may be utilized to reduce chain-mismatching. Another example is that AbbVie's DVD-Ig technology platform addresses the light chain mismatching problem by the way of fusing VH and VL, respectively, to Fab.

Although molecules with symmetrical structures are generally easier to produce than those with asymmetrical

structures, they are not suitable for some specific application scenarios. For example, in the bispecific antibody molecules of immune adaptors targeting CD3, the CD3 binding domain usually needs to be in a monovalent form to avoid non-specific activation caused by a bivalence form. Therefore, such molecules usually adopt an antibody fragment structure without Fc (e.g., BiTE®) or an asymmetric IgG-like antibody structure containing Fc (e.g., DuoBody®). However, these structures all involve extensive protein engineering efforts, including the conversion of Fab into scFv or the introduction of multiple mutations to overcome the chain-mismatching problem.

Therefore, there is still an urgent need to develop a universal technology for generating bispecific (multi-specific) antibody molecules that can: prepare any two conventional IgG antibodies into a bivalent bispecific antibody with a "1+1" structure, retaining the Fab structure without introducing an scFv structure through modification; minimize the requirement of protein engineering work to achieve "plug-and-play"; and be easily extended to other multispecific structures.

**Summary of the Disclosure**

To design a general technique for preparing a bispecific antigen-binding protein molecule from any two conventional IgG antibodies, the present disclosure provides an antigen-binding protein with a "$\kappa/\lambda$" Fab-Fab structure, as well as its preparation method and application.

The "$\kappa/\lambda$" Fab-Fab structure possesses the following advantages:

1) Plug-and-play: The antigen-binding domain retains the Fab structure without the need to introduce an engineered scFv structure.
2) Antigen-binding specificity and function: The two Fab structures have a high degree of spatial freedom, which facilitates binding to the antigen.
3) Simple in structure: It has a small molecular weight, and the linker peptide between the antigen-binding domains is optional.
4) Efficient preparation: The main product and by-products can be efficiently separated using protein purification methods suitable for industrial production, thereby obtaining the purified target molecule.
5) Scalability: This structure can be easily extended to other multispecific structures. For example, a "half-life extension module" can be added to regulate the serum half-life.

Correspondingly, provided in one embodiment of the present disclosure is an antigen-binding protein comprising at least two antigen-binding domain Fabs, wherein: the antigen-binding protein comprises an antigen-binding region A and an antigen-binding region B binding to different antigens or different epitopes of a same antigen; both the antigen-binding region A and the antigen-binding region B are Fab structures; one of the antigen-binding regions in the antigen-binding region A and the antigen-binding region B (A or B) comprises an antibody $\kappa$ light chain constant region (C$\kappa$), whereas the other antigen-binding region (B or A) comprises an antibody $\lambda$ light chain constant region (C$\lambda$); the antigen-binding protein does not comprise an Fc dimer; and the antigen-binding protein may comprise a "half-life extension module" (e.g., a serum albumin-binding domain).

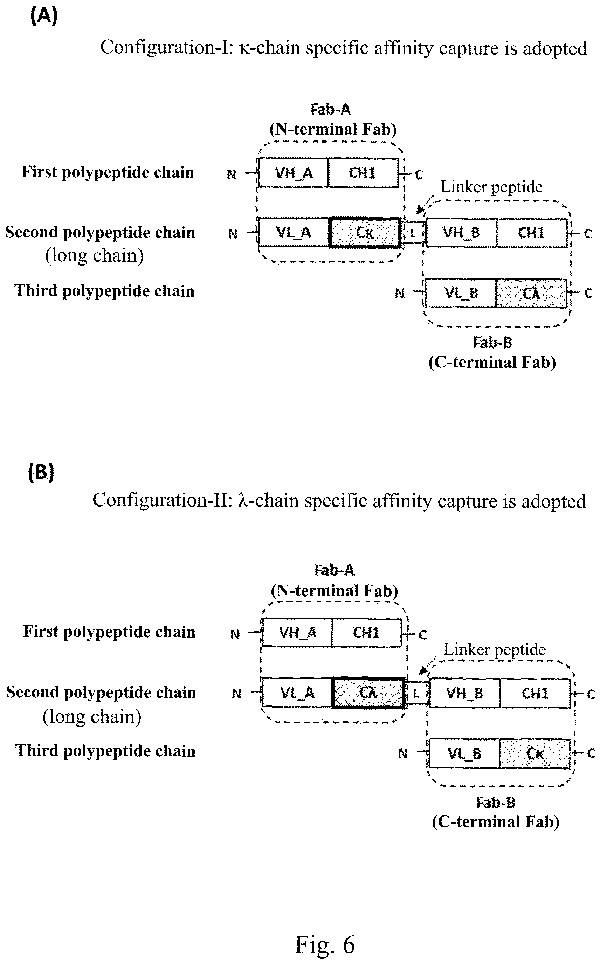

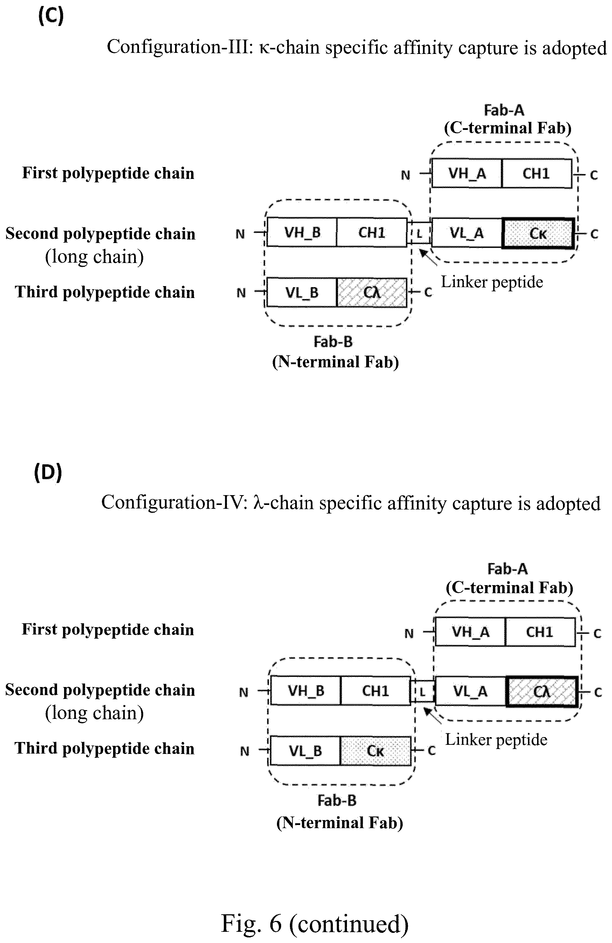

In some embodiments, the antigen-binding protein has a series-connection structure of bispecific antigen-binding fragments composed of three polypeptide chains as shown in Fig. 6 (referred to as the "$\kappa/\lambda$" Fab-Fab structure) or the derived structures thereof.

The antigen-binding protein with the "$\kappa/\lambda$" Fab-Fab structure has three polypeptide chains: a first polypeptide chain, a second polypeptide chain (also referred to as a "long chain"), and a third polypeptide chain. In such embodiments, the first polypeptide chain sequentially comprises VH_A-CH1 from the amino terminus to the carboxyl terminus; the third polypeptide chain sequentially comprises VL_B-CL from the amino terminus to the carboxyl terminus; and the second polypeptide chain sequentially comprises VL_A-CL-L-VH_B-CH1, or VH_B-CH1-L-VL_A-CL from the amino terminus to the carboxyl terminus. In such embodiments, VH_A and V_A are the heavy chain variable region and light chain variable region of antibody A respectively; VH_B and VL_B are the heavy chain variable region and light chain variable region of antibody B respectively; CL is a light chain constant domain; CH1 is the first domain of a heavy chain constant region; and L is a linker peptide. The VL may include a variable region of different light chain types such as V$\kappa$ and/or V$\lambda$; the CL may include a constant region of different light chain types such as C$\kappa$ and/or C$\lambda$; and the CH1 may be a CH1 region of human IgG1, IgG2, or IgG4. The antigen-binding region A (Fab-A) consists of the VH_A-CH1 of the first polypeptide chain and the VL_A-CL of the second polypeptide chain; and the antigen-binding region B (Fab-B) consists of the VL_B-CL of the third polypeptide chain and the VH_B-CH1 of the second polypeptide chain. The linker peptide is optional.

In some embodiments, the "$\kappa/\lambda$ Fab-Fab" structure may have the following characteristics:

(1) The second polypeptide chain (i.e., the "long chain") comprises a light chain variable region of one antibody and a heavy chain variable region of another antibody.
(2) The second polypeptide chain comprises only one light chain constant region.

(3) The third polypeptide chain comprises a light chain variable region of an antibody and a light chain constant region.

(4) The light chain constant regions of the second polypeptide chain and the third polypeptide chain are of different types (Cκ or Cλ). For example, if the light chain constant region of the second polypeptide chain is Cκ, the light chain constant region of the third polypeptide chain is Cλ; alternatively, if the light chain constant region of the second polypeptide chain is Cλ, the light chain constant region of the third polypeptide chain is Cκ.

(5) The type of the light chain constant region of the second polypeptide chain determines which kind of affinity chromatography method is used for purifying the "κ/λ" Fab-Fab protein. For example, if the second polypeptide chain contains a Cκ region, κ-chain specific affinity capture is adapted for purifying the target protein; alternatively, if the second polypeptide chain contains a Cλ region, λ-chain specific affinity capture is adapted for purifying the target protein.

Further, the "κ/λ Fab-Fab" structure may include the following structures:

The antigen-binding protein of "Configuration-I" as shown in Fig. 6(A), comprising three polypeptide chains: a first polypeptide chain comprising VH_A-CH1; a second polypeptide chain comprising VL_A-Cκ-L-VH_B-CH1; and a third polypeptide chain comprising VL_B-Cλ.

The antigen-binding protein of "Configuration-II" as shown in Fig. 6(B) has three polypeptide chains: a first polypeptide chain comprising VH_A-CH1; a second polypeptide chain comprising VL_A-Cλ-L-VH_B-CH1; and a third polypeptide chain comprising VL_B-Cκ.

The antigen-binding protein of "Configuration-III" as shown in Fig. 6(C) has three polypeptide chains: a first polypeptide chain comprising VH_A-CH1; a second polypeptide chain comprising VH _B-CH1-L-VL_A-Cκ; and a third polypeptide chain comprising VL_B-Cλ.

The antigen-binding protein of "Configuration-IV" as shown in Fig. 6(D) has three polypeptide chains: a first polypeptide chain comprising VH_A-CH1; a second polypeptide chain comprising VH_B-CH1-L-VL_A-Cλ; and a third polypeptide chain comprising VL_B-Cκ.

The VL (VL_A and/or VL_B) may comprise a variable region of various light chain types such as Vκ and/or Vλ. The linker peptide L is optional.

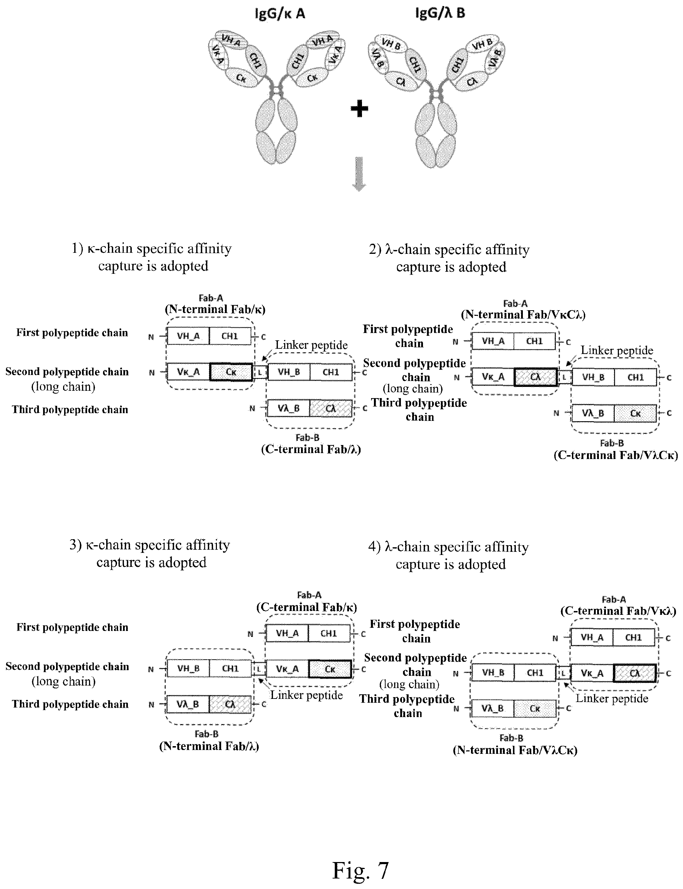

In some particular embodiments, provided is a preparation method for an antigen-binding protein with a "κ/λ" Fab-Fab structure using an IgG antibody A with a κ light chain and an IgG antibody B with a λ light chain. For example, multiple embodiments as shown in Fig. 7:

The antigen-binding protein as shown in Fig. 7(1), comprising three polypeptide chains: a first polypeptide chain comprising VH_A-CH1; a second polypeptide chain comprising Vκ_A-Cκ-L-VH_B-CH1; and a third polypeptide chain comprising Vλ_B-Cλ.

The antigen-binding protein as shown in Fig. 7(2), comprising three polypeptide chains: a first polypeptide chain comprising VH_A-CH1; a second polypeptide chain comprising Vκ_A-Cλ-L-VH_B-CH1; and a third polypeptide chain comprising Vλ_B-Cκ.

The antigen-binding protein as shown in Fig. 7(3), comprising three polypeptide chains: a first polypeptide chain comprising VH_A-CH1; a second polypeptide chain comprising VH_B-CH1-L-Vκ_A-Cκ; and a third polypeptide chain comprising Vλ_B-Cλ.

The antigen-binding protein as shown in Fig. 7(4), comprising three polypeptide chains: a first polypeptide chain comprising VH_A-CH1; a second polypeptide chain comprising VH_B-CH1-L-Vκ_A-Cλ; and a third polypeptide chain comprising Vλ_B-Cκ.

In such embodiments, VH_A and Vκ_A are the heavy chain variable region and the κ light chain variable region of antibody A respectively, VH_B and Vλ_B are the heavy chain variable region and the λ light chain variable region of antibody B respectively; Cκ and Cλ are the constant regions of a κ light chain and a λ light chain respectively; and L is an optional linker peptide.

In the Embodiment (1) and Embodiment (3) as shown in Fig. 7, κ-chain specific affinity capture can be utilized to isolate and purify the antigen-binding protein with a "κ/λ" Fab-Fab structure; and in the Embodiment (2) and Embodiment (4), λ-chain specific affinity capture can be utilized to isolate and purify the antigen-binding protein.

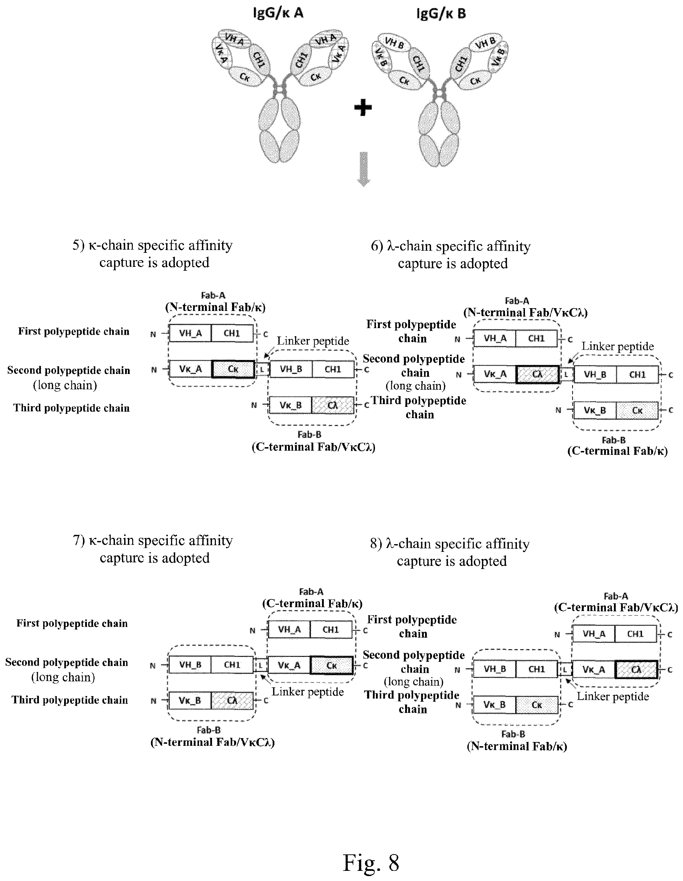

In some particular embodiments, provided is a preparation method for an antigen-binding protein with a "κ/λ" Fab-Fab structure using an IgG antibody A with a κ light chain and an IgG antibody B with a κ light chain. For example, multiple embodiments as shown in Fig. 8:

The antigen-binding protein as shown in Fig. 8(5), comprising three polypeptide chains: a first polypeptide chain comprising VH_A-CH1; a second polypeptide chain comprising Vκ_A-Cκ-L-VH_B-CH1; and a third polypeptide chain comprising Vκ_B-Cλ.

The antigen-binding protein as shown in Fig. 8(6), comprising three polypeptide chains: a first polypeptide chain comprising VH_A-CH1; a second polypeptide chain comprising Vκ_A-Cλ-L-VH_B-CH1; and a third polypeptide chain comprising Vκ_B-Cκ.

The antigen-binding protein as shown in Fig. 8(7), comprising three polypeptide chains: a first polypeptide chain comprising VH_A-CH1; a second polypeptide chain comprising VH_B-CH1-L-Vκ_A-Cκ; and a third polypeptide chain comprising Vκ_B-Cλ.

The antigen-binding protein as shown in Fig. 8(8), comprising three polypeptide chains: a first polypeptide chain comprising VH_A-CH1; a second polypeptide chain comprising VH_B-CH1-L-Vκ_A-Cλ; and a third polypeptide chain comprising Vκ_B-Cκ.

In such embodiments, VH_A and Vκ_A are the heavy chain variable region and the κ light chain variable region of antibody A, respectively, VH_B and Vκ_B are the heavy chain variable region and the κ light chain variable region of antibody B, respectively; Cκ and Cλ are the constant regions of a κ light chain and a λ light chain, respectively; and L is an optional linker peptide.

In the Embodiment (5) and Embodiment (7) as shown in Fig. 8, κ-chain specific affinity capture can be utilized to isolate and purify the antigen-binding protein with a "κ/λ" Fab-Fab structure; and in the Embodiment (6) and Embodiment (8), λ-chain specific affinity-capture can be utilized to isolate and purify the antigen-binding protein.

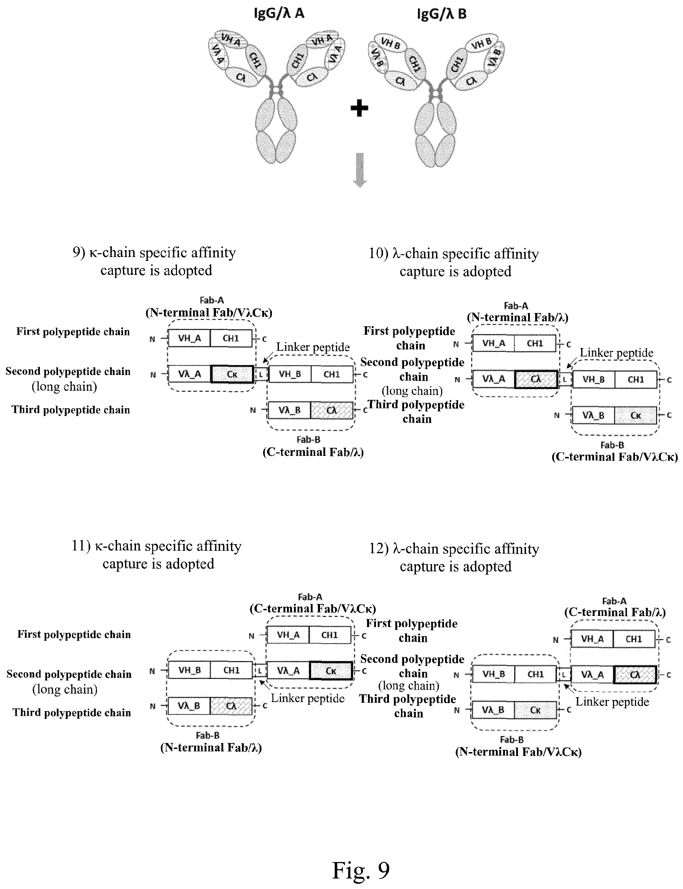

In some particular embodiments, provided is a preparation method for an antigen-binding protein with a "κ/λ" Fab-Fab structure using an IgG antibody A with a λ light chain and an IgG antibody B with a λ light chain. For example, multiple embodiments as shown in Fig. 9:

The antigen-binding protein as shown in Fig. 9(9), comprising three polypeptide chains: a first polypeptide chain comprising VH _A-CH1; a second polypeptide chain comprising Vλ_A-Cκ-L-VH_B-CH1; and a third polypeptide chain comprising Vλ_B-Cλ.

The antigen-binding protein as shown in Fig. 9(10), comprising three polypeptide chains: a first polypeptide chain comprising VH_A-CH1; a second polypeptide chain comprising Vλ_A-Cλ-L-VH_B-CH1; and a third polypeptide chain comprising Vλ_B-Cκ.

The antigen-binding protein as shown in Fig. 9(11), comprising three polypeptide chains: a first polypeptide chain comprising VH_A-CH1; a second polypeptide chain comprising VH_B-CH1-L-Vλ_A-Cκ; and a third polypeptide chain comprising Vλ_B-Cλ.

The antigen-binding protein as shown in Fig. 9(12), comprising three polypeptide chains: a first polypeptide chain comprising VH_A-CH1; a second polypeptide chain comprising VH_B-CH1-L-Vλ_A-Cλ; and a third polypeptide chain comprising Vλ_B-Cκ.

In such embodiments, VH_A and Vλ_A are the heavy chain variable region and the λ light chain variable region of antibody A respectively, VH_B and Vλ_B are the heavy chain variable region and the λ light chain variable region of antibody B respectively; Cκ and Cλ are the constant regions of a κ light chain and a λ light chain respectively; and L is an optional linker peptide.

In the Embodiment (9) and Embodiment (11) as shown in Fig. 9, κ-chain specific affinity capture can be utilized to isolate and purify the antigen-binding protein with a "κ/λ" Fab-Fab structure; and in the Embodiment (10) and Embodiment (12), λ-chain specific affinity capture can be utilized to isolate and purify the antigen-binding protein.

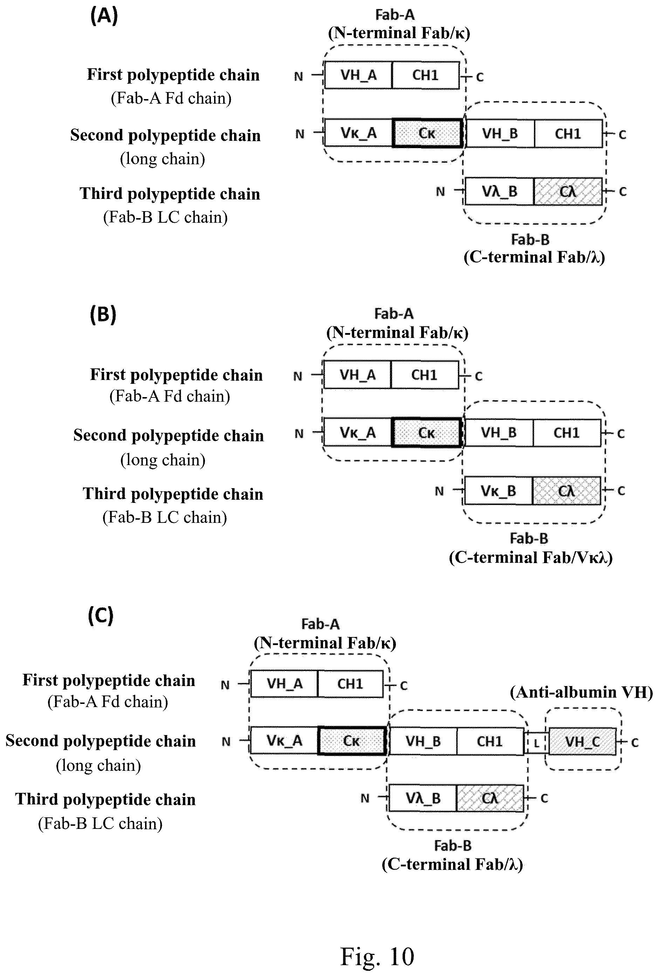

In some specific embodiments, provided is the use of the trispecific antigen-binding protein with the "κ/λ" Fab-Fab structure to expand the application scenarios of the "κ/λ" Fab-Fab structure. In such embodiments, the specificity of the third antigen can be, but is not limited to, a serum albumin-binding domain. As shown in Fig. 10(C), the antigen-binding protein with the "κ/λ" Fab-Fab structure can be fused with the VH domain of an anti-serum albumin antibody to obtain a derivative structure with a half-life extension module.

In some specific embodiments, the antigen-binding protein with the "κ/λ" Fab-Fab structure comprises two antigen-binding regions: antigen-binding region A and antigen-binding region B. Wherein,

The antigen-binding region A comprises a light chain variable region (VL) comprising LCDR1, LCDR2, and LCDR3 having the amino acid sequences as shown in SEQ ID NO: 81, 99, and 117, respectively, and a heavy chain variable region (VH) comprising HCDR1, HCDR2, and HCDR3 having the amino acid sequences as shown in SEQ ID NO: 12, 35, and 58, respectively. And the antigen-binding region B comprises a light chain variable region (VL) comprising LCDR1, LCDR2, and LCDR3 having the amino acid sequences as shown in SEQ ID NO: 82, 100, and 118, respectively, and a heavy chain variable region (VH) comprising HCDR1, HCDR2, and HCDR3 having the amino acid sequences as shown in SEQ ID NO: 13, 36, and 59, respectively. Or,

The antigen-binding region A comprises a light chain variable region (VL) comprising LCDR1, LCDR2, and LCDR3 having the amino acid sequences as shown in SEQ ID NO: 85, 103, and 121, respectively, and a heavy chain variable region (VH) comprising HCDR1, HCDR2, and HCDR3 having the amino acid sequences as shown in SEQ ID NO: 16, 39, and 62, respectively. And the antigen-binding region B comprises a light chain variable region (VL) comprising LCDR1, LCDR2, and LCDR3 having the amino acid sequences as shown in SEQ ID NO: 82, 100, and 118, respectively, and a heavy chain variable region (VH) comprising HCDR1, HCDR2, and HCDR3 having the amino acid sequences as shown in SEQ ID NO: 13, 36, and 59, respectively. Or,

The antigen-binding region A comprises a light chain variable region (VL) comprising LCDR1, LCDR2, and LCDR3 having the amino acid sequences as shown in SEQ ID NO: 83, 101, and 119, respectively, and a heavy chain variable region (VH) comprising HCDR1, HCDR2, and HCDR3 having the amino acid sequences as shown in SEQ ID NO: 14, 37, and 60, respectively. And the antigen-binding region B comprises a light chain variable region (VL) comprising LCDR1, LCDR2, and LCDR3 having the amino acid sequences as shown in SEQ ID NO: 82, 100, and 118, respectively, and a heavy chain variable region (VH) comprising HCDR1, HCDR2, and HCDR3 having the amino acid sequences as shown in SEQ ID NO: 13, 36, and 59, respectively. Or,

The antigen-binding region A comprises a light chain variable region (VL) comprising LCDR1, LCDR2, and LCDR3 having the amino acid sequences as shown in SEQ ID NO: 86, 104, and 122, respectively, and a heavy chain variable region (VH) comprising HCDR1, HCDR2, and HCDR3 having the amino acid sequences as shown in SEQ ID NO: 17, 40, and 63, respectively. And the antigen-binding region B comprises a light chain variable region (VL) comprising LCDR1, LCDR2, and LCDR3 having the amino acid sequences as shown in SEQ ID NO: 82, 100, and 118, respectively, and a heavy chain variable region (VH) comprising HCDR1, HCDR2, and HCDR3 having the amino acid sequences as shown in SEQ ID NO: 13, 36, and 59, respectively. Or,

The antigen-binding region A comprises a light chain variable region (VL) comprising LCDR1, LCDR2, and LCDR3 having the amino acid sequences as shown in SEQ ID NO: 84, 102, and 120, respectively, and a heavy chain variable region (VH) comprising HCDR1, HCDR2, and HCDR3 having the amino acid sequences as shown in SEQ ID NO: 15, 38, and 61, respectively. And the antigen-binding region B comprises a light chain variable region (VL) comprising LCDR1, LCDR2, and LCDR3 having the amino acid sequences as shown in SEQ ID NO: 82, 100, and 118, respectively, and a heavy chain variable region (VH) comprising HCDR1, HCDR2, and HCDR3 having the amino acid sequences as shown in SEQ ID NO: 13, 36, and 59, respectively. Or,

The antigen-binding region A comprises a light chain variable region (VL) comprising LCDR1, LCDR2, and LCDR3 having the amino acid sequences as shown in SEQ ID NO: 81, 99, and 117, respectively, and a heavy chain variable region (VH) comprising HCDR1, HCDR2, and HCDR3 having the amino acid sequences as shown in SEQ ID NO: 12, 35, and 58, respectively. And the antigen-binding region B comprises a light chain variable region (VL) comprising LCDR1, LCDR2, and LCDR3 having the amino acid sequences as shown in SEQ ID NO: 89, 107, and 125, respectively, and a heavy chain variable region (VH) comprising HCDR1, HCDR2, and HCDR3 having the amino acid sequences as shown in SEQ ID NO: 12, 44, and 67, respectively. Or,

The antigen-binding region A comprises a light chain variable region (VL) comprising LCDR1, LCDR2, and LCDR3 having the amino acid sequences as shown in SEQ ID NO: 81, 99, and 117, respectively, and a heavy chain variable region (VH) comprising HCDR1, HCDR2, and HCDR3 having the amino acid sequences as shown in SEQ ID NO: 12, 35, and 58, respectively. And the antigen-binding region B comprises a light chain variable region (VL) comprising LCDR1, LCDR2, and LCDR3 having the amino acid sequences as shown in SEQ ID NO: 89, 107, and 125, respectively, and a heavy chain variable region (VH) comprising HCDR1, HCDR2, and HCDR3 having the amino acid sequences as shown in SEQ ID NO: 12, 45, and 67, respectively. Or,

The antigen-binding region A comprises a light chain variable region (VL) comprising LCDR1, LCDR2, and LCDR3 having the amino acid sequences as shown in SEQ ID NO: 83, 101, and 119, respectively, and a heavy chain variable region (VH) comprising HCDR1, HCDR2, and HCDR3 having the amino acid sequences as shown in SEQ ID NO: 14, 37, and 60, respectively. And the antigen-binding region B comprises a light chain variable region (VL) comprising LCDR1, LCDR2, and LCDR3 having the amino acid sequences as shown in SEQ ID NO: 89, 107, and 125, respectively, and a heavy chain variable region (VH) comprising HCDR1, HCDR2, and HCDR3 having the amino acid sequences as shown in SEQ ID NO: 12, 44, and 67, respectively. Or,

The antigen-binding region A comprises a light chain variable region (VL) comprising LCDR1, LCDR2, and LCDR3 having the amino acid sequences as shown in SEQ ID NO: 83, 101, and 119, respectively, and a heavy chain variable region (VH) comprising HCDR1, HCDR2, and HCDR3 having the amino acid sequences as shown in SEQ ID NO: 14, 37, and 60, respectively. And the antigen-binding region B comprises a light chain variable region (VL) comprising LCDR1, LCDR2, and LCDR3 having the amino acid sequences as shown in SEQ ID NO: 89, 107, and 125, respectively, and a heavy chain variable region (VH) comprising HCDR1, HCDR2, and HCDR3 having the amino acid sequences as shown in SEQ ID NO: 12, 45, and 67, respectively. Or,

The antigen-binding region A comprises a light chain variable region (VL) comprising LCDR1, LCDR2, and LCDR3 having the amino acid sequences as shown in SEQ ID NO: 84, 102, and 120, respectively, and a heavy chain variable region (VH) comprising HCDR1, HCDR2, and HCDR3 having the amino acid sequences as shown in SEQ ID NO: 15, 38, and 61, respectively. And the antigen-binding region B comprises a light chain variable region (VL) comprising LCDR1, LCDR2, and LCDR3 having the amino acid sequences as shown in SEQ ID NO: 88, 106, and 124, respectively, and a heavy chain variable region (VH) comprising HCDR1, HCDR2, and HCDR3 having the amino acid sequences as shown in SEQ ID NO: 19, 42, and 65, respectively. Or,

The antigen-binding region A comprises a light chain variable region (VL) comprising LCDR1, LCDR2, and LCDR3 having the amino acid sequences as shown in SEQ ID NO: 84, 102, and 120, respectively, and a heavy chain variable region (VH) comprising HCDR1, HCDR2, and HCDR3 having the amino acid sequences as shown in SEQ ID NO: 15, 38, and 61, respectively. And the antigen-binding region B comprises a light chain variable region (VL) comprising LCDR1, LCDR2, and LCDR3 having the amino acid sequences as shown in SEQ ID NO: 87, 105, and 123, respectively, and a heavy chain variable region (VH) comprising HCDR1, HCDR2, and HCDR3 having the amino acid sequences as shown in SEQ ID NO: 18, 41, and 64, respectively. Or,

The antigen-binding region A comprises a light chain variable region (VL) comprising LCDR1, LCDR2, and LCDR3 having the amino acid sequences as shown in SEQ ID NO: 81, 99, and 117, respectively, and a heavy chain variable region (VH) comprising HCDR1, HCDR2, and HCDR3 having the amino acid sequences as shown in SEQ ID NO: 12, 35, and 58, respectively. And the antigen-binding region B comprises a light chain variable region (VL) comprising LCDR1, LCDR2, and LCDR3 having the amino acid sequences as shown in SEQ ID NO: 88, 106, and 124, respectively, and a heavy chain variable region (VH) comprising HCDR1, HCDR2, and HCDR3 having the amino acid sequences as shown in SEQ ID NO: 19, 42, and 65, respectively. Or,

The antigen-binding region A comprises a light chain variable region (VL) comprising LCDR1, LCDR2, and LCDR3 having the amino acid sequences as shown in SEQ ID NO: 81, 99, and 117, respectively, and a heavy chain variable region (VH) comprising HCDR1, HCDR2, and HCDR3 having the amino acid sequences as shown in SEQ ID NO: 12, 35, and 58, respectively. And the antigen-binding region B comprises a light chain variable region (VL) comprising LCDR1, LCDR2, and LCDR3 having the amino acid sequences as shown in SEQ ID NO: 87, 105, and 123, respectively, and a heavy chain variable region (VH) comprising HCDR1, HCDR2, and HCDR3 having the amino acid sequences as shown in SEQ ID NO: 18, 41, and 64, respectively. The amino acid sequences of the listed CDRs are defined according to the Chothia numbering system.

In some specific embodiments, the antigen-binding protein with the "κ/λ" Fab-Fab structure comprises two antigen-binding regions: antigen-binding region A and antigen-binding region B. Wherein,

The antigen-binding region A comprises a light chain variable region (VL) comprising the amino acid sequence as shown in SEQ ID NO: 144, and a heavy chain variable region (VH) comprising the amino acid sequence as shown in SEQ ID NO: 131. The antigen-binding region B comprises a light chain variable region (VL) comprising the amino acid sequence as shown in SEQ ID NO: 145, and a heavy chain variable region (VH) comprising the amino acid sequence as shown in SEQ ID NO: 132. Or,

The antigen-binding region A comprises a light chain variable region (VL) comprising the amino acid sequence as shown in SEQ ID NO: 144, and a heavy chain variable region (VH) comprising the amino acid sequence as shown in SEQ ID NO: 131. The antigen-binding region B comprises a light chain variable region (VL) comprising the amino acid sequence as shown in SEQ ID NO: 151, and a heavy chain variable region (VH) comprising the amino acid sequence as shown in SEQ ID NO: 138. Or,

The antigen-binding region A comprises a light chain variable region (VL) comprising the amino acid sequence as shown in SEQ ID NO: 148, and a heavy chain variable region (VH) comprising the amino acid sequence as shown in SEQ ID NO: 135. The antigen-binding region B comprises a light chain variable region (VL) comprising the amino acid sequence as shown in SEQ ID NO: 151, and a heavy chain variable region (VH) comprising the amino acid sequence as shown in SEQ ID NO: 138. Or,

The antigen-binding region A comprises a light chain variable region (VL) comprising the amino acid sequence as shown in SEQ ID NO: 146, and a heavy chain variable region (VH) comprising the amino acid sequence as shown in SEQ ID NO: 133. The antigen-binding region B comprises a light chain variable region (VL) comprising the amino acid sequence as shown in SEQ ID NO: 151, and a heavy chain variable region (VH) comprising the amino acid sequence as shown in SEQ ID NO: 138. Or,

The antigen-binding region A comprises a light chain variable region (VL) comprising the amino acid sequence as shown in SEQ ID NO: 149, and a heavy chain variable region (VH) comprising the amino acid sequence as shown in SEQ ID NO: 136. The antigen-binding region B comprises a light chain variable region (VL) comprising the amino acid sequence as shown in SEQ ID NO: 151, and a heavy chain variable region (VH) comprising the amino acid sequence as shown in SEQ ID NO: 138. Or,

The antigen-binding region A comprises a light chain variable region (VL) comprising the amino acid sequence as shown in SEQ ID NO: 147, and a heavy chain variable region (VH) comprising the amino acid sequence as shown in SEQ ID NO: 134. The antigen-binding region B comprises a light chain variable region (VL) comprising the amino acid sequence as shown in SEQ ID NO: 151, and a heavy chain variable region (VH) comprising the amino acid sequence as shown in SEQ ID NO: 138. Or,

The antigen-binding region A comprises a light chain variable region (VL) comprising the amino acid sequence as shown in SEQ ID NO: 144, and a heavy chain variable region (VH) comprising the amino acid sequence as shown in SEQ ID NO: 131. The antigen-binding region B comprises a light chain variable region (VL) comprising the amino acid sequence as shown in SEQ ID NO: 153, and a heavy chain variable region (VH) comprising the amino acid sequence as shown in SEQ ID NO: 141. Or,

The antigen-binding region A comprises a light chain variable region (VL) comprising the amino acid sequence as shown in SEQ ID NO: 144, and a heavy chain variable region (VH) comprising the amino acid sequence as shown in SEQ ID NO: 131. The antigen-binding region B comprises a light chain variable region (VL) comprising the amino acid sequence as shown in SEQ ID NO: 153, and a heavy chain variable region (VH) comprising the amino acid sequence as shown in SEQ ID NO: 142. Or,

The antigen-binding region A comprises a light chain variable region (VL) comprising the amino acid sequence as shown in SEQ ID NO: 146, and a heavy chain variable region (VH) comprising the amino acid sequence as shown in SEQ ID NO: 133. The antigen-binding region B comprises a light chain variable region (VL) comprising the amino acid sequence as shown in SEQ ID NO: 153, and a heavy chain variable region (VH) comprising the amino acid sequence as shown in SEQ ID NO: 141. Or,

The antigen-binding region A comprises a light chain variable region (VL) comprising the amino acid sequence as shown in SEQ ID NO: 146, and a heavy chain variable region (VH) comprising the amino acid sequence as shown in SEQ ID NO: 133. The antigen-binding region B comprises a light chain variable region (VL) comprising the amino acid sequence as shown in SEQ ID NO: 153, and a heavy chain variable region (VH) comprising the amino acid sequence as shown in SEQ ID NO: 142. Or,

The antigen-binding region A comprises a light chain variable region (VL) comprising the amino acid sequence as shown in SEQ ID NO: 147, and a heavy chain variable region (VH) comprising the amino acid sequence as shown in SEQ ID NO: 134. The antigen-binding region B comprises a light chain variable region (VL) comprising the amino acid sequence as shown in SEQ ID NO: 152, and a heavy chain variable region (VH) comprising the amino acid sequence as shown in SEQ ID NO: 139. Or,

The antigen-binding region A comprises a light chain variable region (VL) comprising the amino acid sequence as shown in SEQ ID NO: 147, and a heavy chain variable region (VH) comprising the amino acid sequence as shown in SEQ ID NO: 134. The antigen-binding region B comprises a light chain variable region (VL) comprising the amino acid sequence as shown in SEQ ID NO: 150, and a heavy chain variable region (VH) comprising the amino acid sequence as shown in SEQ ID NO: 137. Or,

The antigen-binding region A comprises a light chain variable region (VL) comprising the amino acid sequence as shown in SEQ ID NO: 144, and a heavy chain variable region (VH) comprising the amino acid sequence as shown in SEQ ID NO: 131. The antigen-binding region B comprises a light chain variable region (VL) comprising the amino acid sequence as shown in SEQ ID NO: 152, and a heavy chain variable region (VH) comprising the amino acid sequence as shown in SEQ ID NO: 139. Or,

The antigen-binding region A comprises a light chain variable region (VL) comprising the amino acid sequence as shown in SEQ ID NO: 144, and a heavy chain variable region (VH) comprising the amino acid sequence as shown in SEQ ID NO: 131. The antigen-binding region B comprises a light chain variable region (VL) comprising the amino acid sequence as shown in SEQ ID NO: 150, and a heavy chain variable region (VH) comprising the amino acid sequence as shown in SEQ ID NO: 137.

In some specific embodiments, the antigen-binding protein with the "κ/λ" Fab-Fab structure comprises three polypeptide chains. Wherein,

The first polypeptide chain comprises the amino acid sequence as shown in SEQ ID NO: 177; the second polypeptide chain comprises the amino acid sequence as shown in SEQ ID NO: 178; and the third polypeptide chain comprises the amino acid sequence as shown in SEQ ID NO: 168. Or,

The first polypeptide chain comprises the amino acid sequence as shown in SEQ ID NO: 177; the second polypeptide chain comprises the amino acid sequence as shown in SEQ ID NO: 187; and the third polypeptide chain comprises the amino acid sequence as shown in SEQ ID NO: 174. Or,

The first polypeptide chain comprises the amino acid sequence as shown in SEQ ID NO: 179; the second polypeptide chain comprises the amino acid sequence as shown in SEQ ID NO: 180; and the third polypeptide chain comprises the amino acid sequence as shown in SEQ ID NO: 174. Or,

The first polypeptide chain comprises the amino acid sequence as shown in SEQ ID NO: 181; the second polypeptide chain comprises the amino acid sequence as shown in SEQ ID NO: 182; and the third polypeptide chain comprises the amino acid sequence as shown in SEQ ID NO: 174. Or,

The first polypeptide chain comprises the amino acid sequence as shown in SEQ ID NO: 183; the second polypeptide chain comprises the amino acid sequence as shown in SEQ ID NO: 184; and the third polypeptide chain comprises the amino acid sequence as shown in SEQ ID NO: 174. Or,

The first polypeptide chain comprises the amino acid sequence as shown in SEQ ID NO: 185; the second polypeptide chain comprises the amino acid sequence as shown in SEQ ID NO: 186; and the third polypeptide chain comprises the amino acid sequence as shown in SEQ ID NO: 174. Or,

The first polypeptide chain comprises the amino acid sequence as shown in SEQ ID NO: 177; the second polypeptide chain comprises the amino acid sequence as shown in SEQ ID NO: 188; and the third polypeptide chain comprises the amino acid sequence as shown in SEQ ID NO: 176. Or,

The first polypeptide chain comprises the amino acid sequence as shown in SEQ ID NO: 177; the second polypeptide chain comprises the amino acid sequence as shown in SEQ ID NO: 189; and the third polypeptide chain comprises the amino acid sequence as shown in SEQ ID NO: 176. Or,

The first polypeptide chain comprises the amino acid sequence as shown in SEQ ID NO: 181; the second polypeptide chain comprises the amino acid sequence as shown in SEQ ID NO: 190; and the third polypeptide chain comprises the amino acid sequence as shown in SEQ ID NO: 176. Or,

The first polypeptide chain comprises the amino acid sequence as shown in SEQ ID NO: 181; the second polypeptide chain comprises the amino acid sequence as shown in SEQ ID NO: 191; and the third polypeptide chain comprises the amino acid sequence as shown in SEQ ID NO: 176. Or,

The first polypeptide chain comprises the amino acid sequence as shown in SEQ ID NO: 185; the second polypeptide chain comprises the amino acid sequence as shown in SEQ ID NO: 193; and the third polypeptide chain comprises the amino acid sequence as shown in SEQ ID NO: 192. Or,

The first polypeptide chain comprises the amino acid sequence as shown in SEQ ID NO: 185; the second polypeptide chain comprises the amino acid sequence as shown in SEQ ID NO: 194; and the third polypeptide chain comprises the amino acid sequence as shown in SEQ ID NO: 173. Or,

The first polypeptide chain comprises the amino acid sequence as shown in SEQ ID NO: 177; the second polypeptide chain comprises the amino acid sequence as shown in SEQ ID NO: 195; and the third polypeptide chain comprises the amino acid sequence as shown in SEQ ID NO: 192. Or,

The first polypeptide chain comprises the amino acid sequence as shown in SEQ ID NO: 177; the second polypeptide chain comprises the amino acid sequence as shown in SEQ ID NO: 196; and the third polypeptide chain comprises the amino acid sequence as shown in SEQ ID NO: 173.

In this application, the CDRs mentioned may all include the implementations with mutations based on the defined sequences. The mutation is an insertion, deletion or substitution of 3, 2 or 1 amino acids based on the amino acid sequence of the VH CDR1, VH CDR2, VH CDR3, VL CDR1, VL CDR2, and VL CDR3, respectively. In this application, in an expression similar to "having an insertion, deletion, or substitution of 3, 2, or 1 amino acids", "an amino acid mutation" refers to an amino acid mutation, as compared to the original amino acid sequence, in the sequence of the variant, including insertions, deletions, or substitutions of amino acid(s) occurred based on the original amino acid sequence. In an exemplary embodiment, the mutations of CDRs can involve mutations of 3, 2, or 1 amino acids; optionally, the same or different numbers of amino acid residues can be selected for mutations among these CDRs. For example, one amino acid in CDR1 can be mutated, while no amino acid is mutated in CDR2 and CDR3.

In this application, the VH, VL, or the polypeptide chains may all include the implementations with mutations based on the defined sequences. The mutation refers to a deletion, a substitution, or an addition of one or more amino acid residues in the defined amino acid sequence, wherein the mutated amino acid sequence has at least 85%, preferably at least 90%, more preferably at least 95%, and most preferably at least 99% sequence identity with the defined amino acid sequence, and maintains or improves the binding activity of the antibody, its antigen-binding fragment, or binding protein.

In the second aspect of the present disclosure, provided is an isolated nucleic acid encoding the binding protein as described in the first aspect of the present disclosure.

In the third aspect of the present disclosure, provided is a recombinant expression vector comprising the isolated nucleic acid as described in the second aspect of the present disclosure. Preferably, the expression vector comprises a eukaryotic cell expression vector and/or a prokaryotic cell expression vector.

In the fourth aspect of the present disclosure, provided is a host cell comprising the isolated nucleic acid as described in the second aspect of the present disclosure or the recombinant expression vector as described in the third aspect of the present disclosure. Preferably, the host cell is a prokaryotic cell and/or a eukaryotic cell, preferably, the prokaryotic cell is an Escherichia coli (E. coli) cell, such as TG1 or BL21 cell, and preferably, the eukaryotic cell is an HEK293 cell or a CHO cell.

In the fifth aspect of the present disclosure, provided is a preparation method for a binding protein comprising culturing the host cell as described in the fourth aspect of the present disclosure and obtaining the binding protein from the culture.

In the sixth aspect of the present disclosure, provided is a pharmaceutical composition comprising the binding protein as described in the first aspect of the present disclosure and a pharmaceutically acceptable carrier. Preferably, the pharmaceutical composition further comprises one or more additional anti-tumor agents, such as anti-tumor drugs, as active ingredients.

In the seventh aspect of the present disclosure, provided is the use of the binding protein as described in the first aspect of the present disclosure and/or the pharmaceutical composition as described in the fifth aspect of the present disclosure in the manufacture of a medicament for the diagnosis, prevention and/or treatment of a tumor.

In the eighth aspect of the present disclosure, provided is a method for in vitro or in vivo detection of a specific antigen, comprising using the binding protein as described in the first aspect of the present disclosure and/or the pharmaceutical composition as described in the fifth aspect of the present disclosure for detection.

Without countering the common knowledge in the field, the above preferred conditions can be arbitrarily combined to obtain each preferred example of the present invention.

All the reagents and raw materials used in the present disclosure are commercially available.

The present disclosure includes the following numbered embodiments.

Embodiment 1. An antigen-binding protein comprising an antigen-binding region A and an antigen-binding region B binding to different antigens or different epitopes of a same antigen, wherein the antigen-binding protein comprises three polypeptide chains: a first polypeptide chain, a second polypeptide chain, and a third polypeptide chain, and wherein:

the first polypeptide chain comprises VH_A-CH1 from the amino terminus (N-terminus) to the carboxyl terminus (C-terminus);
the second polypeptide chain comprises VL_A-CL-L-VH_B-CH1 or VH_B-CH1-L-VL_A-CL from the N-terminus to the C-terminus, and
the third polypeptide chain comprises VL_B-CL from the N-terminus to the C-terminus,
and wherein: VH_A and VL_A are respectively the heavy chain variable region and the light chain variable region derived from antibody A, which constitute the antigen-binding region A; VH_B and VL_B are respectively the heavy chain variable region and the light chain variable region derived from antibody B, which constitute the antigen-binding region B; CL is a light chain constant region domain, wherein the CLs in the second polypeptide chain and the third polypeptide chain comprise constant regions derived from different light chain types; CH1 is the first domain of a heavy chain constant region, where the CH1s in the first polypeptide chain and the second polypeptide chain may be the same or different; and L is an optional linker peptide.

Embodiment 2. The antigen-binding protein according to Embodiment 1, wherein the CLs in the second polypeptide chain and the third polypeptide chain each independently comprise a constant region derived from a κ light chain or a λ light chain (Cκ or Cλ).

Embodiment 3. The antigen-binding protein according to Embodiment 2, wherein the CL in the second polypeptide chain comprises the constant region Cκ derived from a κ light chain, and the CL in the third polypeptide chain comprises the constant region Cλ derived from a λ light chain.

Embodiment 4. The antigen-binding protein according to Embodiment 2, wherein the CL in the second polypeptide chain comprises the constant region Cλ derived from a λ light chain, and the CL in the third polypeptide chain comprises the constant region Cκ derived from a κ light chain.

Embodiment 5. The antigen-binding protein according to any one of Embodiment 1-4, wherein the VL_A and VL_B each independently comprise a variable region derived from a κ light chain or a λ light chain (Vκ or Vλ).

Embodiment 6. The antigen-binding protein according to Embodiment 5, wherein the VL_A comprises a variable region which is derived from the same type of a light chain with the light chain constant region comprised in the CL of the second polypeptide chain, and/or the VL_B comprises a variable region which is derived from the same type of light chain with the light chain constant region comprised in the CL of the third polypeptide chain.

Embodiment 7. The antigen-binding protein according to any one of Embodiments 1-6, wherein the CH1s each independently comprise a CH1 region derived from an IgG isotype, such as selected from the CH1 region of IgG1, IgG2, and IgG4, for example, selected from the CH1 region of human IgG1, IgG2, and IgG4.

Embodiment 8. The antigen-binding protein according to any one of Embodiments 1-7, wherein:

(a) the first polypeptide chain comprises VH_A-CH1 from the N-terminus to the C-terminus, the second polypeptide chain comprises VL_A-Cκ-L-VH_B-CH1 from the N-terminus to the C-terminus, and the third polypeptide chain comprises VL_B-Cλ from the N-terminus to the C-terminus;
(b) the first polypeptide chain comprises VH_A-CH1 from the N-terminus to the C-terminus, the second polypeptide chain comprises VL_A-Cλ-L-VH_B-CH1 from the N-terminus to the C-terminus, and the third polypeptide chain comprises VL_B-Cκ from the N-terminus to the C-terminus;
(c) the first polypeptide chain comprises VH_A-CH1 from the N-terminus to the C-terminus, the second polypeptide chain comprises VH_B-CH1-L-VL_A-Cκ from the N-terminus to the C-terminus, and the third polypeptide chain comprises VL_B-Cλ from the N-terminus to the C-terminus;
(d) the first polypeptide chain comprises VH_A-CH1 from the N-terminus to the C-terminus, the second polypeptide chain comprises VH_B-CH1-L-VL_A-Cλ from the N-terminus to the C-terminus, and the third polypeptide chain comprises VL_B-Cκ from the N-terminus to the C-terminus;

wherein, the VL_A and VL_B each independently comprise a variable region derived from a κ light chain or a λ light chain (Vκ or Vλ), and L is an optional linker peptide.

Embodiment 9. The antigen-binding protein according to any one of Embodiments 1-8, wherein:

(a) the first polypeptide chain comprises VH_A-CH1 from the N-terminus to the C-terminus, the second polypeptide chain comprises Vκ_A-Cκ-L-VH_B-CH1 from the N-terminus to the C-terminus, and the third polypeptide chain comprises Vλ_B-Cλ from the N-terminus to the C-terminus;

(b) the first polypeptide chain comprises VH_A-CH1 from the N-terminus to the C-terminus, the second polypeptide chain comprises Vκ_A-Cλ-L-VH_B-CH1 from the N-terminus to the C-terminus, and the third polypeptide chain comprises Vλ_B-Cκ from the N-terminus to the C-terminus;

(c) the first polypeptide chain comprises VH_A-CH1 from the N-terminus to the C-terminus, the second polypeptide chain comprises VH_B-CH1-L-Vκ_A-Cκ from the N-terminus to the C-terminus, and the third polypeptide chain comprises Vλ_B-Cλ from the N-terminus to the C-terminus;

(d) the first polypeptide chain comprises VH_A-CH1 from the N-terminus to the C-terminus, the second polypeptide chain comprises VH_B-CH1-L-Vκ_A-Cλ from the N-terminus to the C-terminus, and the third polypeptide chain comprises Vλ_B-Cκ from the N-terminus to the C-terminus;

(e) the first polypeptide chain comprises VH_A-CH1 from the N-terminus to the C-terminus, the second polypeptide chain comprises Vκ_A-Cκ-L-VH_B-CH1 from the N-terminus to the C-terminus, and the third polypeptide chain comprises Vκ_B-Cλ from the N-terminus to the C-terminus;

(f) the first polypeptide chain comprises VH_A-CH1 from the N-terminus to the C-terminus, the second polypeptide chain comprises Vκ_A-Cλ-L-VH_B-CH1 from the N-terminus to the C-terminus, and the third polypeptide chain comprises Vκ_B-Cκ from the N-terminus to the C-terminus;

(g) the first polypeptide chain comprises VH_A-CH1 from the N-terminus to the C-terminus, the second polypeptide chain comprises VH _B-CH1-L-Vκ_A-Cκ from the N-terminus to the C-terminus, and the third polypeptide chain comprises Vκ_B-Cλ from the N-terminus to the C-terminus;

(h) the first polypeptide chain comprises VH_A-CH1 from the N-terminus to the C-terminus, the second polypeptide chain comprises VH _B-CH1-L-Vκ_A-Cλ from the N-terminus to the C-terminus, and the third polypeptide chain comprises Vκ_B-Cκ from the N-terminus to the C-terminus;

(i) the first polypeptide chain comprises VH_A-CH1 from the N-terminus to the C-terminus, the second polypeptide chain comprises Vλ_A-Cκ-L-VH_B-CH1 from the N-terminus to the C-terminus, and the third polypeptide chain comprises Vλ_B-Cλ from the N-terminus to the C-terminus;

(j) the first polypeptide chain comprises VH_A-CH1 from the N-terminus to the C-terminus, the second polypeptide chain comprises Vλ_A-Cλ-L-VH_B-CH1 from the N-terminus to the C-terminus, and the third polypeptide chain comprises Vλ_B-Cκ from the N-terminus to the C-terminus;

(k) the first polypeptide chain comprises VH_A-CH1 from the N-terminus to the C-terminus, the second polypeptide chain comprises VH _B-CH1-L-Vλ_A-Cκ from the N-terminus to the C-terminus, and the third polypeptide chain comprises Vλ_B-Cλ from the N-terminus to the C-terminus;

(l) the first polypeptide chain comprises VH_A-CH1 from the N-terminus to the C-terminus, the second polypeptide chain comprises VH_B-CH1-L-Vλ_A-Cλ from the N-terminus to the C-terminus, and the third polypeptide chain comprises Vλ_B-Cκ from the N-terminus to the C-terminus.

Embodiment 10. The antigen-binding protein according to any one of Embodiments 1-9, wherein the linker peptide comprises (G4S)n, where the n is an integer selected from 1-5.

Embodiment 11. The antigen-binding protein according to any one of Embodiments 1-10, wherein the antigen-binding region A and the antigen-binding region B each independently bind to an antigen selected from the group consisting of: a tumor-associated antigen, an immune cell antigen such as a T cell antigen and/or an NK cell antigen, and an immune checkpoint molecule.

Embodiment 12. The antigen-binding protein according to Embodiment 11, wherein the antigen-binding region A and the antigen-binding region B bind to two different tumor-associated antigens, or different epitopes on a same tumor-associated antigen.

Embodiment 13. The antigen-binding protein according to Embodiment 11, wherein one of the antigen-binding region A and the antigen-binding region B binds to a tumor-associated antigen, and the other binds to an immune cell antigen, such as a T cell antigen and/or an NK cell antigen.

Embodiment 14. The antigen-binding protein according to any one of Embodiments 11-13, wherein the tumor-associated antigen is selected from CD19, BCMA, TSHR, CD171, CS-1, CLL-1, GD3, Tn Ag, FLT3, CD38, CD123, CD44v6, B7H3, B7H4, KIT, IL-13Ra2, IL-11Ra, PSCA, PSMA, PRSS21, VEGFR2, LewisY, CD24, PDGFR-beta, SSEA-4, MUC1, EGFR, NCAM, CAIX, LMP2, EphA2, fucosyl GM1, sLe, GM3, TGS5, HMWMAA, GD2, FOLR1, FOLR2, TEM1/CD248, TEM7R, CLDN6, CLDN18.2, GPRC5D, CXORF61, CD97, CD179a, ALK, polysialic acid, PLAC1, GloboH, NY-BR-1, UPK2, HAVCR1, ADRB3, PANX3, GPR20, LY6K, OR51E2, TAARP, WT1, ETV6-AML, SPA17, XAGE1, Tie 2, MAD-CT-1, MAD-CT-2, FOSL1, hTERT, ML-IAP, ERG, NA17, PAX3, AR, cyclin B1, MYCN, RhoC, CYP1B1, BORIS, SART3, PAX5, OY-TES1, LCK, AKAP-4, SSX2, CD79a, CD79b, CD72, LAIR1, FCAR, LILRA2, CD300LF, CLEC12A, BST2, EMR2, LY75, GPC3, FCRL5, IGLL1, CD20, CD30, HER2, ROR1, FLT3, TAAG72, CD22, CD33, GD2, gp100Tn, FAP, tyrosinase, EPCAM, CEA, IGF-1R, EphB2, mesothelin, cadherin 17, CD32b, EGFRvIII, GPNMB, GPR64, HER3, LRP6, LYPD8, NKG2D, SLC34A2, SLC39A6, SLITRK6, GUCY2C, or TACSTD2; preferably B7H3, B7H4, PSMA, CLDN18.2, GPC3, HER2, ROR1, CD22, EPCAM, mesothelin, EGFRvIII or TACSTD2; preferably, the tumor-associated antigen is selected from B7H4, PSMA, ROR1 and BCMA.

Embodiment 15. The antigen-binding protein according to any one of Embodiments 11-13, wherein the immune cell antigen is selected from T cell receptor (TCR), CD3, CD28, 4-1BB, OX40, GITR, CD27, ICOS, CD2, CD40, CD226, CD16, NKp30, NKp44, NKp46, NKG2D and 2B4, preferably, the immune cell antigen is CD3, CD16, or CD137.

Embodiment 16. The antigen-binding protein according to any one of Embodiments 1-15, further comprising an antigen-binding region C binding to a different antigen or a different epitope on the same antigen from that of the antigen-binding region A and the antigen-binding region B.

Embodiment 17. The antigen-binding protein according to Embodiment 16, wherein the antigen-binding region C binds to serum albumin.

Embodiment 18. The antigen-binding protein according to any one of Embodiments 1 to 10, wherein

the VL_A comprises LCDR1, LCDR2, and LCDR3 having the amino acid sequences as shown in SEQ ID NO: 81, 99, and 117, respectively; the VH_A comprises HCDR1, HCDR2, and HCDR3 having the amino acid sequences as shown in SEQ ID NO: 12, 35, and 58, respectively; and the VL_B comprises LCDR1, LCDR2, and LCDR3 having the amino acid sequences as shown in SEQ ID NO: 82, 100, and 118, respectively; the VH_B comprises HCDR1, HCDR2, and HCDR3 having the amino acid sequences as shown in SEQ ID NO: 13, 36, and 59, respectively;
the VL_A comprises LCDR1, LCDR2, and LCDR3 having the amino acid sequences as shown in SEQ ID NO: 85, 103, and 121, respectively; the VH_A comprises HCDR1, HCDR2, and HCDR3 having the amino acid sequences as shown in SEQ ID NO: 16, 39, and 62, respectively; and the VL_B comprises LCDR1, LCDR2, and LCDR3 having the amino acid sequences as shown in SEQ ID NO: 82, 100, and 118, respectively; the VH_B comprises HCDR1, HCDR2, and HCDR3 having the amino acid sequences as shown in SEQ ID NO: 13, 36, and 59, respectively;
the VL_A comprises LCDR1, LCDR2, and LCDR3 having the amino acid sequences as shown in SEQ ID NO: 83, 101, and 119, respectively; the VH_A comprises HCDR1, HCDR2, and HCDR3 having the amino acid sequences as shown in SEQ ID NO: 14, 37, and 60, respectively; and the VL_B comprises LCDR1, LCDR2, and LCDR3 having the amino acid sequences as shown in SEQ ID NO: 82, 100, and 118, respectively; the VH_B comprises HCDR1, HCDR2, and HCDR3 having the amino acid sequences as shown in SEQ ID NO: 13, 36, and 59, respectively;
the VL_A comprises LCDR1, LCDR2, and LCDR3 having the amino acid sequences as shown in SEQ ID NO: 86, 104, and 122, respectively; the VH_A comprises HCDR1, HCDR2, and HCDR3 having the amino acid sequences as shown in SEQ ID NO: 17, 40, and 63, respectively; and the VL_B comprises LCDR1, LCDR2, and LCDR3 having the amino acid sequences as shown in SEQ ID NO: 82, 100, and 118, respectively; the VH_B comprises HCDR1, HCDR2, and HCDR3 having the amino acid sequences as shown in SEQ ID NO: 13, 36, and 59, respectively;
the VL_A comprises LCDR1, LCDR2, and LCDR3 having the amino acid sequences as shown in SEQ ID NO: 84, 102, and 120, respectively; the VH_A comprises HCDR1, HCDR2, and HCDR3 having the amino acid sequences as shown in SEQ ID NO: 15, 38, and 61, respectively; and the VL_B comprises LCDR1, LCDR2, and LCDR3 having the amino acid sequences as shown in SEQ ID NO: 82, 100, and 118, respectively; the VH_B comprises HCDR1, HCDR2, and HCDR3 having the amino acid sequences as shown in SEQ ID NO: 13, 36, and 59, respectively;
the VL_A comprises LCDR1, LCDR2, and LCDR3 having the amino acid sequences as shown in SEQ ID NO: 81, 99, and 117, respectively; the VH_A comprises HCDR1, HCDR2, and HCDR3 having the amino acid sequences as shown in SEQ ID NO: 12, 35, and 58, respectively; and the VL_B comprises LCDR1, LCDR2, and LCDR3 having the amino acid sequences as shown in SEQ ID NO: 89, 107, and 125, respectively; the VH_B comprises HCDR1, HCDR2, and HCDR3 having the amino acid sequences as shown in SEQ ID NO: 12, 44, and 67, respectively;
the VL_A comprises LCDR1, LCDR2, and LCDR3 having the amino acid sequences as shown in SEQ ID NO: 81, 99, and 117, respectively; the VH_A comprises HCDR1, HCDR2, and HCDR3 having the amino acid sequences as shown in SEQ ID NO: 12, 35, and 58, respectively; and the VL_B comprises LCDR1, LCDR2, and LCDR3 having the amino acid sequences as shown in SEQ ID NO: 89, 107, and 125, respectively; the VH_B comprises HCDR1, HCDR2, and HCDR3 having the amino acid sequences as shown in SEQ ID NO: 12, 45, and 67, respectively;
the VL_A comprises LCDR1, LCDR2, and LCDR3 having the amino acid sequences as shown in SEQ ID NO: 83, 101, and 119, respectively; the VH_A comprises HCDR1, HCDR2, and HCDR3 having the amino acid sequences as shown in SEQ ID NO: 14, 37, and 60, respectively; and the VL_B comprises LCDR1, LCDR2, and LCDR3 having the amino acid sequences as shown in SEQ ID NO: 89, 107, and 125, respectively; the VH_B comprises HCDR1, HCDR2, and HCDR3 having the amino acid sequences as shown in SEQ ID NO: 12, 44, and 67, respectively;
the VL_A comprises LCDR1, LCDR2, and LCDR3 having the amino acid sequences as shown in SEQ ID NO: 83, 101, and 119, respectively; the VH_A comprises HCDR1, HCDR2, and HCDR3 having the amino acid sequences as shown in SEQ ID NO: 14, 37, and 60, respectively; and the VL_B comprises LCDR1, LCDR2, and LCDR3 having the amino acid sequences as shown in SEQ ID NO: 89, 107, and 125, respectively; the VH_B comprises HCDR1, HCDR2, and HCDR3 having the amino acid sequences as shown in SEQ ID NO: 12, 45, and 67, respectively;

the VL_A comprises LCDR1, LCDR2, and LCDR3 having the amino acid sequences as shown in SEQ ID NO: 84, 102, and 120, respectively; the VH_A comprises HCDR1, HCDR2, and HCDR3 having the amino acid sequences as shown in SEQ ID NO: 15, 38, and 61, respectively; and the VL_B comprises LCDR1, LCDR2, and LCDR3 having the amino acid sequences as shown in SEQ ID NO: 88, 106, and 124, respectively; the VH_B comprises HCDR1, HCDR2, and HCDR3 having the amino acid sequences as shown in SEQ ID NO: 19, 42, and 65, respectively;

the VL_A comprises LCDR1, LCDR2, and LCDR3 having the amino acid sequences as shown in SEQ ID NO: 84, 102, and 120, respectively; the VH_A comprises HCDR1, HCDR2, and HCDR3 having the amino acid sequences as shown in SEQ ID NO: 15, 38, and 61, respectively; and the VL_B comprises LCDR1, LCDR2, and LCDR3 having the amino acid sequences as shown in SEQ ID NO: 87, 105, and 123, respectively; the VH_B comprises HCDR1, HCDR2, and HCDR3 having the amino acid sequences as shown in SEQ ID NO: 18, 41, and 64, respectively;

the VL_A comprises LCDR1, LCDR2, and LCDR3 having the amino acid sequences as shown in SEQ ID NO: 81, 99, and 117, respectively; the VH_A comprises HCDR1, HCDR2, and HCDR3 having the amino acid sequences as shown in SEQ ID NO: 12, 35, and 58, respectively; and the VL_B comprises LCDR1, LCDR2, and LCDR3 having the amino acid sequences as shown in SEQ ID NO: 88, 106, and 124, respectively; the VH_B comprises HCDR1, HCDR2, and HCDR3 having the amino acid sequences as shown in SEQ ID NO: 19, 42, and 65, respectively; or

the VL_A comprises LCDR1, LCDR2, and LCDR3 having the amino acid sequences as shown in SEQ ID NO: 81, 99, and 117, respectively; the VH_A comprises HCDR1, HCDR2, and HCDR3 having the amino acid sequences as shown in SEQ ID NO: 12, 35, and 58, respectively; and the VL_B comprises LCDR1, LCDR2, and LCDR3 having the amino acid sequences as shown in SEQ ID NO: 87, 105, and 123, respectively; the VH_B comprises HCDR1, HCDR2, and HCDR3 having the amino acid sequences as shown in SEQ ID NO: 18, 41, and 64, respectively,

wherein the CDR sequences are defined according to Chothia numbering system.

Embodiment 19. The antigen-binding protein according to Embodiment 18, wherein

the VL_A comprises the amino acid sequence as shown in SEQ ID NO: 144; the VH_A comprises the amino acid sequence as shown in SEQ ID NO: 131; the VL_B comprises the amino acid sequence as shown in SEQ ID NO: 145; and the VH_B comprises the amino acid sequence as shown in SEQ ID NO: 132;

the VL_A comprises the amino acid sequence as shown in SEQ ID NO: 144; the VH_A comprises the amino acid sequence as shown in SEQ ID NO: 131; the VL_B comprises the amino acid sequence as shown in SEQ ID NO: 151; and the VH_B comprises the amino acid sequence as shown in SEQ ID NO: 138;

the VL_A comprises the amino acid sequence as shown in SEQ ID NO: 148; the VH_A comprises the amino acid sequence as shown in SEQ ID NO: 135; the VL_B comprises the amino acid sequence as shown in SEQ ID NO: 151; and the VH_B comprises the amino acid sequence as shown in SEQ ID NO: 138;

the VL_A comprises the amino acid sequence as shown in SEQ ID NO: 146; the VH_A comprises the amino acid sequence as shown in SEQ ID NO: 133; the VL_B comprises the amino acid sequence as shown in SEQ ID NO: 151; and the VH_B comprises the amino acid sequence as shown in SEQ ID NO: 138;

the VL_A comprises the amino acid sequence as shown in SEQ ID NO: 149; the VH_A comprises the amino acid sequence as shown in SEQ ID NO: 136; the VL_B comprises the amino acid sequence as shown in SEQ ID NO: 151; and the VH_B comprises the amino acid sequence as shown in SEQ ID NO: 138;

the VL_A comprises the amino acid sequence as shown in SEQ ID NO: 147; the VH_A comprises the amino acid sequence as shown in SEQ ID NO: 134; the VL_B comprises the amino acid sequence as shown in SEQ ID NO: 151; and the VH_B comprises the amino acid sequence as shown in SEQ ID NO: 138;

the VL_A comprises the amino acid sequence as shown in SEQ ID NO: 144; the VH_A comprises the amino acid sequence as shown in SEQ ID NO: 131; the VL_B comprises the amino acid sequence as shown in SEQ ID NO: 153; and the VH_B comprises the amino acid sequence as shown in SEQ ID NO: 141;

the VL_A comprises the amino acid sequence as shown in SEQ ID NO: 144; the VH_A comprises the amino acid sequence as shown in SEQ ID NO: 131; the VL_B comprises the amino acid sequence as shown in SEQ ID NO: 153; and the VH_B comprises the amino acid sequence as shown in SEQ ID NO: 142;

the VL_A comprises the amino acid sequence as shown in SEQ ID NO: 146; the VH_A comprises the amino acid sequence as shown in SEQ ID NO: 133; the VL_B comprises the amino acid sequence as shown in SEQ ID NO: 153; and the VH_B comprises the amino acid sequence as shown in SEQ ID NO: 141;

the VL_A comprises the amino acid sequence as shown in SEQ ID NO: 146; the VH_A comprises the amino acid sequence as shown in SEQ ID NO: 133; the VL_B comprises the amino acid sequence as shown in SEQ ID NO: 153; and the VH_B comprises the amino acid sequence as shown in SEQ ID NO: 142;

the VL_A comprises the amino acid sequence as shown in SEQ ID NO: 147; the VH_A comprises the amino acid sequence as shown in SEQ ID NO: 134; the VL_B comprises the amino acid sequence as shown in SEQ ID NO: 152; and the VH_B comprises the amino acid sequence as shown in SEQ ID NO: 139;

the VL_A comprises the amino acid sequence as shown in SEQ ID NO: 147; the VH_A comprises the amino acid sequence as shown in SEQ ID NO: 134; the VL_B comprises the amino acid sequence as shown in SEQ ID NO: 150; and the VH_B comprises the amino acid sequence as shown in SEQ ID NO: 137;

the VL_A comprises the amino acid sequence as shown in SEQ ID NO: 144; the VH_A comprises the amino acid sequence as shown in SEQ ID NO: 131; the VL_B comprises the amino acid sequence as shown in SEQ ID NO: 152; and the VH_B comprises the amino acid sequence as shown in SEQ ID NO: 139; or

the VL_A comprises the amino acid sequence as shown in SEQ ID NO: 144; the VH_A comprises the amino acid sequence as shown in SEQ ID NO: 131; the VL_B comprises the amino acid sequence as shown in SEQ ID NO: 150; and the VH_B comprises the amino acid sequence as shown in SEQ ID NO: 137.

Embodiment 20. The antigen-binding protein according to Embodiment 1, wherein

the first polypeptide chain comprises the amino acid sequence as shown in SEQ ID NO: 177; the second polypeptide chain comprises the amino acid sequence as shown in SEQ ID NO: 178; and the third polypeptide chain comprises the amino acid sequence as shown in SEQ ID NO: 168;

the first polypeptide chain comprises the amino acid sequence as shown in SEQ ID NO: 177; the second polypeptide chain comprises the amino acid sequence as shown in SEQ ID NO: 187; and the third polypeptide chain comprises the amino acid sequence as shown in SEQ ID NO: 174;

the first polypeptide chain comprises the amino acid sequence as shown in SEQ ID NO: 179; the second polypeptide chain comprises the amino acid sequence as shown in SEQ ID NO: 180; and the third polypeptide chain comprises the amino acid sequence as shown in SEQ ID NO: 174;

the first polypeptide chain comprises the amino acid sequence as shown in SEQ ID NO: 181; the second polypeptide chain comprises the amino acid sequence as shown in SEQ ID NO: 182; and the third polypeptide chain comprises the amino acid sequence as shown in SEQ ID NO: 174;

the first polypeptide chain comprises the amino acid sequence as shown in SEQ ID NO: 183; the second polypeptide chain comprises the amino acid sequence as shown in SEQ ID NO: 184; and the third polypeptide chain comprises the amino acid sequence as shown in SEQ ID NO: 174;

the first polypeptide chain comprises the amino acid sequence as shown in SEQ ID NO: 185; the second polypeptide chain comprises the amino acid sequence as shown in SEQ ID NO: 186; and the third polypeptide chain comprises the amino acid sequence as shown in SEQ ID NO: 174;

the first polypeptide chain comprises the amino acid sequence as shown in SEQ ID NO: 177; the second polypeptide chain comprises the amino acid sequence as shown in SEQ ID NO: 188; and the third polypeptide chain comprises the amino acid sequence as shown in SEQ ID NO: 176;

the first polypeptide chain comprises the amino acid sequence as shown in SEQ ID NO: 177; the second polypeptide chain comprises the amino acid sequence as shown in SEQ ID NO: 189; and the third polypeptide chain comprises the amino acid sequence as shown in SEQ ID NO: 176;

the first polypeptide chain comprises the amino acid sequence as shown in SEQ ID NO: 181; the second polypeptide chain comprises the amino acid sequence as shown in SEQ ID NO: 190; and the third polypeptide chain comprises the amino acid sequence as shown in SEQ ID NO: 176;

the first polypeptide chain comprises the amino acid sequence as shown in SEQ ID NO: 181; the second polypeptide chain comprises the amino acid sequence as shown in SEQ ID NO: 191; and the third polypeptide chain comprises the amino acid sequence as shown in SEQ ID NO: 176;

the first polypeptide chain comprises the amino acid sequence as shown in SEQ ID NO: 185; the second polypeptide chain comprises the amino acid sequence as shown in SEQ ID NO: 193; and the third polypeptide chain comprises the amino acid sequence as shown in SEQ ID NO: 192;

the first polypeptide chain comprises the amino acid sequence as shown in SEQ ID NO: 185; the second polypeptide chain comprises the amino acid sequence as shown in SEQ ID NO: 194; and the third polypeptide chain comprises the amino acid sequence as shown in SEQ ID NO: 173;

the first polypeptide chain comprises the amino acid sequence as shown in SEQ ID NO: 177; the second polypeptide chain comprises the amino acid sequence as shown in SEQ ID NO: 195; and the third polypeptide chain comprises the amino acid sequence as shown in SEQ ID NO: 192; or

the first polypeptide chain comprises the amino acid sequence as shown in SEQ ID NO: 177; the second polypeptide chain comprises the amino acid sequence as shown in SEQ ID NO: 196; and the third polypeptide chain comprises the amino acid sequence as shown in SEQ ID NO: 173.

Embodiment 21. The antigen-binding protein according to any one of Embodiments 1-20, wherein the antigen-binding protein does not comprise an antibody Fc region.

Embodiment 22. An isolated nucleic acid encoding the antigen-binding protein according to any one of Embodiments 1-21.

Embodiment 23. A recombinant expression vector comprising the isolated nucleic acid according to Embodiment 22;

preferably, the expression vector comprises a eukaryotic cell expression vector and/or a prokaryotic cell expression vector.

Embodiment 24. A host cell comprising the isolated nucleic acid according to Embodiment 22 or the recombinant expression vector according to Embodiment 23; preferably, the host cell is a prokaryotic cell and/or a eukaryotic cell, preferably, the prokaryotic cell is an E. coli cell, such as TG1 or BL21 cell, and preferably, the eukaryotic cell is an HEK293 cell or a CHO cell.

Embodiment 25. A preparation method for an antigen-binding protein, comprising culturing the host cell according to Embodiment 24, and obtaining the antigen-binding protein from the culture.

Embodiment 26. A pharmaceutical composition characterized in that the pharmaceutical composition comprises the antigen-binding protein according to any one of Embodiments 1-21, and a pharmaceutically acceptable carrier;

preferably, the pharmaceutical composition further comprises one or more additional anti-tumor agents as active ingredients.

Embodiment 27. A method for preventing and/or treating a disease in a subject in need thereof, comprising a step of administering to the subject an effective amount of the antigen-binding protein according to any one of Embodiments 1-21 or the pharmaceutical composition according to Embodiment 26.

Embodiment 28. The method according to Embodiment 27, wherein the disease is cancer.

Embodiment 29. A preparation method for the antigen-binding protein according to any one of Embodiments 1-21, comprising:

(a) expressing the first polypeptide chain, the second polypeptide chain, and the third polypeptide chain of the antigen-binding protein in a host cell to obtain a protein mixture assembled by the first polypeptide chain, the second polypeptide chain, and the third polypeptide chain;

(b) based on the type of the light chain constant region comprised in the second polypeptide chain of the antigen-binding protein, affinity-capturing the second polypeptide chain with a corresponding affinity chromatography method, so as to obtain the antigen-binding protein composed of the first polypeptide chain and the third polypeptide chain interacting with the second polypeptide chain, and to remove the mismatched products not containing the second polypeptide.

Embodiment 30. The preparation method according to Embodiment 29, wherein the second polypeptide chain contains a Cκ region, and the antigen-binding protein is obtained with a κ-chain specific affinity chromatography method.

Embodiment 31. The preparation method according to Embodiment 29, wherein the second polypeptide chain contains a Cλ region, and the antigen-binding protein is obtained with a λ-chain specific affinity chromatography method.

## Brief Description of the Drawings

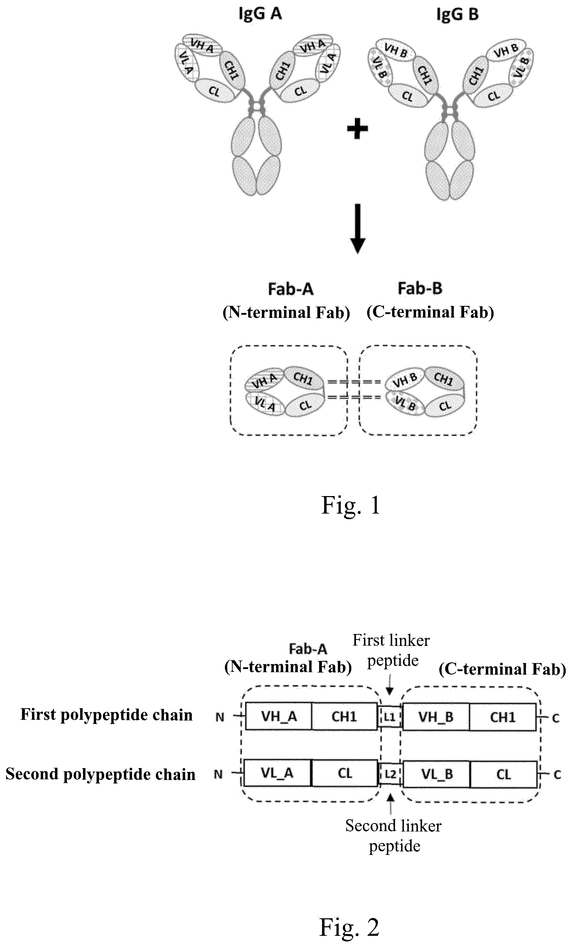

Fig. 1 shows the construction of a "1+1" bispecific antibody fragment Fab-Fab series-connection structure with two IgG antibodies (IgG A and IgG B).

Fig. 2 shows Solution 1 for constructing the Fab-Fab bispecific antibody structure consisting of two polypeptide chains. That is, a first polypeptide chain is formed by linking the C-terminus of the heavy chain CH1 of a first Fab (Fab-A) to the N-terminus of the heavy chain VH of a second Fab (Fab-B) with one linker peptide, and a second polypeptide chain is formed by linking the C-terminus of the light chain CL of Fab-A to the N-terminus of the light chain VL of Fab-B with another linker peptide. The VL may comprise a Vκ and/or a Vλ; and the CL may comprise a Cκ and/or a Cλ. The CH1 may be a CH1 region of human IgG1, IgG2, or IgG4.

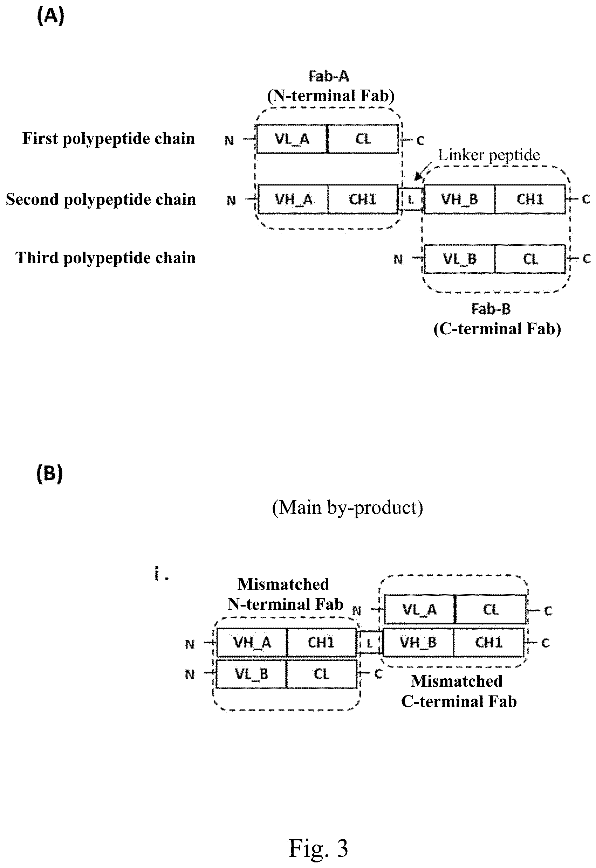

Fig. 3 shows Solution 2 for constructing the Fab-Fab bispecific antibody structure consisting of three polypeptide chains. (A) One of the polypeptide chains is formed by linking the C-terminus of the heavy chain CH1 of a first Fab (Fab-A) to the N-terminus of the heavy chain VH of a second Fab (Fab-B) with a linker peptide, wherein the light chain of Fab-A (VL_A-CL) and the light chain of Fab-B (VL_B-CL) are two independent polypeptide chains. (B) The main by-product of this solution is the mismatched product formed by the exchange of the two light chains of Fab-A and Fab-B, i.e., the light chain of Fab-A (VL_A-CL) pairs with the heavy chain Fd of Fab-B (VH_B-CH1), and the light chain of Fab-B (VL_B-CL) pairs with the heavy chain Fd of Fab-A (VH_A-CH1). The resulting mismatched by-product loses its original antigen-binding specificity. The VL may comprise a V$\kappa$ and/or a V$\lambda$; and the CL may comprise a C$\kappa$ and/or a C$\lambda$.

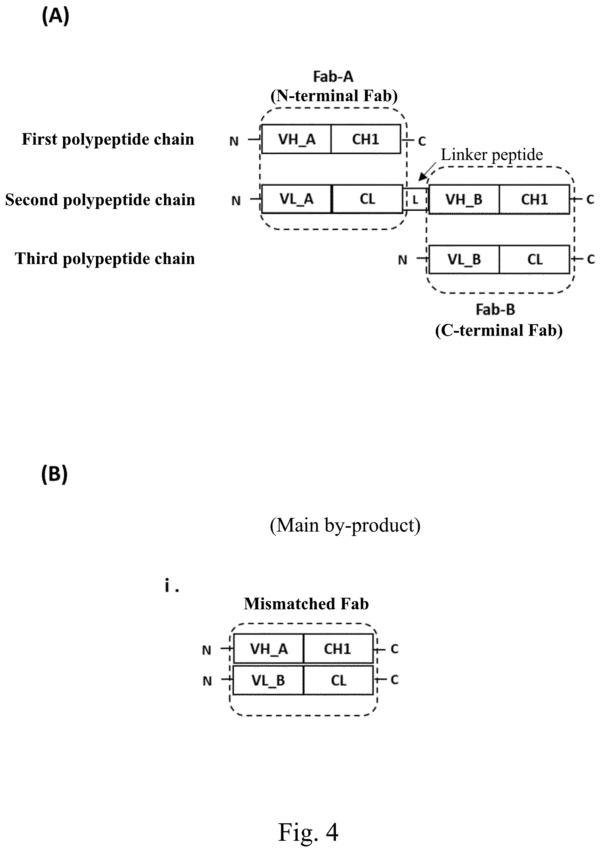

Fig. 4 shows Solution 3 for constructing the Fab-Fab bispecific antibody structure consisting of three polypeptide chains. (A) One of the polypeptide chains is formed by linking the C-terminus of the light chain CL of a first Fab (Fab-A) to the N-terminus of the heavy chain VH of a second Fab (Fab-B) with a linker peptide, wherein the heavy chain Fd of Fab-A (VH_A-CH1) and the light chain of Fab-B (VL_B-CL) are two independent polypeptide chains. (B) The main by-product in this solution is the mismatched Fab formed by the pairing of the heavy chain Fd of Fab-A (VH_A-CH1) and the light chain of Fab-B (VL_B-CL); and the resulting mismatched by-product loses its original antigen-binding specificity. The VL may comprise a V$\kappa$ and/or a V$\lambda$; and the CL may comprise a C$\kappa$ and/or a C$\lambda$.

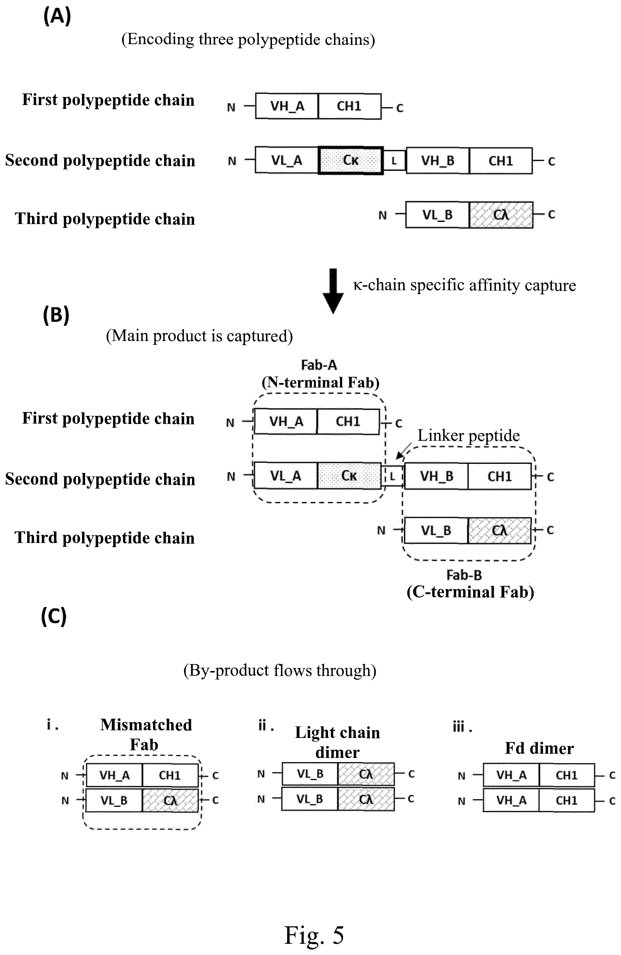

Fig. 5 shows a structural form and preparation method of the "$\kappa$/$\lambda$" Fab-Fab series-connection bispecific antibody described herein. The structure shown in (A) has three polypeptide chains: a first polypeptide chain sequentially comprising VH_A-CH1 from the N-terminus to the C-terminus, a second polypeptide chain sequentially comprising VL_A-C$\kappa$-L-VH_B-CH1 from the N-terminus to the C-terminus, and a third polypeptide chain sequentially comprising VL_B-C$\lambda$ from the N-terminus to the C-terminus. The VL may comprise a V$\kappa$ and/or a V$\lambda$. The structure is similar to the structure as shown in Fig. 4, except that the CL domain in the second polypeptide chain specifically refers to C$\kappa$, while the CL domain in the third polypeptide chain specifically refers to C$\lambda$. (B) Cells were transfected with a plasmid encoding three polypeptide chains for expression; and the main target product, which consists of three peptide chains, including one C$\kappa$ domain and one C$\lambda$ domain, can be captured using a $\kappa$-chain specific affinity chromatography media. (C) Since the chain-mismatched by-products do not contain the C$\kappa$ domain, they cannot be captured by the $\kappa$-chain specific affinity chromatography media and flow through as impurities.

Fig. 6 shows the four structural forms of the "$\kappa$/$\lambda$" Fab-Fab series-connection bispecific antibody described herein and the adopted preparation methods. (A) Configuration-I (i.e., the structure as shown in Fig. 5): The part of the N-terminal Fab is formed by a C$\kappa$ domain located in the second domain of the second polypeptide chain (i.e., the long chain); the part of the C-terminal Fab is formed by a C$\lambda$ domain located in the second domain of the third polypeptide chain; and $\kappa$-chain specific affinity capture is adopted for the main product. (B) Configuration-II: The part of the N-terminal Fab is formed by a C$\lambda$ domain located in the second domain of the second polypeptide chain (i.e., the long chain); the part of the C-terminal Fab is formed by a C$\kappa$ domain located in the second domain of the third polypeptide chain; and $\lambda$-chain specific affinity capture is adopted for the main product. (C) Configuration-III: The part of the C-terminal Fab is formed by a C$\kappa$ domain located in the fourth domain of the second polypeptide chain (i.e., the long chain); the part of the N-terminal Fab is formed by a C$\lambda$ domain located in the second domain of the third polypeptide chain; and $\kappa$-chain specific affinity capture is adopted for the main product. (D) Configuration-IV: The part of the C-terminal Fab is formed by a C$\lambda$ domain located in the fourth domain of the second polypeptide chain (i.e., the long chain); the part of the N-terminal Fab is formed by a C$\kappa$ domain located in the second domain of the third polypeptide chain; and $\lambda$-chain specific affinity capture is adopted for the main product. The VL in the Configuration-I, Configuration-II, Configuration-III, and Configuration-IV shown in Fig. 6 may include a V$\kappa$ and/or a V$\lambda$.

Fig. 7 shows various Solutions for preparing the "$\kappa$/$\lambda$" Fab-Fab series-connection bispecific antibody described herein using an IgG antibody A with a $\kappa$ light chain and an IgG antibody B with a $\lambda$ light chain. It instances the Configuration-I, Configuration-II, Configuration-III, and Configuration-IV as shown in Fig. 6, with VL_A therein as V$\kappa$_A and VL_B designated as V$\lambda$_B. In Solution (1) and Solution (3), the linkages between VL and CL are of the same type. That is, V$\kappa$_A is linked to C$\kappa$ to form a $\kappa$ light chain of Fab-A, and V$\lambda$_B is linked to C$\lambda$ to form a $\lambda$ light chain of Fab-B. $\kappa$-chain specific affinity capture is adopted for the main product. In Solution (2) and Solution (4), the linkages between VL and CL are of different types. That is, V$\kappa$_A is linked with C$\lambda$ to form a V$\kappa$C$\lambda$ light chain of Fab-A, and V$\lambda$_B is linked with C$\kappa$ to form a V$\lambda$C$\kappa$ light chain. $\lambda$-chain specific affinity is adopted for the main product.

Fig. 8 shows various Solutions for preparing the "$\kappa$/$\lambda$" Fab-Fab series-connection bispecific antibody described herein using an IgG antibody A with a $\kappa$ light chain and an IgG antibody B with a $\kappa$ light chain. It instances the Configuration-I, Configuration-II, Configuration-III, and Configuration-IV as shown in Fig. 6, with VL_A therein designated as V$\kappa$_A and VL_B designated as V$\kappa$_B. To distinguish the main product from the by-product using different affinity chromatography media ($\kappa$-chain specific affinity or $\lambda$-chain specific affinity), one of the C$\kappa$ is replaced with C$\lambda$. In Solution (5) and Solution (7), the C$\kappa$ of Fab-B is replaced with C$\lambda$, so that its light chain has a V$\kappa$C$\lambda$ configuration. $\kappa$-chain specific affinity capture is adopted for the main product. In Solution (6) and Solution (8), the C$\kappa$ of Fab-A is replaced with C$\lambda$, so that its light chain has a V$\kappa$C$\lambda$ configuration. $\lambda$-chain specific affinity is adopted for the main product.

Fig. 9 shows various Solutions for preparing the "$\kappa$/$\lambda$" Fab-Fab series-connection bispecific antibody described herein using an IgG antibody A with a $\lambda$ light chain and an IgG antibody B with a $\lambda$ light chain. It instances the Configuration-I, Configuration-II, Configuration-III, and Configuration-IV as shown in Fig. 6 with VL_A therein designated as V$\lambda$_A and VL_B designated as V$\lambda$_B. To distinguish the main product from the by-product using different affinity chromatography media ($\kappa$-chain specific affinity or $\lambda$-chain specific affinity), one of the C$\lambda$ is replaced with C$\kappa$. In Solution (9) and Solution (11), the C$\lambda$ of Fab-A is replaced with C$\kappa$, so that its light chain has a V$\lambda$C$\kappa$ configuration. $\kappa$-chain specific affinity capture is adopted for the main product. In Solution (10) and Solution (12), the C$\lambda$ of Fab-B is replaced with C$\kappa$,

so that its light chain has a VλCκ configuration. λ-chain specific affinity is adopted for the main product.

Fig. 10 shows the structures of the "κ/λ" Fab-Fab series-connection bispecific/multispecific antibodies produced in specific embodiments of the present disclosure. (A) Using an IgG antibody A with a κ light chain and an IgG antibody B with a λ light chain to prepare a "κ/λ" Fab-Fab series-connection bispecific antibody having the structure as shown in Solution (1) in Fig. 7, with no additional linker peptide between Fab-A and Fab-B. (B) Using an IgG antibody A with a κ light chain and an IgG antibody B with a κ light chain to prepare a "κ/λ" Fab-Fab series-connection bispecific antibody having the structure as shown in Solution (5) in Fig. 7, with no additional linker peptide between Fab-A and Fab-B. (C) A third antigen-binding domain is added to the structure shown in (A) to form a trispecific antibody with a "κ/λ" Fab-Fab series-connection structure. In particular, the heavy chain antibody variable region VH of an anti-serum albumin antibody is linked to the C-terminus of the CH1 region of the second polypeptide chain (i.e., the long chain) via a linker peptide.

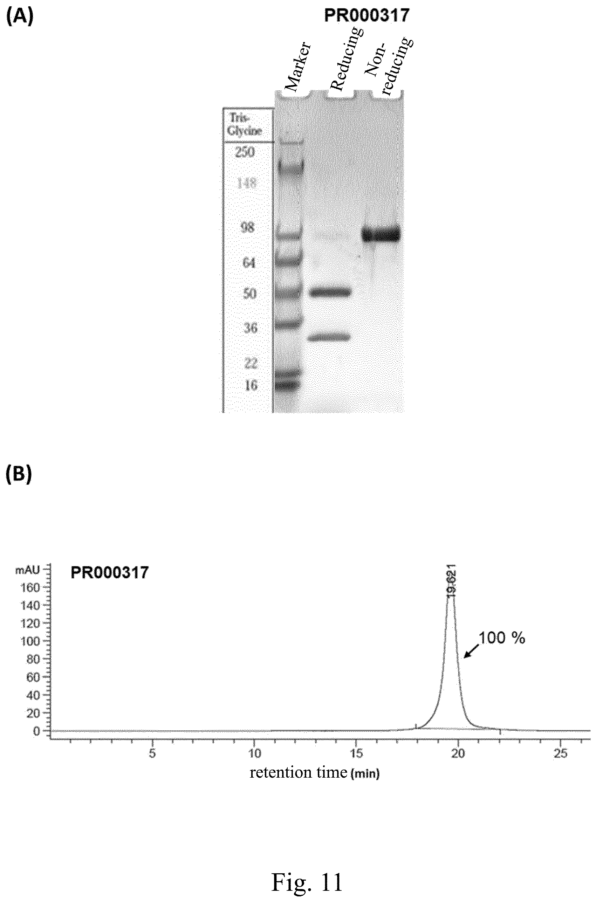

Fig. 11 shows (A) the SDS-PAGE results and (B) the SEC-HPLC results of a "κ/λ" Fab-Fab bispecific antibody PR000317 after one-step KappaSelect affinity capture purification.

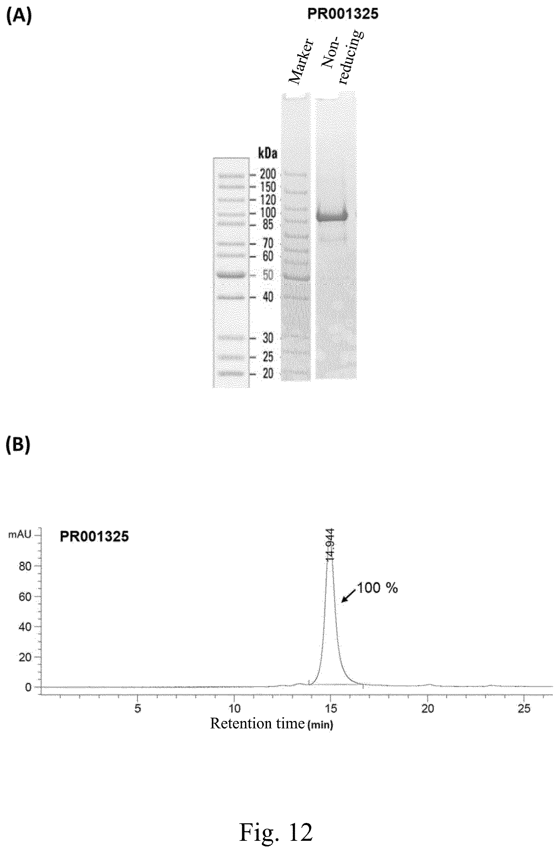

Fig. 12 shows (A) the SDS-PAGE results and (B) the SEC-HPLC results of a "κ/λ" Fab-Fab trispecific antibody PR001325 after one-step KappaSelect affinity capture purification.

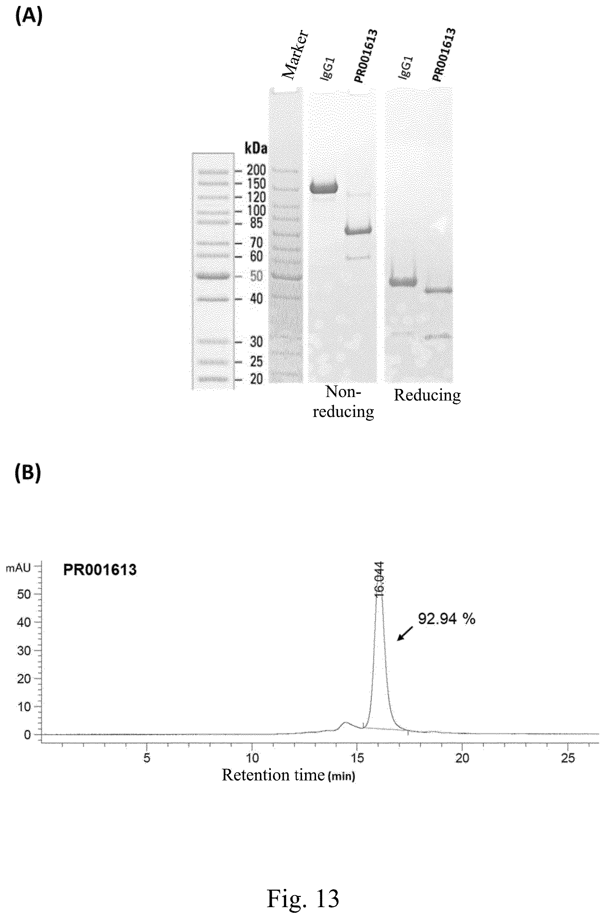

Fig. 13 shows (A) the SDS-PAGE results and (B) the SEC-HPLC results of a "κ/λ" Fab-Fab bispecific antibody PR001613 after one-step KappaSelect affinity capture purification.

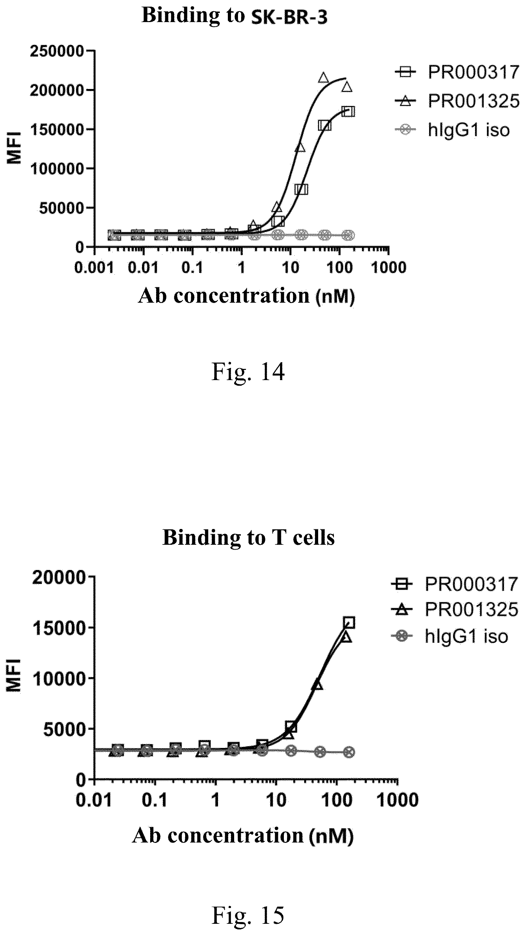

Fig. 14 shows the binding activity of B7H4xCD3 "κ/λ" Fab-Fab antibodies (PR000317 and PR001325) to the tumor cells SK-BR-3.

Fig. 15 shows the binding activity of B7H4xCD3 "κ/λ" Fab-Fab antibodies (PR000317 and PR001325) to human T cells.

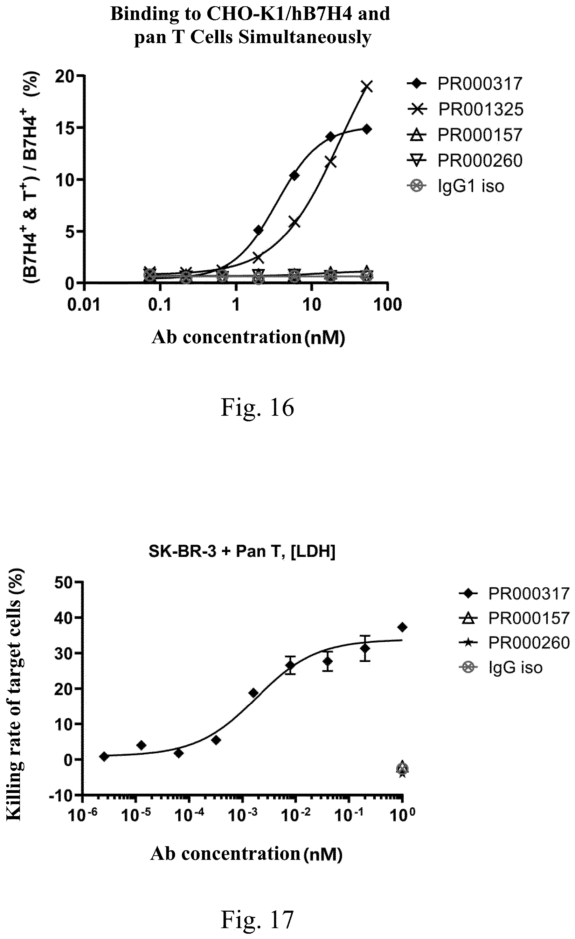

Fig. 16 shows that B7H4xCD3 "κ/λ" Fab-Fab antibodies (PR000317 and PR001325) can simultaneously bind to the target cells CHO-K1/hB7H4 with high B7H4 expression and human T cells, while neither the B7H4 monoclonal antibody (PR000157) nor the CD3 monoclonal antibody (PR000260) can simultaneously bind to both of them.

Fig. 17 shows that a B7H4xCD3 "κ/λ" Fab-Fab antibody (PR000317) can effectively kill SK-BR-3 cells with high expression of B7H4, while neither the B7H4 monoclonal antibody (PR000157) nor the CD3 monoclonal antibody (PR000260) can cause significant cell killing.

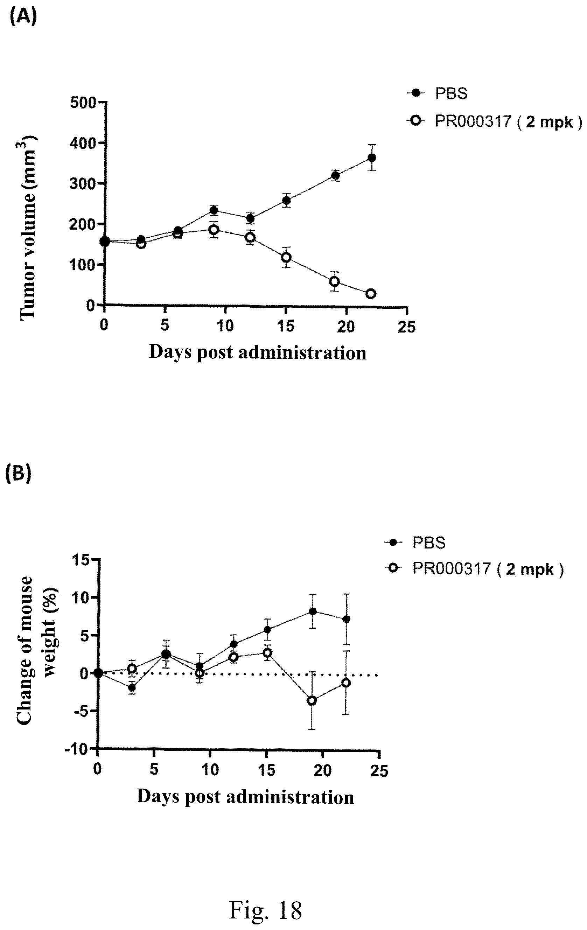

Fig. 18 shows that a B7H4xCD3 "κ/λ" Fab-Fab antibody (PR000317) exhibits significant anti-tumor effects in the MDA-MB-468 tumor model with human PBMC-reconstituted NCG mice. (A) Changes in tumor volume, (B) Changes in mouse body weight.

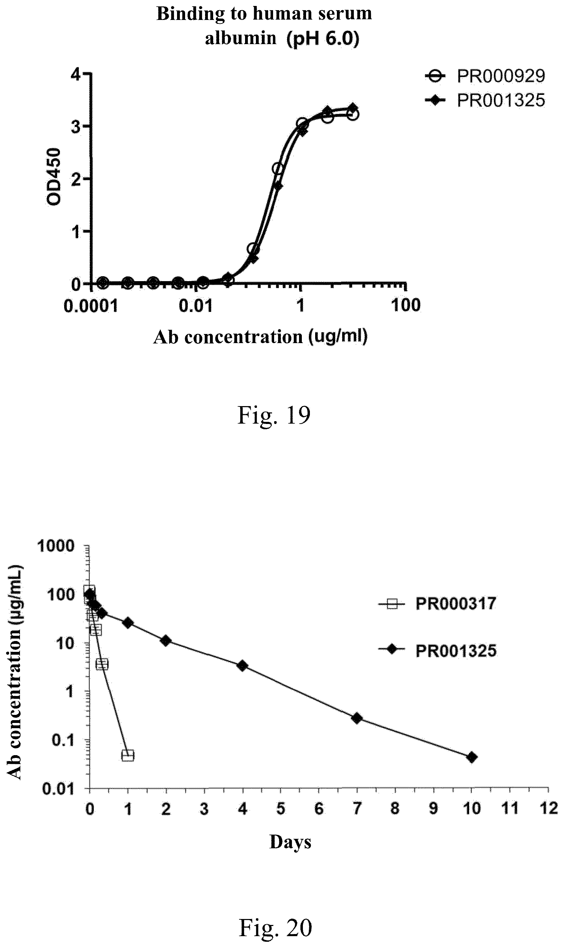

Fig. 19 shows that a B7H4xCD3 "κ/λ" Fab-Fab antibody (PR001325) containing the VH domain of anti-serum albumin can bind to human serum albumin.

Fig. 20 shows the comparison of the pharmacokinetics of B7H4xCD3 "κ/λ" Fab-Fab antibodies (PR000317 and PR001325) in mice. PR001325 which is formed by the addition of a serum albumin-binding domain basd on PR000317 exhibits a longer serum half-life than PR000317.

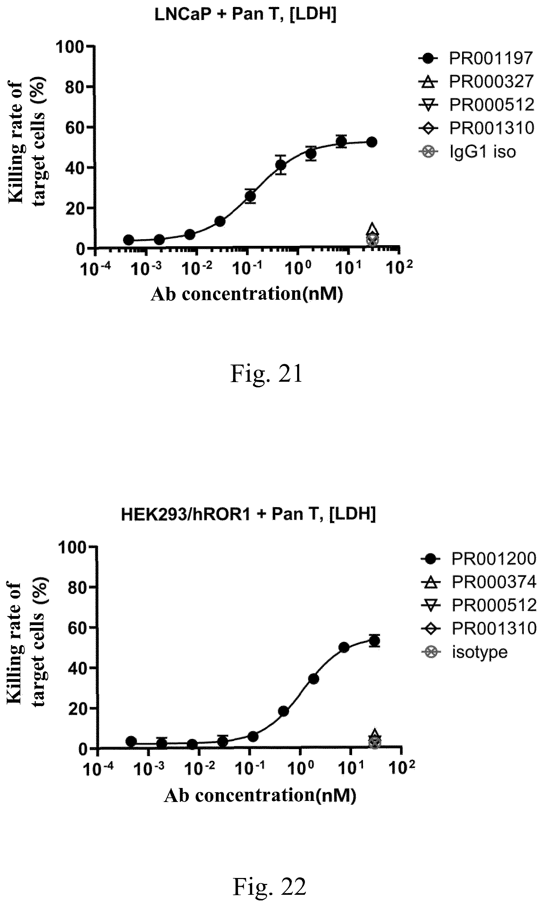

Fig. 21 shows that a PSMAxCD3 "κ/λ" Fab-Fab antibody (PR001197) can effectively kill LNCaP cells with high PSMA expression, while a PSMA monoclonal antibody (PR000327), a CD3 monoclonal antibody (PR000512), and the control HELxCD3 "κ/λ" Fab-Fab antibody (PR001310) cannot cause significant cell killing.

Fig. 22 shows that a ROR1xCD3 "κ/λ" Fab-Fab antibody (PR001200) can effectively kill HEK293/hRORI cells with high ROR1 expression, while a ROR1 monoclonal antibody (PR000374), a CD3 monoclonal antibody (PR000512), and the control HELxCD3 "κ/λ" Fab-Fab antibody (PR001310) cannot cause significant cell killing.

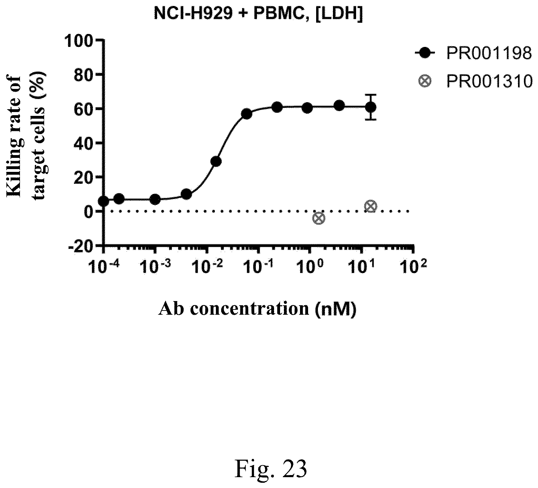

Fig. 23 shows that a BCMAxCD3 "κ/λ" Fab-Fab antibody (PR001198) can effectively kill NCI-H929 cells with high BCMA expression, while the control HELxCD3 "κ/λ" Fab-Fab antibody (PR001310) cannot cause significant cell killing.

### Detailed Description of the Disclosure

The implementation of the invention of this application is described by the following specific embodiments. Those skilled in the art can readily understand other advantages and effects of the present application from the disclosure of this specification.

In the present application, the term "binding protein" or "antigen-binding protein" generally refers to a protein comprising an antigen-binding moiety, and optionally a scaffold or framework moiety that allows the antigen-binding moiety to adopt a conformation that facilitates the binding of the antigen-binding protein to the antigen. An antibody may typically comprise an antibody light chain variable region (VL) or an antibody heavy chain variable region (VH), or both. The VH and VL regions can be further divided into hypervariable regions termed complementarity determining regions (CDRs), which are scattered over more conserved regions termed framework regions (FRs). Each VH and VL can consist of three CDR regions and four FR regions arranged from amino-terminus to carboxyl-terminus in the following order: FR-1, CDR1, FR-2, CDR2, FR-3, CDR3 and FR-4. The variable regions of the heavy and light chains comprise binding domains that interact with antigens. The three CDRs of VH are denoted as HCDR1, HCDR2 and HCDR3, respectively, and may also be denoted as VH CDR1, VH CDR2 and VH CDR3, respectively; and the three CDRs of VL are denoted as LCDR1, LCDR2 and LCDR3, respectively, and may also be denoted as VL CDR1, VL CDR2 and VL CDR3, respectively. Examples of the antigen-binding proteins include, but are not limited to, antibodies, antigen-binding fragments (Fab, Fab', F(ab)2, Fv fragment, F(ab')2, scFv, di-scFv and/or dAb), immunoconjugates, multispecific antibodies (e.g., bispecific antibodies), antibody fragments, antibody derivatives, antibody analogs or fusion proteins, etc., as long as they exhibit the desired antigen-binding activity.

In the present application, the amino acid sequences of the CDRs are shown according to the Chothia scheme. However, it is well known to those skilled in the art that the CDRs of an antibody can be defined in the art using a variety of methods, such as the Kabat scheme based on sequence variability (see Kabat et al., Sequences of Proteins of Immunological Interest, Fifth Edition, National Institutes of Health (U.S.), Bethesda, Maryland (1991)), and the Chothia scheme based on the location of the structural loop regions (see J Mol Biol 273: 927-48, 1997). In the technical solution of the present invention, the Combined scheme comprising the Kabat scheme and the Chothia scheme can also be used to determine the amino acid residues in a variable domain sequence. The Combined scheme combines the Kabat scheme with the Chothia scheme to obtain a larger range. See the table below for details. It will be understood by those skilled in the art that unless otherwise specified, the terms "CDR" and "complementary determining region" of a given antibody or a region (e.g., variable region) thereof are considered as encompassing complementary determining regions as defined by any one of the above known schemes described herein. Although the scope claimed in the present invention is the sequences shown based on the Chothia scheme, the amino acid sequences corresponding to the other schemes for numbering CDRs shall also fall within the scope of the present invention.

Table 1: Method for the definition of the CDR of the antibodies in the present application

|       | Kabat    | Chothia  | Combined |
|-------|----------|----------|----------|
| LCDR1 | L24--L34 | L24--L34 | L24-L34  |
| LCDR2 | L50--L56 | L50--L56 | L50-L56  |
| LCDR3 | L89--L97 | L89--L97 | L89-L97  |
| HCDR1 | H31--H35 | H26--H32 | H26-H35  |
| HCDR2 | H50--H65 | H52--H56 | H50-H65  |
| HCDR3 | H95--H102| H95--H102| H95-H102 |

Laa-Lbb can refer to an amino acid sequence from position aa (the Chothia scheme) to position bb (the Chothia scheme) beginning at the N-terminus of the light chain of the antibody. Haa-Hbb can refer to an amino acid sequence from position aa (the Chothia scheme) to position bb (the Chothia scheme) beginning at the N-terminus of the heavy chain of the antibody. For example, L24-L34 can refer to the amino acid sequence from position 24 to position 34 according to the Chothia scheme beginning at the N-terminus of the light chain of the antibody; H26-H32 can refer to the amino acid sequence from position 26 to position 32 according to the Chothia scheme beginning at the N-terminus of the heavy chain of the antibody. It should be known to those skilled in the art that there are positions where insertion sites are present in numbering CDRs with the Chothia scheme (see http://bioinf.org.uk/abs/).

In the present application, the term "monoclonal antibody" generally refers to an antibody obtained from a population of substantially homogeneous antibodies, that is, the individual antibodies in the population are identical except for a small amount of natural mutations that may exist. Monoclonal antibodies are generally highly specific for a single antigenic site. Moreover, unlike conventional polyclonal antibody preparations (which generally have different antibodies directed against different determinants), each monoclonal antibody is directed against a single determinant on the antigen. In addition to their specificity, monoclonal antibodies have the advantage that they can be synthesized by hybridoma culture without contamination by other immunoglobulins. The modifier "monoclonal" indicates the characteristic of the antibody obtained from a population of substantially homogeneous antibodies, and is not to be construed as requiring production of the antibody by any particular method. For example, monoclonal antibodies used according to the present invention can be prepared in hybridoma cells or can be prepared by the recombinant DNA method.

In the present application, the term "fully human antibody" generally refers to an antibody that is expressed by a genetically engineered antibody gene-deleted animal into which the entire gene that encodes an antibody in human is transferred. All parts of the antibody (including the variable and constant regions of the antibody) are encoded by genes of human origin. The fully human antibody can greatly reduce the immune side effects caused in the human body by the heterologous antibody. Methods for obtaining fully human antibodies in the art can include phage display, transgenic mice, and the like.

In the present application, the term "specifically bind to" generally refers to that an antibody binds to an epitope via its antigen-binding domain, and that the binding requires some complementarity between the antigen-binding domain and the epitope. According to this definition, an antibody is said to "specifically bind to" an antigen when the antibody more easily binds to an epitope via its antigen-binding domain than binds to a random, unrelated epitope. "Epitope" refers to a specific atomic group (e.g., saccharide side chain, phosphoryl, sulfonyl) or an amino acid on an antigen that binds to an antigen-binding protein (e.g., an antibody).

In the present application, the term "Fab" generally refers to the portion of a conventional antibody (e.g., IgG) that binds to an antigen, including the heavy chain variable region VH, the light chain variable region VL, the heavy chain constant region domain CH1 and the light chain constant region CL of the antibody. In conventional antibodies, the C-terminus of VH is linked to the N-terminus of CH1 to form a heavy chain Fd fragment, the C-terminus of VL is linked to the N-terminus of CL to form a light chain, and the C-terminus of CH1 is further linked to the hinge region and other constant region domains of the heavy chain to form a heavy chain. In some embodiments, "Fab" also refers to a variant structure of the Fab. For example, in certain embodiments, the C-terminus of VH is linked to the N-terminus of CL to form one polypeptide chain, and the C-terminus of VL is linked to the N-terminus of CH1 to form another polypeptide chain, in which case a Fab (cross VH/VL) structure is formed; in certain embodiments, CH1 of the Fab is not linked to the hinge region, but rather the C-terminus of CL is linked to the hinge region of the heavy chain, in which case a Fab (cross Fd/LC) structure is formed.

In the present application, the term "Fd" generally refers to a fragment formed by linking a heavy chain variable region VH and a heavy chain constant region domain CH1, which also contains some amino acids in the heavy chain hinge region to form an interchain disulfide bond with the constant region of the antibody light chain.

In the present application, the term "VH" generally refers to the heavy chain variable region VH domain of an antibody, i.e., the heavy chain variable region VH of a conventional antibody (H2L2 structure) from human or other animals, the heavy chain variable region VHH of a heavy-chain antibody (HCAb structure) from animals such as those of Camelidae species, or the heavy chain variable region VH of a fully human heavy-chain antibody (HCAb structure) produced by a Harbour HCAb transgenic mouse.

In the present application, the term "LC" generally refers to the light chain of a conventional antibody, which includes a light chain variable region VL and a light chain constant region CL. The antibody light chains of humans, mice, and other mammals are further classified into two types, such as $\kappa$ (kappa) and $\lambda$ (lambda), which result from the rearrangement of gene segments at loci such as IGK and IGL located on different chromosomes. Based on the difference between $\kappa$ and $\lambda$, the variable regions of the light chains are further classified into $V\kappa$ and $V\lambda$, and the corresponding constant regions of the light chains are $C\kappa$ and $C\lambda$, respectively. Generally, the linkages between VL and CL are of same types. That is, $V\kappa$ combines with $C\kappa$ to form a $\kappa$ light chain, and $V\lambda$ combines with $C\lambda$ to form a $\lambda$ light chain. However, in some specific embodiments of the present invention, the linkages between VL and CL can be of different types. For example, $V\kappa$ may combine with $C\lambda$ to form a $V\kappa C\lambda$ light chain, and $V\lambda$ may combine with $C\kappa$ to form a $V\lambda C\kappa$ light chain.

In the present application, the term "VL" generally refers to the variable domain VL of the light chain of an antibody. According to the type of light chain ($\kappa$ or $\lambda$), VL can be further classified into $V\kappa$ and $V\lambda$. In some specific embodiments, VL may be a general term encompassing $V\kappa$ and $V\lambda$. In some other specific embodiments, VL may specifically refer to $V\kappa$. In still other specific embodiments, VL may specifically refer to $V\lambda$.

In the present application, the term "CL" generally refers to the constant domain CL of the light chain of an antibody. Depending on the type of light chain ($\kappa$ or $\lambda$), CL can be further classified into $C\kappa$ and $C\lambda$. In some specific embodiments, CL may be a general term encompassing $C\kappa$ and $C\lambda$. In some other specific embodiments, CL may specifically refer to $C\kappa$. In still other specific embodiments, CL may specifically refer to $C\lambda$.

In the present application, the term "scFv" generally refers to a single chain variable fragment or a single chain antibody, which includes the heavy chain variable region VH, the light chain variable region VL of an antibody, and a flexible linker peptide linking VH and VL. An scFv contains a polypeptide chain, which, from the amino terminus to the carboxy terminus, comprises VH-L-VL or VL-L-VH; wherein L is a flexible linker peptide of varying lengths. For example, (G4S)3 is a commonly used flexible linker peptide composed of 12 glycines and 3 serines arranged at intervals.

In the present application, the term "antigen-binding fragment" generally refers to any protein functional region that can specifically bind to an antigen. It can be a "Fab", a "VH", or other antigen-binding forms (such as a derived protein structure of lipocalins, neural cell adhesion molecule (NCAM), fibronectin, DARPins, etc.).

In the present application, the term "Fab-Fab" generally refers to a series-connection structure of bispecific antigen-binding fragments composed of three polypeptide chains as shown in (A) in Fig. 4. It comprises three polypeptide chains: a first polypeptide chain comprising VH_A-CH1 from the amino terminus to the carboxyl terminus, a second polypeptide chain comprising VL_A-CL-L-VH_B-CH1 from the amino terminus to the carboxyl terminus, and a third polypeptide chain comprising VL_B-CL from the amino terminus to the carboxyl terminus. In such embodiments, VH_A and VL_A are the heavy chain variable region and light chain variable region of antibody A, respectively; VH_B and VL_B are the heavy chain variable region and light chain variable region of antibody B, respectively; CL is a light chain constant domain; CH1 is the first domain of a heavy chain constant region; and L is a linker peptide. The VL may include a $V\kappa$ and/or

Vλ; the CL may include a Cκ and/or Cλ; and the CH1 may be a CH1 region of human IgG1, IgG2, or IgG4. The first polypeptide chain comprises the heavy chain Fd of the Fab portion of an antibody A (i.e., Fab-A); the third polypeptide chain comprises the light chain of the Fab portion of an antibody B (i.e., Fab-B); and the second polypeptide chain comprises the light chain of Fab-A and the heavy chain Fd of Fab-B.

In the present application, the term "κ/λ Fab-Fab" generally refers to a series-connection structure of bispecific antigen-binding fragments composed of three polypeptide chains as shown in Fig. 6(A) and Fig. 5(A), and its derived structures. It comprises three polypeptide chains: A first polypeptide chain comprising VH_A-CH1 from the amino terminus to the carboxy terminus, a third polypeptide chain comprising VL_B-CL from the amino terminus to the carboxy terminus, wherein the CL can be Cκ or Cλ; and a second polypeptide chain, also known as the "long chain", comprising VL_A-CL-L-VH_B-CH1, or, VH_B-CH1-L-VL_A-CL from the amino terminus to the carboxy terminus. In such embodiments, VH_A and VL_A are the heavy chain variable region and light chain variable region of antibody A, respectively; VH_B and VL_B are the heavy chain variable region and light chain variable region of antibody B, respectively; CL is a light chain constant domain; CH1 is the first domain of a heavy chain constant region; and L is a linker peptide. The VL may include a Vκ and/or Vλ; the CL may include a Cκ and/or Cλ; and the CH1 may be a CH1 region of human IgG1, IgG2, or IgG4. The above described "κ/λ Fab-Fab" has the following structural characteristics: (1) The second polypeptide chain (i.e., the "long chain") comprises the light chain variable region of one antibody and the heavy chain variable region of another antibody. (2) The second polypeptide chain (i.e., the "long chain") can only comprise one light chain constant region. (3) The third polypeptide chain comprises the light chain variable region of one antibody and one light chain constant region. (4) The light chain constant region of the second polypeptide chain (i.e., the "long chain") and the light chain constant region of the third polypeptide chain must be of different types (Cκ or Cλ). For example, if the light chain constant region of the second polypeptide chain is Cκ, the light chain constant region of the third polypeptide chain must be Cλ. Alternatively, if the light chain constant region of the second polypeptide chain is Cλ, the light chain constant region of the third polypeptide chain must be Cκ. (5) The type of the light chain constant region of the second polypeptide chain (i.e., the "long chain") determines which kind of affinity chromatography method is used to purify the target protein. For example, if the second polypeptide chain (i.e., the "long chain") contains the Cκ region, κ-chain specific affinity capture is adapted for obtaining the purified κ/λ Fab-Fab protein. Alternatively, if the second polypeptide chain (i.e., the "long chain") contains the Cλ region, λ-chain specific affinity capture is adapted for obtaining the purified κ/λ Fab-Fab protein.

In most specific embodiments of the present application, a "Fab-Fab" specifically refers to the structure of "κ/λ Fab-Fab".

In the present application, the terms "κ-chain specific affinity chromatography" or "κ-chain specific affinity capture" generally refer to the use of an affinity chromatography medium that specifically recognizes human κ light chains to capture and separate the target protein containing a Cκ region. For example, the affinity medium of KappaSelect (Cytiva, #17545801) can specifically bind to the Cκ region of antibodies, so it can be utilized to capture and separate the target protein containing Cκ.

In the present application, the terms "λ-chain specific affinity chromatography" or "λ-chain specific affinity capture" generally refer to the use of an affinity chromatography medium that specifically recognizes human λ light chains to capture and separate the target protein containing a Cλ region. For example, the affinity medium of LambdaFabSelect (Cytiva, #17548201) can specifically bind to the Cλ region of antibodies, so it can be utilized to capture and separate the target protein containing Cλ.

In the present application, the term "B7H4" generally refers to the V-Set domain-containing T-cell activation inhibitor 1 (also known as VTCN1), a functional variant thereof and/or a functional fragment thereof. The sequence of B7H4 is known in the art. For example, the sequence of an exemplary full-length human B7H4 can be found under Uniprot accession No. Q7Z7D3; the sequence of an exemplary full-length cynomolgus monkey B7H4 can be found under NCBI accession No. XP_005542249; and the sequence of an exemplary full-length mouse B7H4 can be found under Uniprot accession No. Q7TSP5. Antibodies targeting B7H4 are known in the art. For example, an antibody 1D11v1.9_varC2 targeting B7H4 is disclosed in Patent WO2016040724.

In the present application, the term "PSMA" generally refers to prostate-specific membrane antigen (also known as FOLH1, glutamate carboxypeptidase 2), a functional variant thereof and/or a functional fragment thereof. The sequence of PSMA is known in the art. For example, the sequence of an exemplary full-length human PSMA can be found under Uniprot accession No. Q04609. And the sequence of an exemplary full-length cynomolgus monkey PSMA can be found under NCBI accession No. XP_005579379. Antibodies targeting PSMA are known in the art. For example, pasotuxizumab is a bispecific antibody targeting PSMA and CD3, and its sequence can be obtained from the IMGT database.

In the present application, the term "BCMA" generally refers to tumor necrosis factor receptor superfamily member 17 (also known as CD269, or B-cell maturation protein), a functional variant thereof and/or a functional fragment thereof. The sequence of BCMA is known in the art. For example, the sequence of an exemplary full-length human BCMA can be found under Uniprot accession No. Q02223. And the sequence of an exemplary full-length cynomolgus monkey BCMA can be found under NCBI accession No. XP_005591343. Antibodies targeting BCMA are known in the art. For example, belantamab is an antibody targeting BCMA, and its sequence can be obtained from the IMGT database.

In the present application, the term "ROR1" generally refers to the inactivated tyrosine-protein kinase transmembrane receptor ROR1 (also known as NTRKR1), a functional variant thereof and/or a functional fragment thereof. The sequence of ROR1 is known in the art. For example, the sequence of an exemplary full-length human ROR1 can be found under Uniprot accession No. Q01973. And the sequence of an exemplary full-length cynomolgus monkey ROR1 can be found under NCBI accession No. XP_015290264. Antibodies targeting ROR1 are known in the art. For example, an antibody R11H18L9 targeting ROR1 is disclosed in Patent WO2016094873.

In the present application, the term "CD137" generally refers to tumor necrosis factor receptor superfamily member 9 (also known as 4-1BB receptor, or 4-1BBL receptor), a functional variant thereof, and/or a functional fragment thereof. The sequence of CD137 is known in the art. For example, the sequence of an exemplary full-length human CD137 can be found under Uniprot accession No. Q07011. And the sequence of an exemplary full-length cynomolgus monkey CD137 can be found under NCBI accession No. XP_005544945. Antibodies targeting CD137 are known in the art. For example, urelumab and utomilumab are both agonistic antibodies targeting CD137, and their sequences can be obtained from the IMGT database.

In the present application, the term "CD3" generally refers to the TCR/CD3 receptor protein complex on T cells. The specificity of T cell responses is mediated by the recognition of pMHC by the molecular complex of TCR and CD3. A TCR is a heterodimer composed of two different transmembrane polypeptide chains, and the peptide chain is selected from four types of $\alpha$, $\beta$, $\gamma$, or $\delta$. According to the different combinations of the polypeptide chains, TCRs are classified into TCR$\alpha\beta$ and TCR$\gamma\delta$. CD3 has different transmembrane polypeptide chains, namely $\gamma$, $\delta$, $\varepsilon$, or $\zeta$. These peptide chains interact with each other to form homodimers or heterodimers, and serve as portion of the TCR-CD3 complex. Since the cytoplasmic region of the TCR peptide chain is very short, it is generally believed that the activation signal generated by TCR antigen recognition is transduced into T cells by the CD3 peptide chain. Antibodies targeting the CD3 complex are known in the art. For example, Clone SP34 and Clone OKT3 are both known agonistic antibodies targeting CD3. And multiple humanized antibody sequences derived from SP34 are disclosed in Patent WO2021063330.

In the present application, the term "CD3E" generally refers to the $\varepsilon$ peptide chain of "CD3". The sequence of CD3E is known in the art. For example, the sequence of an exemplary full-length human CD3E can be found under Uniprot accession No. P07766. And the sequence of an exemplary full-length cynomolgus monkey CD3E can be found under Uniprot accession No. Q95LI5.

In the present application, the term "CD16A" generally refers to Fc$\gamma$ receptor IIIA (Fc$\gamma$RIIIA) (also known as Fc-gamma RIII-alpha, Fc-gamma RIIIa, or FCGR3A), a functional variant thereof, and/or a functional fragment thereof. The sequence of CD16A is known in the art. For example, the sequence of an exemplary full-length human CD16A can be found under Uniprot accession No. P08637. Antibodies targeting CD16A are known in the art. For example, an antibody targeting CD16A is disclosed in Patent WO2018158349.

In the present application, the term "HEL" generally refers to hen egg white lysozyme, a functional variant thereof, and/or a functional fragment thereof. The sequence of HEL is known in the art. For example, the sequence of an exemplary full-length hen egg white lysozyme can be found under Uniprot accession No. P00698. Antibodies targeting HEL are known in the art. For example, Clone HYHEL-5 is an antibody targeting HEL, and its sequence can be obtained from the AbYsis database or the PDB database (PDB ID: 1BQL).

In the present application, the term "ALB" generally refers to Albumin, a functional variant thereof, and/or a functional fragment thereof. The sequence of ALB is known in the art. For example, the sequence of an exemplary full-length human ALB can be found under Uniprot accession No. P02768; the sequence of an exemplary full-length mouse ALB can be found under Uniprot accession No. P07724; and the sequence of an exemplary full-length rat ALB can be found under Uniprot accession No. P02770. Antibodies targeting ALB are known in the art. For example, a nanobody Alb23 targeting ALB is disclosed in Patent WO2012175400.

## Examples

The present invention is further illustrated by the following examples, which are not intended to limit the present invention to the described scope. The examples do not include detailed descriptions of conventional methods, such as those methods for constructing vectors and plasmids, methods for inserting genes encoding proteins into such vectors and plasmids, or methods for introducing plasmids into host cells. Such methods are well known to those skilled in the art and are described in numerous publications. Experimental procedures without specified conditions in the following examples are selected according to conventional procedures and conditions, or according to commercial instructions.

## Example 1 Design of a "plug-and-play" "$\kappa/\lambda$" Fab-Fab structure

Designed in this Example was a universal technology for generating bispecific antibody molecules that can: prepare any two conventional IgG antibodies into a bivalent bispecific antibody with a "1+1" structure, retaining the Fab structure without introducing an scFv structure through modification; minimize the requirement of protein engineering work to achieve "plug-and-play"; use existing protein purification methods to effectively separate the main product and by-products; and be easily extended to other multispecific structures.

one feasible method is to connect the antigen-binding domain Fab-A derived from IgG A and the antigen-binding domain Fab-B derived from IgG B in a head-to-tail manner to form the Fab-Fab series-connection structure as shown in Fig. 1. If Fab-A is located at the amino terminus of Fab-B, Fab-A can also be referred to as the N-terminal Fab, while Fab-B as the C-terminal Fab.

Multiple specific implementations were provided for constructing the antigen-binding protein with a Fab-Fab series-connection structure.

Fig. 2 shows the first Solution. The antigen-binding protein was composed of two polypeptide chains: a first polypeptide chain comprising VH_A-CH1-L1-VH_B-CH1 from the amino terminus to the carboxy terminus, and a second polypeptide chain comprising VL_A-CL-L2-VL_B-CL from the amino terminus to the carboxy terminus. Wherein, VH_A and VL_A were the heavy chain variable region and light chain variable region of antibody A respectively; VH_B and VL_B were the heavy chain variable region and light chain variable region of antibody B respectively; CL was a light chain constant domain; CH1 was the first domain of a heavy chain constant region; and L1 and L2 were a first linker peptide and a second linker peptide respectively, which can be glycine or serine-rich flexible polypeptides with a length of 0 or other lengths. The VL may include a Vκ and/or Vλ; the CL may include a Cκ and/or Cλ; and the CH1 may be a CH1 region of human IgG1, IgG2, or IgG4. In this Solution, two linker peptides were used to link the two C-terminus of Fab-A with the two N-terminus of Fab-B respectively to form two polypeptide chains. In this structure, the presence of two linker peptides may restrict the relative positions of Fab-A and Fab-B. Moreover, due to the influence of potential steric hindrance, the antigen-binding site of Fab-B at the C-terminus could be affected by the two linker peptides and Fab-A at the N-terminus, and thus its antigen-binding ability may be severely weakened. To reduce these restrictions, the two linker peptides needed to be optimized to minimize their impact on the antigen-binding ability of the two Fabs. However, the optimization of such linker peptides may not be universally applicable, making it difficult to achieve arbitrary combinations of Fabs. Therefore, it was more preferable to allow the Fab at the C-terminus to retain the necessary spatial freedom.

Fig. 3 shows the second Solution. The antigen-binding protein (Fig. 3(A)) was compsed of three polypeptide chains: a first polypeptide chain comprising VL_A-CL from the amino terminus to the carboxyl terminus, a second polypeptide chain comprising VH_A-CH1-L-VH_B-CH1 from the amino terminus to the carboxyl terminus, and a third polypeptide chain comprising VL_B-CL from the amino terminus to the carboxyl terminus. Wherein, VH_A and VL_A, VH_B and VL_B, CH1 and CL were as previously described; and L was a linker peptide. In this solution, one of the polypeptide chains was formed by linking the C-terminus of the heavy chain CH1 of Fab-A to the N-terminus of the heavy chain VH of Fab-B with only one linker peptide, while the light chain of Fab-A (VL_A-CL) and the light chain of Fab-B (VL_B-CL) were two independent polypeptide chains. The N-terminus of the light chain of Fab-B was connected only to Fab-A at the N-terminus of the heavy chain VH, with no connections to other peptides or domains. Thus, Fab-B located at the C-terminus retained a relatively large degree of spatial freedom, making it easier to bind to the corresponding antigen. However, on the other hand, due to the diversity of the combination of three peptide chains, by-products were formed in addition to the antigen-binding protein. The main by-product was the mismatched product formed by the exchange of the two light chains of Fab-A and Fab-B (Fig. 3(B)), i.e., the light chain of Fab-A (VL_A-CL) paired with the heavy chain Fd of Fab-B (VH_B-CH1), and the light chain of Fab-B (VL_B-CL) paired with the heavy chain Fd of Fab-A (VH_A-CH1). The resulting mismatched by-product lost its original antigen-binding specificity. Moreover, the molecular weight and structure of the by-product were almost the same as those of the correctly paired target protein, making it difficult to separate the two by conventional methods. Therefore, a more preferable structure was required to facilitate the separation of the target protein from by-products.

Fig. 4 shows the third Solution. The antigen-binding protein (Fig. 4(A)) consists of three polypeptide chains: a first polypeptide chain comprising VH_A-CH1 from the amino terminus to the carboxyl terminus, a second polypeptide chain comprising VL_A-CL-L-VH_B-CH1 from the amino terminus to the carboxyl terminus, and a third polypeptide chain comprising VL_B-CL from the amino terminus to the carboxyl terminus. In this Solution, VH_A and VL_A, VH_B and VL_B, CH1 and CL were as previously described; and L was a linker peptide. In this solution, one of the polypeptide chains was formed by linking the C-terminus of the light chain CL of Fab-A to the N-terminus of the heavy chain VH of Fab-B with only one linker peptide, while the heavy chain Fd of Fab-A (VH_A-CH1) and the light chain of Fab-B (VL_B-CL) were two independent polypeptide chains. The N-terminus of the light chain of Fab-B was connected only to Fab-A at the N-terminus of the heavy chain VH, with no connections to other peptides or domains. Thus, Fab-B located at the C-terminus retained a relatively large degree of spatial freedom, making it easier to bind to the corresponding antigen. However, on the other hand, due to the diversity of the combination of three peptide chains, by-products were formed in addition to the antigen-binding protein. The main by-product was the mismatched Fab formed by the pairing of the heavy chain Fd of Fab-A (VH_A-CH1) and the light chain of Fab-B (VL_B-CL) (Fig. 4(B)). The resulting mismatched by-product lost its original antigen-binding specificity. The molecular weight of this by-product was different from that of the correctly paired target protein (approximately 50KDa vs. approximately 100KDa), allowing separation of the target protein from the by-product by molecular size exclusion chromatography (SEC). However, SEC was not suitable for the high-efficiency large-scale protein purification. Therefore, the optimization of the structure was required to be suitable for high-efficiency large-scale protein purification methods, such as affinity capture chromatography.

On the basis that the target antigen-binding protein (Fig. 4(A)) comprised two CL regions and the by-product (Fig. 4(B)) comprised one CL region, designed in the present invention were novel structural combinations allowing efficiently separating the target protein from the by-product using CL-specific affinity purification method.

The light chains of human antibodies are classified into two types, κ (kappa) and λ (lambda). The variable regions of the light chains VL are Vκ and Vλ, respectively, while the constant regions of the light chains CL are Cκ and Cλ, respectively. KappaSelect (Cytiva, #17545801) is a type of affinity medium that specifically binds to the Cκ region of antibodies, and can be used to capture and separate the target protein containing Cκ. LambdaFabSelect (Cytiva, #17548201) is a type of affinity medium that specifically binds to the Cλ region of antibodies, and can be used to capture and separate the target protein containing Cλ.

If the CL of the second polypeptide chain and the CL of the third polypeptide chain of the target antigen binding protein (Fig. 4(A)) are of different types (Cκ or Cλ) (for example, the CLs of the second polypeptide chain and the third polypeptide chain are Cκ and Cλ, respectively, or Cλ and Cκ, respectively), then the target antigen-binding protein (Fig. 4(A)) and the by-product (Fig. 4(B)) can be efficiently separated using CL-specific affinity purification method. In such embodiments, the CL type of the second polypeptide chain determines which kind of affinity chromatography method is used for purifying the target protein. For example, if the second polypeptide chain contains a Cκ region, KappaSelect (Cytiva, #17545801) can be used to purify the target protein, while the by-products cannot be affinity-captured because they do not contain a Cκ region (Fig. 5). Alternatively, if the second polypeptide chain contains a Cλ region, Lambda-FabSelect (Cytiva, #17548201) can be used to purify the target protein, while the by-products cannot be affinity-captured because they do not contain a Cλ region.

Fig. 5 shows a structural form and preparation method of the "κ/λ" Fab-Fab series-connection bispecific antigen-binding protein designed in the present application. In such embodiments, based on the structure of the three polypeptide chains as shown in Fig. 4(A), a Cκ or Cλ domain was introduced into the second and third polypeptide chains, respectively.

As shown in Fig. 5(A), the "κ/λ" Fab-Fab structure was composed of three polypeptide chains: a first polypeptide chain comprising VH_A-CH1 from the amino-terminal to the carboxy-terminal; a second polypeptide chain comprising VL_A-Cκ-L-VH_B-CH1 from the amino-terminal to the carboxy-terminal; and a third polypeptide chain comprising VL_B-Cλ from the amino-terminal to the carboxy-terminal. The VL may comprise a Vκ and/or a Vλ. The linker peptide L is optional.

Fig. 5(B-C) shows the preparation method for the "κ/λ" Fab-Fab antigen-binding protein. Specifically, plasmids encoding the three polypeptide chains were transfected into host cells; κ-chain specific affinity chromatography medium, such as KappaSelect (Cytiva, #17545801), can be used to capture the expressed target main product consisting of three peptide chains containing one Cκ domain and one Cλ domain; the chain-mismatched by-products not containing a Cκ domain flowed through as an impurity without being captured by the κ-chain specific affinity chromatography medium. See Example 2 for the specific preparation method.

Furthermore, various "κ/λ" Fab-Fab structures can be derived by adjusting the relative positions of Fab-A and Fab-B and the position of the Cκ or Cλ domain on the second polypeptide chain (i.e., the "long chain").

Fig. 6 shows four structural forms and the adopted preparation methods of the "κ/λ" Fab-Fab series-connection bispecific antigen-binding protein designed in the present application. (A) Configuration-I (i.e., the structure as shown in Fig. 5): The part of the N-terminal Fab was formed by a Cκ domain located in the second domain of the second polypeptide chain (i.e., the long chain); the part of the C-terminal Fab was formed by a Cλ domain located in the second domain of the third polypeptide chain; and κ-chain specific affinity capture was adopted for the main product. (B) Configuration-II: The part of the N-terminal Fab was formed by a Cλ domain located in the second domain of the second polypeptide chain (i.e., the long chain); the part of the C-terminal Fab was formed by a Cκ domain located in the second domain of the third polypeptide chain; and λ-chain specific affinity capture was adopted for the main product. (C) Configuration-III: The part of the C-terminal Fab was formed by a Cκ domain located in the fourth domain of the second polypeptide chain (i.e., the long chain); the part of the N-terminal Fab was formed by a Cλ domain located in the second domain of the third polypeptide chain; and κ-chain specific affinity capture was adopted for the main product. (D) Configuration-IV: The part of the C-terminal Fab was formed by a Cλ domain located in the fourth domain of the second polypeptide chain (i.e., the long chain); the part of the N-terminal Fab was formed by a Cκ domain located in the second domain of the third polypeptide chain; and λ-chain specific affinity capture was adopted for the main product.

The "κ/λ" Fab-Fab of the present invention has the following structural characteristics:

(1) The second polypeptide chain (i.e., the "long chain") comprises a light chain variable region of one antibody and a heavy chain variable region of another antibody.
(2) The second polypeptide chain comprises only one light chain constant region.
(3) The third polypeptide chain comprises a light chain variable region of an antibody and a light chain constant region.

(4) The light chain constant regions of the second polypeptide chain and the third polypeptide chain are of different types (Cκ or Cλ). For example, if the light chain constant region of the second polypeptide chain is Cκ, the light chain constant region of the third polypeptide chain is Cλ; alternatively, if the light chain constant region of the second polypeptide chain is Cλ, the light chain constant region of the third polypeptide chain is Cκ.

(5) The type of the light chain constant region of the second polypeptide chain determines which kind of affinity chromatography method is used for purifying the "κ/λ" Fab-Fab protein. For example, if the second polypeptide chain contains a Cκ region, κ-chain specific affinity capture is adapted for purifying the target protein; alternatively, if the second polypeptide chain contains a Cλ region, λ-chain specific affinity capture is adapted for purifying the target protein.

## Example 1.1 Preparation of a "κ/λ" Fab-Fab with an IgG/κ antibody and an IgG/λ antibody.

Fig. 7 shows various Solutions for preparing the "κ/λ" Fab-Fab series-connection bispecific antibody described herein using an IgG antibody A with a κ light chain and an IgG antibody B with a λ light chain. Respectively, Solutions (1), (2), (3), and (4) instanced the Configuration-I, Configuration-II, Configuration-III, and Configuration-IV as shown in Fig. 6, with VL_A therein designated as Vκ_A and VL_B designated as Vλ_B. In Solution (1) and Solution (3), the linkages between VL and CL were of the same type. That is, Vκ_A was linked to Cκ to form a κ light chain of Fab-A, and Vλ_B was linked to Cλ to form a λ light chain of Fab-B. In Solution (2) and Solution (4), the linkages between VL and CL were of different types. That is, Vκ_A was linked with Cλ to form a hybrid light chain with a Vκ_Cλ of Fab-A, or Vλ_B was linked with Cκ to form a hybrid light chain with a Vκ-Cλ. KappaSelect (Cytiva, #17545801) may be used to purify the target protein in Solution (1) and Solution (3); and LambdaFabSelect (Cytiva, #17548201) may be used to purify the target protein in Solution (2) and Solution (4).

## Example 1.2 Preparation of a "κ/λ" Fab-Fab with two IgG/κ antibodies

Fig. 8 shows various Solutions for preparing the "κ/λ" Fab-Fab series-connection bispecific antibody described herein using an IgG antibody A with a κ light chain and an IgG antibody B with a κ light. Respectively, Solutions (5), (6), (7), and (8) instanced the Configuration-I, Configuration-II, Configuration-III, and Configuration-IV as shown in Fig. 6, with VL_A therein designated as Vκ_A and VL_B designated as Vκ_B.

One of the Cκ was replaced with Cλ in order to separate the target protein from the by-product using different affinity chromatography media (κ-chain or λ-chain specific affinity capture). In Solution (5) and Solution (7), the Cκ of Fab-B was replaced with Cλ, so that its light chain had a Vκ-Cλ configuration. In Solution (6) and Solution (8), the Cκ of Fab-A was replaced with Cλ, so that its light chain had a Vκ-Cλ configuration. KappaSelect may be used to purify the target protein in Solution (5) and Solution (7); and LambdaFabSelect may be used to purify the target protein in Solution (6) and Solution (8).

## Example 1.3 Preparation of a "κ/λ" Fab-Fab with two IgG/λ antibodies

Fig. 9 shows various Solutions for preparing the "κ/λ" Fab-Fab series-connection bispecific antibody described herein using an IgG antibody A with a λ light chain and an IgG antibody B with a λ light. Respectively, Solutions (9), (10), (11), and (12) instanced the Configuration-I, Configuration-II, Configuration-III, and Configuration-IV as shown in Fig. 6 with VL_A therein designated as Vλ_A and VL_B designated as Vλ_B.

One of the Cλ was replaced with Cκ in order to separate the target protein from the by-product using different affinity chromatography media (κ-chain or λ-chain specific affinity capture). In Solution (9) and Solution (11), the Cλ of Fab-A was replaced with Cκ, so that its light chain had a Vλ-Cκ configuration. In Solution (10) and Solution (12), the Cλ of Fab-B was replaced with Cκ, so that its light chain had a Vλ-Cκ configuration. KappaSelect may be used to purify the target protein in Solution (9) and Solution (11); and LambdaFabSelect may be used to purify the target protein in Solution (10) and Solution (12).

## Example 1.4 Extension of the serum half-life of the "κ/λ" Fab-Fab with serum albumin

The serum half-life of the "κ/λ" Fab-Fab structure lacking a Fc region was expected to be shorter than that of normal IgG. Currently, there have been various means to improve the serum half-life of protein molecules. For example, polyethylene glycol modification (PEGylation) may be used to further improve the serum half-life by increasing the molecular hydration radius, or an antibody against human serum albumin (HSA) or serum albumin (ALB) may be coupled to further improve the serum half-life by utilizing the FcRn-mediated recycling mechanism. For example, the single-domain antibody Alb23 is a known single-domain antibody that can specifically bind to serum albumin and has cross-reactivity among species such as human, cynomolgus monkey, and mouse. The serum half-life was increased by further fusing the variable region of Alb23 to the second polypeptide chain (long chain) of the "κ/λ" Fab-Fab structure to construct a trispecific antigen-binding protein containing a "κ/λ" Fab-Fab structure (as shown in Fig. 10(C)).

For example, as described in Example 7, PR000317 is a B7H4xCD3 bispecific antibody with a "κ/λ" Fab-Fab structure, and PR001325 is a B7H4xCD3xALB trispecific antibody containing a "κ/λ" Fab-Fab structure and a "half-life extension module" (serum albumin binding domain). An in vivo pharmacokinetic test in mice demonstrated that PR001325 had a longer serum half-life as compared to PR000317.

In summary, the "κ/λ" Fab-Fab structure of the present application exhibits excellent flexibility and universality. The structure is suitable for preparing any two conventional IgG antibodies into bispecific antibodies, and can be extended to various other structures, with the advantage of "plug and play".

### Example 2 Expression and purification of proteins with a "κ/λ" Fab-Fab structure

This Example describes a preparation method for a protein with a "κ/λ" Fab-Fab structure (Fig. 6) using a mammalian host cell (e.g., human embryonic kidney cell HEK293 or Chinese hamster ovary cell CHO and their derived cells), and techniques such as transient transfection expression, and affinity capture separation and purification.

#### Molecular cloning

The amino acid sequences of the three polypeptide chains of the "κ/λ" Fab-Fab protein were converted into nucleotide sequences using a codon optimization method. And the encoded nucleotide sequences were synthesized and cloned into an expression vector compatible with the host cell.

#### Transient transfection and expression using the 293-F expression system

The FreeStyle™ 293-F cells (Thermo, #R79007) were expanded in FreeStyle™ F17 Expression Medium (Thermo, #A1383504). Before the transient transfection, the cell density was adjusted to $5 \times 10^5$ cells/ml, and the cells were cultured in a shaker at 37°C with 8% $CO_2$ for 24 hours. Then the cell density was adjusted to $1 \times 10^6$ cells/ml. 30 ml of cultured cells were prepared for the transfection. A total of 30 μg (the ratio of plasmids to cells is 1 μg: 1 ml) of plasmids encoding three polypeptide chains were mixed in a certain ratio (e.g., 1:1:1), and dissolved in 1.5 ml of Opti-MEM reduced-serum medium (Thermo, #31985088), and filter-sterilized using a 0.22 μm filter membrane. 1.5 ml of Opti-MEM with dissolved 120 μl of 1 mg/ml PEI (Polysciences, #23966-2) was prepared to obtain a total of 120 μg of PEI. Allow it to stand for 5 minutes. The PEI was slowly added to the plasmid (the ratio of PEI to plasmid is approximately 4:1) and incubated at room temperature for 15 minutes. The plasmid-PEI mixed solution was slowly dropped into the cell culture while shaking the culture flask. The cells were cultured for 5 days in a shaker at 37°C with 8% $CO_2$. The cell viability was observed after 5 days. And the culture was collected when the cell viability was lower than 70%.

#### Transient transfection and expression using the ExpiCHO expression system

The ExpiCHO-S™ cells (Thermo, #A29127) were expanded in ExpiCHO™ Expression Medium (Thermo, #A2910001). One day before the transient transfection, the cell density was adjusted to $3 - 4 \times 10^6$ cells/ml and the cells were cultured overnight in a shaker at 37°C with 8% $CO_2$. The transfection may be carried out when the cell density reached $7 - 10 \times 10^6$ cells/ml and the cell viability was 95-99%. The cell density was adjusted to $6 \times 10^6$ cells/ml. The plasmids encoding three polypeptide chains in a certain ratio (e.g., 1:1:1) were mixed and slowly dissolved into pre-cooled OptiPRO™ Medium (Thermo, #12309019). And the ExpiFectamine™ CHO Transfection Reagent (Thermo, #A29129) was diluted with OptiPRO™ Medium. Then, the diluted plasmid was mixed with the diluted transfection reagent and allowed to stand at room temperature for 1-5 minutes. The mixture of the plasmid and the transfection reagent is slowly transferred into the cell culture flask. The cells were cultured for 8-10 days in a shaker at 37°C with 8% $CO_2$.

#### Selection of purification methods

The protein with a "κ/λ" Fab-Fab structure, such as the configuration as shown in Fig. 6 and derived structures thereof containing this configuration, consists of three polypeptide chains. In such embodiments, the type of light chain constant region of the second polypeptide chain (i.e., "long chain") determines which kind of affinity chromatography method is used for purifying the target protein. For example, if the second polypeptide chain contains a Cκ region, the affinity medium of KappaSelect (Cytiva, #17545801) can be used to purify the target protein, while the by-products cannot be affinity-captured because they do not contain a Cκ region (Fig. 5). Alternatively, if the second polypeptide chain contains a Cλ region, the affinity medium of LambdaFabSelect (Cytiva, #17548201) can be used to purify the target protein.

**Protein purification using KappaSelect**

The cell culture was collected, centrifuged at 4000g for 40 minutes, and then filtered to remove cell debris and impurities to obtain the supernatant. The KappaSelect packing material (Cytiva, #17545801) was pre-equilibrated with PBS buffer. Then, the supernatant was added to a gravity column (Bio-Rad, #7311550) containing KappaSelect. And the column was washed with 20 column volumes of PBS buffer, followed by elution of the target protein with 5-10 column volumes of 0.1M glycine buffer at pH 2.5-3.0. Subsequently, the column was adjusted to neutrality with Tris-HCl at pH 8.0.

**Protein purification using LambdaFabSelect**

The LambdaFabSelect packing material (Cytiva, #17548201) was pre-equilibrated with PBS buffer. Then, the supernatant was added to a gravity column (Bio-Rad, #7311550) containing LambdaFabSelect. And the column was washed with 20 column volumes of PBS buffer, followed by elution of the target protein with 5-10 column volumes of 0.1M acetate buffer at pH 3.5. Subsequently, the column was adjusted to neutrality with Tris-HCl at pH 8.0.

**Quantification and storage**

An ultrafiltration tube (Millipore, #UFC901024) was used to concentrate the solution and exchange the solution to PBS buffer or a buffer containing other agents to obtain a purified protein solution. Then the concentration was determined using NanoDrop (Thermo, NanoDrop™ One). Finally, the solution was sub-packaged and stored for later use.

**Example 3 Analysis of protein purity and aggregates using SEC-HPLC**

In this Example, analytical molecular size-exclusion chromatography (SEC) was used to analyze the purity and aggregate forms of protein samples. An analytical column TSKgel G3000SWx1 (Tosoh Bioscience, #08541, 5 $\mu$m, 7.8 mm x 30 cm) was connected to a high-pressure liquid chromatograph (HPLC) (Agilent Technologies, Agilent 1260 Infinity II) and equilibrated with PBS buffer at room temperature for at least 1 hour. An appropriate amount of protein sample (at least 10 $\mu$g) was filtered through a 0.22 $\mu$m filter membrane and then injected into the system. And the HPLC program was set as follows: The sample was passed through the chromatographic column with a PBS buffer solution at a flow rate of 1.0 ml/min for a maximum of 25 minutes. An analysis report was generated by the HPLC, indicating the retention times of components with different molecular sizes in the sample.

**Example 4 Construction of bispecific (multispecific) antigen-binding proteins with** a "$\kappa/\lambda$" **Fab-Fab structure**

In this Example, various IgG antibodies (Table 2) were used to construct different antigen-binding proteins (Table 4) with a "$\kappa/\lambda$" Fab-Fab structure.

IgG parent monoclonal antibodies or control antibodies used in the present application are listed in Table 2, showing the diversity of IgG antibodies used to construct the "$\kappa/\lambda$" Fab-Fab. These antibodies have different IgG subtypes (IgG1, IgG2, or IgG4). Additionally, they are derived from different species (mouse antibodies, humanized antibodies, or fully human antibodies), and include different light chain types ($\kappa$ and $\lambda$ light chains). A sequence numbering of the amino acid sequences of related antibody molecules is listed in Table 3, and the amino acid sequences of related antibody molecules is listed in Table 8.

The bispecific (multispecific) antigen-binding protein molecules with the structure of "$\kappa/\lambda$" Fab-Fab constructed in the present invention are listed in Table 4. The antigen-binding domains Fab-A and Fab-B of these molecules are derived from the IgG antibodies listed in Table 2. The "$\kappa/\lambda$" Fab-Fab molecules listed in Table 4 have a variety of structures as shown in Fig. 10, including embodiments where the linkages of VL and CL are of different types, for example, the combination of V$\kappa$ and C$\lambda$ to form a V$\kappa$C$\lambda$ light chain (in Fig. 10(B)); as well as embodiments where derived structures with half-life extension modules (Fig. 10(C)). Therefore, Table 4 reflected the structural diversity of the constructed "$\kappa/\lambda$" Fab-Fab antigen-binding proteins. A sequence numbering of the amino acid sequences of the relevant "$\kappa/\lambda$" Fab-Fab antigen-binding proteins is listed in Table 5. A sequence numbering of the CDRs of the corresponding antigen-binding domains (Fab ends) of the "$\kappa/\lambda$" Fab-Fab antigen-binding proteins is listed in Table 6. The amino acid sequences of the relevant "$\kappa/\lambda$" Fab-Fab antigen-binding proteins are listed in Table 9.

The method as described in Example 2 was used to prepare the "$\kappa/\lambda$" Fab-Fab antigen-binding protein listed in Table 4, and the SEC-HPLC method as described in Example 3 was used to analyze the purity and aggregation of the antigen-binding protein samples. The information about the preparation of relevant samples is summarized in Table 7. The results were shown in Table 7. Most "$\kappa/\lambda$" Fab-Fab antigen-binding proteins were obtained with > 90% purity samples after one-step KappaSelect affinity capture purification.

Specifically, PR000317 is a B7H4xCD3 bispecific antibody with a "$\kappa/\lambda$" Fab-Fab structure. The results of SDS-PAGE analysis and SEC-HPLC analysis of PR000317 after one-step KappaSelect affinity capture purification were shown in Fig. 11. SEC-HPLC results demonstrated that the purity of the target protein was 100%. SDS-PAGE results demonstrated that under non-reducing conditions, the target protein exhibited a uniform band near the molecular weight marker of 98 KDa; and under reducing conditions, it exhibited two bands near 50 KDa and less than 36 KDa respectively. The molecular weights represented by these bands were consistent with the calculated molecular weight (94.5 KDa) of the target protein (PR000317) and the molecular weights of the three polypeptide chains (23.7 KDa, 48.1 KDa, and 22.7 KDa, respectively). It was demonstrated that three polypeptide chains were effectively paired to form a protein with a "κ/λ" Fab-Fab structure.

Specifically, PR001325 is a B7H4xCD3xALB trispecific antibody containing a "κ/λ" Fab-Fab structure. It has a serum albumin-binding domain added based on the structure of PR000317. Fig. 12 shows the results of SDS-PAGE analysis and SEC-HPLC analysis of PR001325 after one-step KappaSelect affinity capture purification. SEC-HPLC results demonstrated that the purity of the target protein was 100%.

Specifically, PR001613 is a B7H4xCD16A bispecific antibody with a "κ/λ" Fab-Fab structure. Fig. 13 shows the results of SDS-PAGE analysis and SEC-HPLC analysis of PR001613 after one-step KappaSelect affinity capture purification. SEC-HPLC results demonstrated that the purity of the target protein was 92.9%.

This experiment demonstrated that the preparation processes of "κ/λ" Fab-Fab were relatively simple. Any IgG antibody can be used to easily construct bispecific (multispecific) antigen-binding proteins with "plug-and-play" flexibility.

**Table 2: The parent monoclonal antibodies and control antibodies used in the present application**

| Antibody ID | Target | Species of antibody variable regions | Light chain type | Source of antibody variable region sequences | IgG subtype |
|---|---|---|---|---|---|
| PR000157 | B7H4 | Humaniz ed | κ | Clone 1D11v1.9varC2; Patent WO2016040724: VH (SEQ ID NO: 127); VL (SEQ ID NO: 126) | hIgG1 |
| PR000327 | PSMA | Humaniz ed | κ | The anti-PSMA terminus of pasotuxizumab; INN 9937 | hIgG1 |
| PR000274 | BCMA | Humaniz ed | κ | belantamab; INN 10797 | hIgG1 |
| PR0003 74 | ROR1 | Humaniz ed | κ | Clone R11H18L9; Patent WO2016094873: VH (SEQ ID NO: 252); VL (SEQ ID NO: 250) | hIgG1 |
| PR000326 | HEL | Mouse | κ | Clone HYHEL-5; AbYsis database | hIgG1 |
| PR000260 | CD3 | Mouse | λ | Clone SP34; Patent WO2016071004: VH (SEQ ID NO: 60); VL (SEQ ID NO: 61) | hIgG1 |
| PR000512 | CD3 | Humaniz ed | λ | The humanized variants of Clone SP34; Patent WO2021063330: VH (SEQ ID NO: 51); VL (SEQ ID NO: 58) | hIgG1 |
| PR001848 | CD3 | Humaniz ed | λ | The humanized variants of Clone SP34; Patent WO2021063330: VH (SEQ ID NO: 45); VL (SEQ ID NO: 58) | hIgG1 |
| PR001611 | CD16A | Fully human | λ | Patent WO2018158349: VH (SEQ ID NO: 8); VL (SEQ ID NO: 9) | hIgG1 |
| PR001612 | CD16A | Fully human | λ | Patent WO2018158349: VH (SEQ ID NO: 10); VL (SEQ ID NO: 9) | hIgG1 |
| PR000628 | CD137 | Fully human | κ | urelumab; INN 9365 | hIgG4 |
| PR000483 | CD137 | Fully human | λ | utomilumab; INN 10385 | hIgG2 |
| PR000929 | ALB | Humaniz ed | NA | Single-domain antibody Alb23; Patent WO2012175400: VH (SEQ ID NO: 1) | No Fc |

**Table 3: Sequence numbering table of the sequences of parent monoclonal antibodies and control antibodies and CDR sequences**

| Numberi ng of the antibodies | Light chain | Heavy chain | VL | VH | LCDR 1 | LCDR 2 | LCDR 3 | HCDR 1 | HCDR 2 | HCDR 3 |
|---|---|---|---|---|---|---|---|---|---|---|
| PR000157 | 167 | 154 | 144 | 131 | 81 | 99 | 117 | 12 | 35 | 58 |
| PR000327 | 171 | 158 | 148 | 135 | 85 | 103 | 121 | 16 | 39 | 62 |
| PR000274 | 169 | 156 | 146 | 133 | 83 | 101 | 119 | 14 | 37 | 60 |
| PR000374 | 172 | 159 | 149 | 136 | 86 | 104 | 122 | 17 | 40 | 63 |
| PR000326 | 170 | 157 | 147 | 134 | 84 | 102 | 120 | 15 | 38 | 61 |
| PR000260 | 168 | 155 | 145 | 132 | 82 | 100 | 118 | 13 | 36 | 59 |
| PR000512 | 174 | 161 | 151 | 138 | 82 | 100 | 118 | 13 | 36 | 59 |
| PR001848 | 174 | 166 | 151 | 143 | 82 | 100 | 118 | 21 | 36 | 59 |
| PR001611 | 176 | 164 | 153 | 141 | 89 | 107 | 125 | 12 | 44 | 67 |
| PR001612 | 176 | 165 | 153 | 142 | 89 | 107 | 125 | 12 | 45 | 67 |
| PR000628 | 175 | 162 | 152 | 139 | 88 | 106 | 124 | 19 | 42 | 65 |
| PR000483 | 173 | 160 | 150 | 137 | 87 | 105 | 123 | 18 | 41 | 64 |
| PR000929 | | 163 | | 140 | | | | 20 | 43 | 66 |

**Table 4: Bispecific (multispecific) antibodies with a "κ/λ" Fab-Fab structure constructed in the present application**

| Antibody ID | Target-1 | Fab-A | Targ et-2 | Fab-B | Schematic diagram of the structure in Fig. 10 | VL-CL configurati on of the second polypeptide chain | VL-CL configurati on of the third polypeptide chain |
|---|---|---|---|---|---|---|---|
| PR000317 | B7H4 | PR00015 7 | CD3 | PR000260 | (A) | Vκ-Cκ | Vλ-Cλ |
| PR001325 | B7H4 | PR00015 7 | CD3 | PR000512 | (C) | Vκ-Cκ | Vλ-Cλ |
| PR001197 | PSMA | PR00032 7 | CD3 | PR000512 | (A) | Vκ-Cκ | Vλ-Cλ |
| PR001198 | BCMA | PR00027 4 | CD3 | PR000512 | (A) | Vκ-Cκ | Vλ-Cλ |
| PR001200 | ROR1 | PR00037 4 | CD3 | PR000512 | (A) | Vκ-Cκ | Vλ-Cλ |
| PR001310 | HEL | PR00032 6 | CD3 | PR000512 | (A) | Vκ-Cκ | Vλ-Cλ |
| PR001613 | B7H4 | PR00015 7 | CD16 A | PR001611 | (A) | Vκ-Cκ | Vλ-Cλ |
| PR001614 | B7H4 | PR00015 7 | CD16 A | PR001612 | (A) | Vκ-Cκ | Vλ-Cλ |
| PR001615 | BCMA | PR00027 4 | CD16 A | PR001611 | (A) | Vκ-Cκ | Vλ-Cλ |
| PR001616 | BCMA | PR00027 4 | CD16 A | PR001612 | (A) | Vκ-Cκ | Vλ-Cλ |
| PR001941 | HEL | PR00032 6 | CD13 7 | PR000628 | (B) | Vκ-Cκ | **Vκ-Cλ** |
| PR001942 | HEL | PR00032 6 | CD13 7 | PR000483 | (A) | Vκ-Cκ | Vλ-Cλ |
| PROO 1944 | B7H4 | PR00015 7 | CD13 7 | PR000628 | (B) | Vκ-Cκ | **Vκ-Cλ** |
| PR001945 | B7H4 | PR00015 7 | CD13 7 | PR000483 | (A) | Vκ-Cκ | Vλ-Cλ |

**Table 5: Sequence numbering table of the polypeptide chains of the "κ/λ" Fab-Fab antibody constructed in the present application. (See Fig. 10 for the arrangement of the first, second, or third polypeptide chains)**

| Antibody ID | SEQ ID NO of the first polypeptide chain (Fab-A Fd chain) | SEQ ID NO of the second polypeptide chain (Long chain) | SEQ ID NO of the third polypeptide chain (Fab-B LC chain) |
|---|---|---|---|
| PR000317 | 177 | 178 | 168 |
| PR001325 | 177 | 187 | 174 |
| PR001197 | 179 | 180 | 174 |
| PR001198 | 181 | 182 | 174 |
| PR001200 | 183 | 184 | 174 |
| PR001310 | 185 | 186 | 174 |
| PR001613 | 177 | 188 | 176 |
| PR001614 | 177 | 189 | 176 |
| PR001615 | 181 | 190 | 176 |
| PR001616 | 181 | 191 | 176 |
| PR001941 | 185 | 193 | 192 |
| PR001942 | 185 | 194 | 173 |
| PR001944 | 177 | 195 | 192 |
| PR001945 | 177 | 196 | 173 |

**Table 6: Sequence numbering table of CDRs at each Fab terminus of the "κ/λ" Fab-Fab antibody constructed in the present application**

| Antibody ID | Fab NO. | LCDR1 | LCDR2 | LCDR3 | HCDR1 | HCDR2 | HCDR3 |
|---|---|---|---|---|---|---|---|
| PR000317 | A | 81 | 99 | 117 | 12 | 35 | 58 |
| | B | 82 | 100 | 118 | 13 | 36 | 59 |
| PR001325 | A | 81 | 99 | 117 | 12 | 35 | 58 |
| | B | 82 | 100 | 118 | 13 | 36 | 59 |
| PR001197 | A | 85 | 103 | 121 | 16 | 39 | 62 |
| | B | 82 | 100 | 118 | 13 | 36 | 59 |
| PR001198 | A | 83 | 101 | 119 | 14 | 37 | 60 |
| | B | 82 | 100 | 118 | 13 | 36 | 59 |
| PR001200 | A | 86 | 104 | 122 | 17 | 40 | 63 |
| | B | 82 | 100 | 118 | 13 | 36 | 59 |
| PR001310 | A | 84 | 102 | 120 | 15 | 38 | 61 |
| | B | 82 | 100 | 118 | 13 | 36 | 59 |
| PR001613 | A | 81 | 99 | 117 | 12 | 35 | 58 |
| | B | 89 | 107 | 125 | 12 | 44 | 67 |
| PR001614 | A | 81 | 99 | 117 | 12 | 35 | 58 |
| | B | 89 | 107 | 125 | 12 | 45 | 67 |
| PR001615 | A | 83 | 101 | 119 | 14 | 37 | 60 |
| | B | 89 | 107 | 125 | 12 | 44 | 67 |
| PR001616 | A | 83 | 101 | 119 | 14 | 37 | 60 |
| | B | 89 | 107 | 125 | 12 | 45 | 67 |

(continued)

| Antibody ID | Fab NO. | LCDR1 | LCDR2 | LCDR3 | HCDR1 | HCDR2 | HCDR3 |
|---|---|---|---|---|---|---|---|
| PR001941 | A | 84 | 102 | 120 | 15 | 38 | 61 |
| | B | 88 | 106 | 124 | 19 | 42 | 65 |
| PR001942 | A | 84 | 102 | 120 | 15 | 38 | 61 |
| | B | 87 | 105 | 123 | 18 | 41 | 64 |
| PR001944 | A | 81 | 99 | 117 | 12 | 35 | 58 |
| | B | 88 | 106 | 124 | 19 | 42 | 65 |
| PR001945 | A | 81 | 99 | 117 | 12 | 35 | 58 |
| | B | 87 | 105 | 123 | 18 | 41 | 64 |

**Table 7: Production of the "κ/λ" Fab-Fab antibody constructed in the present application**

| Numbering of the antibodies | Expression system and volume | Ratio for plasmid transfection | Purificatio n method | Yield after the first-step purification (mg/L) | HPLC-SEC Purity (%) | Sample batch |
|---|---|---|---|---|---|---|
| PR000317 | ExpiCHO-s (30 ml) | 1 : 1 : 1 | KappaSelect | 114.76 | 100.00 | YYX201809 19 |
| PR001325 | HEK293 (50 ml) | 1 : 1 : 1 | KappaSelect | 18.94 | 100.00 | YYX201811 29 |
| PR001197 | ExpiCHO-s (30 ml) | 1 : 1 : 1 | KappaSelect | 27.36 | 100.00 | YYX201809 19 |
| PR001198 | ExpiCHO-s (30 ml) | 1 : 1 : 1 | KappaSelect | 91.88 | 100.00 | YYX201809 19 |
| PR001200 | ExpiCHO-s (30 ml) | 1 : 1 : 1 | KappaSelect | 191.08 | 98.39 | YYX201809 19 |
| PR001310 | HEK293 (30 ml) | 1 : 1 : 1 | KappaSelect | 17.81 | 87.66 | YYX201812 11 |
| PR001613 | HEK293 (50 ml) | 1 : 1 : 1 | KappaSelect | 23.50 | 92.94 | YYX201812 15 |
| PR001614 | HEK293 (50 ml) | 1 : 1 : 1 | KappaSelect | 24.65 | 95.00 | YYX201812 15 |
| PR001615 | HEK293 (50 ml) | 1 : 1 : 1 | KappaSelect | 5.30 | 82.94 | YYX201812 15 |
| PR001616 | HEK293 (50 ml) | 1 : 1 : 1 | KappaSelect | 1.74 | 90.86 | YYX201812 15 |
| PR001941 | ExpiCHO-s (100 ml) | 1 : 1 : 1 | KappaSelect | 31.00 | 76.59 | 200317006 |
| PR001942 | ExpiCHO-s (100 ml) | 1 : 1 : 1 | KappaSelect | 60.00 | 83.88 | 200317007 |
| PR001944 | ExpiCHO-s (100 ml) | 1 : 1 : 1 | KappaSelect | 0.90 | Non-tested | 200317009 |
| PR001945 | ExpiCHO-s (100 ml) | 1 : 1 : 1 | KappaSelect | 103.00 | 90.39 | 200317010 |

**Table 8: Sequence number and corresponding amino acid sequences of parent monoclonal antibodies and control antibodies**

| SEQ ID NO | PR000157 | anti-B7H4 1D11.v1.9varC2 hIgG1 |
|---|---|---|
| 154 | PR000157 Heavy Chain Sequence | EVQLVQSGAEVKKPGASVKVSCKASGYTFTSYWIGWVRQAPGQGLEWIGDIYPGGGYTNYNEKFKGRVTITRDTSTSTAYLELSSLRSEDTAVYYCARLAGSSYRGAMDSWGQGTLVTVSSASTKGPSVFPLAPSSKSTSGGTAALGCLVKDYFPEPVTVSWNSGALTSGVHTFPAVLQSSGLYSLSSVVTVPSSSLGTQTYICNVNHKPSNTKVDKKVEPKSCDKTHTCPPCPAPELLGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPSREEMTKNQVSLTCLVKGFYPSDIAVEWESNGQPENNYKTTPPVLDSDGSFFLYSKLTVDKSRWQQGNVFSCSVMHEALHNHYTQKSLSLSPGK |
| 131 | PR000157 VH Sequence | EVQLVQSGAEVKKPGASVKVSCKASGYTFTSYWIGWVRQAPGQGLEWIGDIYPGGGYTNYNEKFKGRVTITRDTSTSTAYLELSSLRSEDTAVYYCARLAGSSYRGAMDSWGQGTLVTVSS |
| 1 | PR000157 VH FWR1 (Chothia) | EVQLVQSGAEVKKPGASVKVSCKAS |
| 12 | PR000157 VH CDR1 (Chothia) | GYTFTSY |
| 22 | PR000157 VH FWR2 (Chothia) | WIGWVRQAPGQGLEWIGDI |
| 35 | PR000157 VH CDR2 (Chothia) | YPGGGY |
| 46 | PR000157 VH FWR3 (Chothia) | TNYNEKFKGRVTITRDTSTSTAYLELSSLRSEDTAVYYCAR |
| 58 | PR000157 VH CDR3 (Chothia) | LAGSSYRGAMDS |
| 68 | PR000157 VH FWR4 (Chothia) | WGQGTLVTVSS |

(continued)

| SEQ ID NO | PR000157 | anti-B7H4 1D11.v1.9varC2 hIgG1 |
|---|---|---|
| 167 | PR000157 Light Chain Sequence | DIQMTQSPSSLSASVGDRVTITCKASQGFNKYVAWYQQKPGK APKLLIYYTSTLQPGVPSRFSGSGSGRDYTLTISSLQPEDFATY YCLQYGDLLYAFGQGTKVEIKRTVAAPSVFIFPPSDEQLKSGT ASVVCLLNNFYPREAKVQWKVDNALQSGNSQESVTEQDSKD STYSLSSTLTLSKADYEKHKVYACEVTHQGLSSPVTKSFNRG EC |
| 144 | PR000157 VL Sequence | DIQMTQSPSSLSASVGDRVTITCKASQGFNKYVAWYQQKPGK APKLLIYYTSTLQPGVPSRFSGSGSGRDYTLTISSLQPEDFATY YCLQYGDLLYAFGQGTKVEIK |
| 73 | PR000157 VL FWR1 (Chothia) | DIQMTQSPSSLSASVGDRVTITC |
| 81 | PR000157 VL CDR1 (Chothia) | KASQGFNKYVA |
| 90 | PR000157 VL FWR2 (Chothia) | WYQQKPGKAPKLLIY |
| 99 | PR000157 VL CDR2 (Chothia) | YTSTLQP |
| 108 | PR000157 VL FWR3 (Chothia) | GVPSRFSGSGSGRDYTLTISSLQPEDFATYYC |
| 117 | PR000157 VL CDR3 (Chothia) | LQYGDLLYA |
| 126 | PR000157 VL FWR4 (Chothia) | FGQGTKVEIK |

(continued)

| SEQ ID NO | PR000327 | anti-PSMA pasotuxizumab(fab) hIgG1 |
|---|---|---|
| 158 | PR000327 Heavy Chain Sequence | QVQLVESGGGLVKPGESLRLSCAASGFTFSDYYMYWVRQAP GKGLEWVAIISDGGYYTYYSDIIKGRFTISRDNAKNSLYLQMN SLKAEDTAVYYCARGFPLLRHGAMDYWGQGTLVTVSSASTK GPSVFPLAPSSKSTSGGTAALGCLVKDYFPEPVTVSWNSGALT SGVHTFPAVLQSSGLYSLSSVVTVPSSSLGTQTYICNVNHKPS NTKVDKKVEPKSCDKTHTCPPCPAPELLGGPSVFLFPPKPKDT LMISRTPEVTCVVVDVSHEDPEVKFNWYVDGVEVHNAKTKP REEQYNSTYRVVSVLTVLHQDWLNGKEYKCKVSNKALPAPI EKTISKAKGQPREPQVYTLPPSREEMTKNQVSLTCLVKGFYPS DIAVEWESNGQPENNYKTTPPVLDSDGSFFLYSKLTVDKSRW QQGNVFSCSVMHEALHNHYTQKSLSLSPGK |
| 135 | PR000327 VH Sequence | QVQLVESGGGLVKPGESLRLSCAASGFTFSDYYMYWVRQAP GKGLEWVAIISDGGYYTYYSDIIKGRFTISRDNAKNSLYLQMN SLKAEDTAVYYCARGFPLLRHGAMDYWGQGTLVTVSS |
| 5 | PR000327 VH FWR1 (Chothia) | QVQLVESGGGLVKPGESLRLSCAAS |
| 16 | PR000327 VH CDR1 (Chothia) | GFTFSDY |
| 26 | PR000327 VH FWR2 (Chothia) | YMYWVRQAPGKGLEWVAII |
| 39 | PR000327 VH CDR2 (Chothia) | SDGGYY |
| 50 | PR000327 VH FWR3 (Chothia) | TYYSDIIKGRFTISRDNAKNSLYLQMNSLKAEDTAVYYCAR |
| 62 | PR000327 VH CDR3 (Chothia) | GFPLLRHGAMDY |
| 68 | PR000327 VH FWR4 (Chothia) | WGQGTLVTVSS |

(continued)

| SEQ ID NO | PR000327 | anti-PSMA pasotuxizumab(fab) hlgG1 |
|---|---|---|
| 171 | PR000327 Light Chain Sequence | DIQMTQSPSSLSASVGDRVTITCKASQNVDTNVAWYQQKPG QAPKSLIYSASYRYSDVPSRFSGSASGTDFTLTISSVQSEDFAT YYCQQYDSYPYTFGGGTKLEIKRTVAAPSVFIFPPSDEQLKSG TASVVCLLNNFYPREAKVQWKVDNALQSGNSQESVTEQDSK DSTYSLSSTLTLSKADYEKHKVYACEVTHQGLSSPVTKSFNR GEC |
| 148 | PR000327 VL Sequence | DIQMTQSPSSLSASVGDRVTITCKASQNVDTNVAWYQQKPG QAPKSLIYSASYRYSDVPSRFSGSASGTDFTLTISSVQSEDFAT YYCQQYDSYPYTFGGGTKLEIK |
| 73 | PR000327 VL FWR1 (Chothia) | DIQMTQSPSSLSASVGDRVTITC |
| 85 | PR000327 VL CDR1 (Chothia) | KASQNVDTNVA |
| 93 | PR000327 VL FWR2 (Chothia) | WYQQKPGQAPKSLIY |
| 103 | PR000327 VL CDR2 (Chothia) | SASYRYS |
| 112 | PR000327 VL FWR3 (Chothia) | DVPSRFSGSASGTDFTLTISSVQSEDFATYYC |
| 121 | PR000327 VL CDR3 (Chothia) | QQYDSYPYT |
| 129 | PR000327 VL FWR4 (Chothia) | FGGGTKLEIK |

(continued)

| SEQ ID NO | PR000274 | anti-BCMA CA8-J6M0 in hIgG1 |
|---|---|---|
| 156 | PR000274 Heavy Chain Sequence | QVQLVQSGAEVKKPGSSVKVSCKASGGTFSNYWMHWVRQA PGQGLEWMGATYRGHSDTYYNQKFKGRVTITADKSTSTAY MELSSLRSEDTAVYYCARGAIYDGYDVLDNWGQGTLVTVSS ASTKGPSVFPLAPSSKSTSGGTAALGCLVKDYFPEPVTVSWNS GALTSGVHTFPAVLQSSGLYSLSSVVTVPSSSLGTQTYICNVN HKPSNTKVDKKVEPKSCDKTHTCPPCPAPELLGGPSVFLFPPK PKDTLMISRTPEVTCVVVDVSHEDPEVKFNWYVDGVEVHNA KTKPREEQYNSTYRVVSVLTVLHQDWLNGKEYKCKVSNKAL PAPIEKTISKAKGQPREPQVYTLPPSREEMTKNQVSLTCLVKG FYPSDIAVEWESNGQPENNYKTTPPVLDSDGSFFLYSKLTVDK SRWQQGNVFSCSVMHEALHNHYTQKSLSLSPGK |
| 133 | PR000274 VH Sequence | QVQLVQSGAEVKKPGSSVKVSCKASGGTFSNYWMHWVRQA PGQGLEWMGATYRGHSDTYYNQKFKGRVTITADKSTSTAY MELSSLRSEDTAVYYCARGAIYDGYDVLDNWGQGTLVTVSS |
| 3 | PR000274 VH FWR1 (Chothia) | QVQLVQSGAEVKKPGSSVKVSCKAS |
| 14 | PR000274 VH CDR1 (Chothia) | GGTFSNY |
| 24 | PR000274 VH FWR2 (Chothia) | WMHWVRQAPGQGLEWMGAT |
| 37 | PR000274 VH CDR2 (Chothia) | YRGHSD |
| 48 | PR000274 VH FWR3 (Chothia) | TYYNQKFKGRVTITADKSTSTAYMELSSLRSEDTAVYYCAR |
| 60 | PR000274 VH CDR3 (Chothia) | GAIYDGYDVLDN |
| 68 | PR000274 VH FWR4 (Chothia) | WGQGTLVTVSS |
| 169 | PR000274 Light Chain Sequence | DIQMTQSPSSLSASVGDRVTITCSASQDISNYLNWYQQKPGK APKLLIYYTSNLHSGVPSRFSGSGSGTDFTLTISSLQPEDFATY YCQQYRKLPWTFGQGTKLEIKRTVAAPSVFIFPPSDEQLKSGT ASVVCLLNNFYPREAKVQWKVDNALQSGNSQESVTEQDSKD STYSLSSTLTLSKADYEKHKVYACEVTHQGLSSPVTKSFNRG |

(continued)

| SEQ ID NO | PR000274 | anti-BCMA CA8-J6M0 in hIgG1 |
|---|---|---|
| | | EC |
| 146 | PR000274 VL Sequence | DIQMTQSPSSLSASVGDRVTITCSASQDISNYLNWYQQKPGK APKLLIYYTSNLHSGVPSRFSGSGSGTDFTLTISSLQPEDFATY YCQQYRKLPWTFGQGTKLEIK |
| 73 | PR000274 VL FWR1 (Chothia) | DIQMTQSPSSLSASVGDRVTITC |
| 83 | PR000274 VL CDR1 (Chothia) | SASQDISNYLN |
| 90 | PR000274 VL FWR2 (Chothia) | WYQQKPGKAPKLLIY |
| 101 | PR000274 VL CDR2 (Chothia) | YTSNLHS |
| 110 | PR000274 VL FWR3 (Chothia) | GVPSRFSGSGSGTDFTLTISSLQPEDFATYYC |
| 119 | PR000274 VL CDR3 (Chothia) | QQYRKLPWT |
| 128 | PR000274 VL FWR4 (Chothia) | FGQGTKLEIK |
| SEQ ID NO | PR000374 | anti-ROR1 R11H18L9 hIgG1 |
| 159 | PR0003 74 Heavy Chain Sequence | EVQLVESGGGLVQPGRSLRLSCTASGSDINDYPITWVRQAPG QGLEWIGFINSGGSTWYASWVKGRFTISRDDSKSIAYLQMNS LKTEDTAVYYCARGYSTYYRDFNIWGQGTLVTVSSASTKGPS VFPLAPSSKSTSGGTAALGCLVKDYFPEPVTVSWNSGALTSG VHTFPAVLQSSGLYSLSSVVTVPSSSLGTQTYICNVNHKPSNT KVDKKVEPKSCDKTHTCPPCPAPELLGGPSVFLFPPKPKDTLM ISRTPEVTCVVVDVSHEDPEVKFNWYVDGVEVHNAKTKPRE EQYNSTYRVVSVLTVLHQDWLNGKEYKCKVSNKALPAPIEK TISKAKGQPREPQVYTLPPSREEMTKNQVSLTCLVKGFYPSDI AVEWESNGQPENNYKTTPPVLDSDGSFFLYSKLTVDKSRWQ QGNVFSCSVMHEALHNHYTQKSLSLSPGK |

(continued)

| SEQ ID NO | PR000374 | anti-ROR1 R11H18L9 hIgG1 |
|---|---|---|
| 136 | PR000374 VH Sequence | EVQLVESGGGLVQPGRSLRLSCTASGSDINDYPITWVRQAPGQGLEWIGFINSGGSTWYASWVKGRFTISRDDSKSIAYLQMNSLKTEDTAVYYCARGYSTYYRDFNIWGQGTLVTVSS |
| 6 | PR000374 VH FWR1 (Chothia) | EVQLVESGGGLVQPGRSLRLSCTAS |
| 17 | PR000374 VH CDR1 (Chothia) | GSDINDY |
| 27 | PR000374 VH FWR2 (Chothia) | PITWVRQAPGQGLEWIGFI |
| 40 | PR000374 VH CDR2 (Chothia) | NSGGS |
| 51 | PR000374 VH FWR3 (Chothia) | TWYASWVKGRFTISRDDSKSIAYLQMNSLKTEDTAVYYCAR |
| 63 | PR000374 VH CDR3 (Chothia) | GYSTYYRDFNI |
| 68 | PR000374 VH FWR4 (Chothia) | WGQGTLVTVSS |
| 172 | PR000374 Light Chain Sequence | DIQMTQSPSSLSASVGDRVTINCQASQSIDSNLAWFQQKPGQPPKLLIYRASNLASGVPDRFSGSGSGTDFTLTISSLEAEDVATYYCLGGVGAVSYRTSFGGGTKVEIKRTVAAPSVFIFPPSDEQLKSGTASVVCLLNNFYPREAKVQWKVDNALQSGNSQESVTEQDSKDSTYSLSSTLTLSKADYEKHKVYACEVTHQGLSSPVTKSFNRGEC |
| 149 | PR000374 VL Sequence | DIQMTQSPSSLSASVGDRVTINCQASQSIDSNLAWFQQKPGQPPKLLIYRASNLASGVPDRFSGSGSGTDFTLTISSLEAEDVATYYCLGGVGAVSYRTSFGGGTKVEIK |
| 76 | PR000374 VL FWR1 (Chothia) | DIQMTQSPSSLSASVGDRVTINC |
| 86 | PR000374 VL CDR1 (Chothia) | QASQSIDSNLA |
| 94 | PR000374 VL FWR2 (Chothia) | WFQQKPGQPPKLLIY |
| 104 | PR000374 VL CDR2 (Chothia) | RASNLAS |

(continued)

| SEQ ID NO | PR000374 | anti-ROR1 R11H18L9 hIgG1 |
|---|---|---|
| 113 | PR000374 VL FWR3 (Chothia) | GVPDRFSGSGSGTDFTLTISSLEAEDVATYYC |
| 122 | PR000374 VL CDR3 (Chothia) | LGGVGAVSYRTS |
| 130 | PR000374 VL FWR4 (Chothia) | FGGGTKVEIK |

| SEQ ID NO | PR000326 | clone HYHEL-5 in hIgG1 |
|---|---|---|
| 157 | PR000326 Heavy Chain Sequence | EVQLQQSGAELMKPGASVKISCKASGYTFSDYWIEWVKQRPGHGLEWIGEILPGSGSTNYHERFKGKATFTADTSSSTAYMQLNSLTSEDSGVYYCLHGNYDFDGWGQGTTLTVSSASTKGPSVFPLAPSSKSTSGGTAALGCLVKDYFPEPVTVSWNSGALTSGVHTFPAVLQSSGLYSLSSVVTVPSSSLGTQTYICNVNHKPSNTKVDKKVEPKSCDKTHTCPPCPAPELLGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPSREEMTKNQVSLTCLVKGFYPSDIAVEWESNGQPENNYKTTPPVLDSDGSFFLYSKLTVDKSRWQQGNVFSCSVMHEALHNHYTQKSLSLSPGK |
| 134 | PR000326 VH Sequence | EVQLQQSGAELMKPGASVKISCKASGYTFSDYWIEWVKQRPGHGLEWIGEILPGSGSTNYHERFKGKATFTADTSSSTAYMQLNSLTSEDSGVYYCLHGNYDFDGWGQGTTLTVSS |
| 4 | PR000326 VH FWR1 (Chothia) | EVQLQQSGAELMKPGASVKISCKAS |
| 15 | PR000326 VH CDR1 (Chothia) | GYTFSDY |
| 25 | PR000326 VH FWR2 (Chothia) | WIEWVKQRPGHGLEWIGEI |
| 38 | PR000326 VH CDR2 (Chothia) | LPGSGS |
| 49 | PR000326 VH FWR3 (Chothia) | TNYHERFKGKATFTADTSSSTAYMQLNSLTSEDSGVYYCLH |
| 61 | PR000326 VH CDR3 (Chothia) | GNYDFDG |

(continued)

| SEQ ID NO | PR000326 | clone HYHEL-5 in hIgG1 |
|---|---|---|
| 70 | PR000326 VH FWR4 (Chothia) | WGQGTTLTVSS |
| 170 | PR000326 Light Chain Se- quence | DIVLTQSPAIMSASPGEKVTMTCSASSSVNYMYWYQQKSGTS PKRWIYDTSKLASGVPVRFSGSGSGTSYSLTISSMETEDAAEY YCQQWGRNPTFGGGTKLEIKRTVAAPSVFIFPPSDEQLKSGTA SVVCLLNNFYPREAKVQWKVDNALQSGNSQESVTEQDSKDS TYSLSSTLTLSKADYEKHKVYACEVTHQGLSSPVTKSFNRGE C |
| 147 | PR000326 VL Sequence | DIVLTQSPAIMSASPGEKVTMTCSASSSVNYMYWYQQKSGTS PKRWIYDTSKLASGVPVRFSGSGSGTSYSLTISSMETEDAAEY YCQQWGRNPTFGGGTKLEIK |
| 75 | PR000326 VL FWR1 (Chothia) | DIVLTQSPAIMSASPGEKVTMTC |
| 84 | PR000326 VL CDR1 (Chothia) | SASSSVNYMY |
| 92 | PR000326 VL FWR2 (Chothia) | WYQQKSGTSPKRWIY |
| 102 | PR000326 VL CDR2 (Chothia) | DTSKLAS |
| 111 | PR000326 VL FWR3 (Chothia) | GVPVRFSGSGSGTSYSLTISSMETEDAAEYYC |
| 120 | PR000326 VL CDR3 (Chothia) | QQWGRNPT |
| 129 | PR000326 VL FWR4 (Chothia) | FGGGTKLEIK |

| SEQ ID NO | PR000260 | anti-CD3 SP34 hIgG1(LALA) |
|---|---|---|
| 155 | PR000260 Heavy Chain Sequence | EVQLVESGGGLVQPKGSLKLSCAASGFTFNTYAMNWVRQAPGKGLEWVARIRSKYNNYATYYADSVKDRFTISRDDSQSILYLQMNNLKTEDTAMYYCVRHGNFGNSYVSWFAYWGQGTLVTVSAASTKGPSVFPLAPSSKSTSGGTAALGCLVKDYFPEPVTVSWNSGALTSGVHTFPAVLQSSGLYSLSSVVTVPSSSLGTQTYICNVNHKPSNTKVDKKVEPKSCDKTHTCPPCPAPEAAGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPSREEMTKNQVSLTCLVKGFYPSDIAVEWESNGQPENNYKTTPPVLDSDGSFFLYSKLTVDKSRWQQGNVFSCSVMHEALHNHYTQKSLSLSPGK |
| 132 | PR000260 VH Sequence | EVQLVESGGGLVQPKGSLKLSCAASGFTFNTYAMNWVRQAPGKGLEWVARIRSKYNNYATYYADSVKDRFTISRDDSQSILYLQMNNLKTEDTAMYYCVRHGNFGNSYVSWFAYWGQGTLVTVSA |
| 2 | PR000260 VH FWR1 (Chothia) | EVQLVESGGGLVQPKGSLKLSCAAS |
| 13 | PR000260 VH CDR1 (Chothia) | GFTFNTY |
| 23 | PR000260 VH FWR2 (Chothia) | AMNWVRQAPGKGLEWVARI |
| 36 | PR000260 VH CDR2 (Chothia) | RSKYNNYA |
| 47 | PR000260 VH FWR3 (Chothia) | TYYADSVKDRFTISRDDSQSILYLQMNNLKTEDTAMYYCVR |
| 59 | PR000260 VH CDR3 (Chothia) | HGNFGNSYVSWFAY |
| 69 | PR000260 VH FWR4 (Chothia) | WGQGTLVTVSA |
| 168 | PR000260 Light Chain Sequence | QAVVTQESALTTSPGETVTLTCRSSTGAVTTSNYANWVQEKPDHLFTGLIGGTNKRAPGVPARFSGSLIGDKAALTITGAQTEDEAIYFCALWYSNLWVFGGGTKLTVLGQPKAAPSVTLFPPSSEELQANKATLVCLISDFYPGAVTVAWKADSSPVKAGVETTTPSK |

(continued)

| SEQ ID NO | PR000260 | anti-CD3 SP34 hIgG1(LALA) |
|---|---|---|
|  |  | QSNNKYAASSYLSLTPEQWKSHRSYSCQVTHEGSTVEKTVAP TECS |
| 145 | PR000260 VL Sequence | QAVVTQESALTTSPGETVTLTCRSSTGAVTTSNYANWVQEKP DHLFTGLIGGTNKRAPGVPARFSGSLIGDKAALTITGAQTEDE AIYFCALWYSNLWVFGGGTKLTVL |
| 74 | PR000260 VL FWR1 (Chothia) | QAVVTQESALTTSPGETVTLTC |
| 82 | PR000260 VL CDR1 (Chothia) | RSSTGAVTTSNYAN |
| 91 | PR000260 VL FWR2 (Chothia) | WVQEKPDHLFTGLIG |
| 100 | PR000260 VL CDR2 (Chothia) | GTNKRAP |
| 109 | PR000260 VL FWR3 (Chothia) | GVPARFSGSLIGDKAALTITGAQTEDEAIYFC |
| 118 | PR000260 VL CDR3 (Chothia) | ALWYSNLWV |
| 127 | PR000260 VL FWR4 (Chothia) | FGGGTKLTVL |
| SEQ ID NO | PR000512 | anti-CD3 SP34 hz_VH3731_VL7461 hIgG1(LALA) |
| 161 | PR000512 Heavy Chain Sequence | EVQLVESGGGLVQPGGSLKLSCAASGFTFNTYAMNWVRQAS GKGLEWVGRIRSKYNNYATYYADSVKDRFTISRDDSKSTLYL QMNSLKTEDTAVYYCVRHGNFGNSYVSWFAYWGQGTLVTV SSASTKGPSVFPLAPSSKSTSGGTAALGCLVKDYFPEPVTVSW NSGALTSGVHTFPAVLQSSGLYSLSSVVTVPSSSLGTQTYICN VNHKPSNTKVDKKVEPKSCDKTHTCPPCPAPEAAGGPSVFLF PPKPKDTLMISRTPEVTCVVVDVSHEDPEVKFNWYVDGVEV HNAKTKPREEQYNSTYRVVSVLTVLHQDWLNGKEYKCKVS NKALPAPIEKTISKAKGQPREPQVYTLPPSREEMTKNQVSLTC LVKGFYPSDIAVEWESNGQPENNYKTTPPVLDSDGSFFLYSKL TVDKSRWQQGNVFSCSVMHEALHNHYTQKSLSLSPGK |

(continued)

| SEQ ID NO | PR000512 | anti-CD3 SP34 hz_VH3731_VL7461 hIgG1(LALA) |
|---|---|---|
| 138 | PR000512 VH Sequence | EVQLVESGGGLVQPGGSLKLSCAASGFTFNTYAMNWVRQASGKGLEWVGRIRSKYNNYATYYADSVKDRFTISRDDSKSTLYLQMNSLKTEDTAVYYCVRHGNFGNSYVSWFAYWGQGTLVTVSS |
| 8 | PR000512 VH FWR1 (Chothia) | EVQLVESGGGLVQPGGSLKLSCAAS |
| 13 | PR000512 VH CDR1 (Chothia) | GFTFNTY |
| 29 | PR000512 VH FWR2 (Chothia) | AMNWVRQASGKGLEWVGRI |
| 36 | PR000512 VH CDR2 (Chothia) | RSKYNNYA |
| 53 | PR000512 VH FWR3 (Chothia) | TYYADSVKDRFTISRDDSKSTLYLQMNSLKTEDTAVYYCVR |
| 59 | PR000512 VH CDR3 (Chothia) | HGNFGNSYVSWFAY |
| 68 | PR000512 VH FWR4 (Chothia) | WGQGTLVTVSS |
| 174 | PR000512 Light Chain Sequence | QAVVTQEPSLTVSPGGTVTLTCRSSTGAVTTSNYANWVQQKPGQAPRGLIGGTNKRAPWTPARFSGSLLGDKAALTLLGAQPEDEAEYFCALWYSNLWVFGGGTKLTVLGQPKAAPSVTLFPPSSEELQANKATLVCLISDFYPGAVTVAWKADSSPVKAGVETTTPSKQSNNKYAASSYLSLTPEQWKSHRSYSCQVTHEGSTVEKTVAPTECS |
| 151 | PR000512 VL Sequence | QAVVTQEPSLTVSPGGTVTLTCRSSTGAVTTSNYANWVQQKPGQAPRGLIGGTNKRAPWTPARFSGSLLGDKAALTLLGAQPEDEAEYFCALWYSNLWVFGGGTKLTVL |
| 78 | PR000512 VL FWR1 (Chothia) | QAVVTQEPSLTVSPGGTVTLTC |
| 82 | PR000512 VL CDR1 (Chothia) | RSSTGAVTTSNYAN |
| 96 | PR000512 VL FWR2 (Chothia) | WVQQKPGQAPRGLIG |

(continued)

| SEQ ID NO | PR000512 | anti-CD3 SP34 hz_VH3731_VL7461 hIgG1(LALA) |
|---|---|---|
| 100 | PR000512 VL CDR2 (Chothia) | GTNKRAP |
| 115 | PR000512 VL FWR3 (Chothia) | WTPARFSGSLLGDKAALTLLGAQPEDEAEYFC |
| 118 | PR000512 VL CDR3 (Chothia) | ALWYSNLWV |
| 127 | PR000512 VL FWR4 (Chothia) | FGGGTKLTVL |
| **SEQ ID NO** | **PR001848** | **anti-CD3 SP34 hz_VH3232_VL7461 hIgG1(AAG)** |
| 166 | PR001848 Heavy Chain Sequence | EVQLLESGGGLVQPGGSLRLSCAASGFTFSTYAMNWVRQAPGKGLEWVSRIRSKYNNYATYYADSVKDRFTISRDDSKSTLYLQMNSLRAEDTAVYYCVRHGNFGNSYVSWFAYWGQGTLVTVSSASTKGPSVFPLAPSSKSTSGGTAALGCLVKDYFPEPVTVSWNSGALTSGVHTFPAVLQSSGLYSLSSVVTVPSSSLGTQTYICNVNHKPSNTKVDKKVEPKSCDKTHTCPPCPAPEAAGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALGAPIEKTISKAKGQPREPQVYTLPPSREEMTKNQVSLTCLVKGFYPSDIAVEWESNGQPENNYKTTPPVLDSDGSFFLYSKLTVDKSRWQQGNVFSCSVMHEALHNHYTQKSLSLSPGK |
| 143 | PR001848 VH Sequence | EVQLLESGGGLVQPGGSLRLSCAASGFTFSTYAMNWVRQAPGKGLEWVSRIRSKYNNYATYYADSVKDRFTISRDDSKSTLYLQMNSLRAEDTAVYYCVRHGNFGNSYVSWFAYWGQGTLVTVSS |
| 10 | PR001848 VH FWR1 (Chothia) | EVQLLESGGGLVQPGGSLRLSCAAS |
| 21 | PR001848 VH CDR1 (Chothia) | GFTFSTY |
| 34 | PR001848 VH FWR2 (Chothia) | AMNWVRQAPGKGLEWVSRI |
| 36 | PR001848 VH CDR2 (Chothia) | RSKYNNYA |

(continued)

| SEQ ID NO | PR001848 | anti-CD3 SP34 hz_VH3232_VL7461 hIgG1(AAG) |
|---|---|---|
| 57 | PR001848 VH FWR3 (Chothia) | TYYADSVKDRFTISRDDSKSTLYLQMNSLRAEDTAVYYCVR |
| 59 | PR001848 VH CDR3 (Chothia) | HGNFGNSYVSWFAY |
| 68 | PR001848 VH FWR4 (Chothia) | WGQGTLVTVSS |
| 174 | PR001848 Light Chain Sequence | QAVVTQEPSLTVSPGGTVTLTCRSSTGAVTTSNYANWVQQKP GQAPRGLIGGTNKRAPWTPARFSGSLLGDKAALTLLGAQPED EAEYFCALWYSNLWVFGGGTKLTVLGQPKAAPSVTLFPPSSE ELQANKATLVCLISDFYPGAVTVAWKADSSPVKAGVETTTPS KQSNNKYAASSYLSLTPEQWKSHRSYSCQVTHEGSTVEKTV APTECS |
| 151 | PR001848 VL Sequence | QAVVTQEPSLTVSPGGTVTLTCRSSTGAVTTSNYANWVQQKP GQAPRGLIGGTNKRAPWTPARFSGSLLGDKAALTLLGAQPED EAEYFCALWYSNLWVFGGGTKLTVL |
| 78 | PR001848 VL FWR1 (Chothia) | QAVVTQEPSLTVSPGGTVTLTC |
| 82 | PR001848 VL CDR1 (Chothia) | RSSTGAVTTSNYAN |
| 96 | PR001848 VL FWR2 (Chothia) | WVQQKPGQAPRGLIG |
| 100 | PR001848 VL CDR2 (Chothia) | GTNKRAP |
| 115 | PR001848 VL FWR3 (Chothia) | WTPARFSGSLLGDKAALTLLGAQPEDEAEYFC |
| 118 | PR001848 VL CDR3 (Chothia) | ALWYSNLWV |
| 127 | PR001848 VL FWR4 (Chothia) | FGGGTKLTVL |

| SEQ ID NO | PR001611 | anti-CD16A CD16A_VH-1 hIgG1(AAG) |
|---|---|---|
| 164 | PR001611 Heavy Chain Sequence | QVQLVQSGAEVKKPGESLKVSCKASGYTFTSYYMHWVRQAPGQGLEWMGAIEPMYGSTSYAQKFQGRVTMRDTSTSTVYMELSSLRSEDTAVYYCARGSAYYYDFADYWGQGTLVTVSSASTKGPSVFPLAPSSKSTSGGTAALGCLVKDYFPEPVTVSWNSGALTSGVHTFPAVLQSSGLYSLSSVVTVPSSSLGTQTYICNVNHKPSNTKVDKKVEPKSCDKTHTCPPCPAPEAAGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALGAPIEKTISKAKGQPREPQVYTLPPSREEMTKNQVSLTCLVKGFYPSDIAVEWESNGQPENNYKTTPPVLDSDGSFFLYSKLTVDKSRWQQGNVFSCSVMHEALHNHYTQKSLSLSPGK |
| 141 | PR001611 VH Sequence | QVQLVQSGAEVKKPGESLKVSCKASGYTFTSYYMHWVRQAPGQGLEWMGAIEPMYGSTSYAQKFQGRVTMRDTSTSTVYMELSSLRSEDTAVYYCARGSAYYYDFADYWGQGTLVTVSS |
| 11 | PR001611 VH FWR1 (Chothia) | QVQLVQSGAEVKKPGESLKVSCKAS |
| 12 | PR001611 VH CDR1 (Chothia) | GYTFTSY |
| 32 | PR001611 VH FWR2 (Chothia) | YMHWVRQAPGQGLEWMGAI |
| 44 | PR001611 VH CDR2 (Chothia) | EPMYGS |
| 56 | PR001611 VH FWR3 (Chothia) | TSY AQKFQGRVTMRDTSTSTVYMELSSLRSEDTA VYYCAR |
| 67 | PR001611 VH CDR3 (Chothia) | GSAYYYDFADY |
| 68 | PR001611 VH FWR4 (Chothia) | WGQGTLVTVSS |
| 176 | PR001611 Light Chain Sequence | SYVLTQPSSVSVAPGQTATISCGGHNIGSKNVHWYQQRPGQSPVLVIYQDNKRPSGIPERFSGSNSGNTATLTISGTQAMDEADYYCQVWDNYSVLFGGGTKLTVLGQPKAAPSVTLFPPSSEELQANKATLVCLISDFYPGAVTVAWKADSSPVKAGVETTTPSKQSN |

| SEQ ID NO | PR001611 | anti-CD16A CD16A_VH-1 hIgG1(AAG) |
|---|---|---|
| | | NKYAASSYLSLTPEQWKSHRSYSCQVTHEGSTVEKTVAPTECS |
| 153 | PR001611 VL Sequence | SYVLTQPSSVSVAPGQTATISCGGHNIGSKNVHWYQQRPGQSPVLVIYQDNKRPSGIPERFSGSNSGNTATLTISGTQAMDEADYYCQVWDNYSVLFGGGTKLTVL |
| 80 | PR001611 VL FWR1 (Chothia) | SYVLTQPSSVSVAPGQTATISC |
| 89 | PR001611 VL CDR1 (Chothia) | GGHNIGSKNVH |
| 98 | PR001611 VL FWR2 (Chothia) | WYQQRPGQSPVLVIY |
| 107 | PR001611 VL CDR2 (Chothia) | QDNKRPS |
| 114 | PR001611 VL FWR3 (Chothia) | GIPERFSGSNSGNTATLTISGTQAMDEADYYC |
| 125 | PR001611 VL CDR3 (Chothia) | QVWDNYSVL |
| 127 | PR001611 VL FWR4 (Chothia) | FGGGTKLTVL |
| **SEQ ID NO** | **PR001612** | **anti-CD16A CD16A_VH-2 hIgG1(AAG)** |
| 165 | PR001612 Heavy Chain Sequence | QVQLVQSGAEVKKPGESLKVSCKASGYTFTSYYMHWVRQAPGQGLEWMGIINPSGGSTSYAQKFQGRVTMTRDTSTSTVYMELSSLRSEDTAVYYCARGSAYYYDFADYWGQGTLVTVSSASTKGPSVFPLAPSSKSTSGGTAALGCLVKDYFPEPVTVSWNSGALTSGVHTFPAVLQSSGLYSLSSVVTVPSSSLGTQTYICNVNHKPSNTKVDKKVEPKSCDKTHTCPPCPAPEAAGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALGAPIEKTISKAKGQPREPQVYTLPPSREEMTKNQVSLTCLVKGFYPSDIAVEWESNGQPENNYKTTPPVLDSDGSFFLYSKLTVDKSRWQQGNVFSCSVMHEALHNHYTQKSLSLSPGK |

(continued)

| SEQ ID NO | PR001612 | anti-CD16A CD16A_VH-2 hIgG1(AAG) |
|---|---|---|
| 142 | PR001612 VH Sequence | QVQLVQSGAEVKKPGESLKVSCKASGYTFTSYYMHWVRQAPGQGLEWMGIINPSGGSTSYAQKFQGRVTMTRDTSTSTVYMELSSLRSEDTAVYYCARGSAYYYDFADYWGQGTLVTVSS |
| 11 | PR001612 VH FWR1 (Chothia) | QVQLVQSGAEVKKPGESLKVSCKAS |
| 12 | PR001612 VH CDR1 (Chothia) | GYTFTSY |
| 33 | PR001612 VH FWR2 (Chothia) | YMHWVRQAPGQGLEWMGII |
| 45 | PR001612 VH CDR2 (Chothia) | NPSGGS |
| 56 | PR001612 VH FWR3 (Chothia) | TSY AQKFQGRVTMTRDTSTSTVYMELSSLRSEDTA VYYCAR |
| 67 | PR001612 VH CDR3 (Chothia) | GSAYYYDFADY |
| 68 | PR001612 VH FWR4 (Chothia) | WGQGTLVTVSS |
| 176 | PR001612 Light Chain Sequence | SYVLTQPSSVSVAPGQTATISCGGHNIGSKNVHWYQQRPGQSPVLVIYQDNKRPSGIPERFSGSNSGNTATLTISGTQAMDEADYYCQVWDNYSVLFGGGTKLTVLGQPKAAPSVTLFPPSSEELQANKATLVCLISDFYPGAVTVAWKADSSPVKAGVETTTPSKQSNNKYAASSYLSLTPEQWKSHRSYSCQVTHEGSTVEKTVAPTECS |
| 153 | PR001612 VL Sequence | SYVLTQPSSVSVAPGQTATISCGGHNIGSKNVHWYQQRPGQSPVLVIYQDNKRPSGIPERFSGSNSGNTATLTISGTQAMDEADYYCQVWDNYSVLFGGGTKLTVL |
| 80 | PR001612 VL FWR1 (Chothia) | SYVLTQPSSVSVAPGQTATISC |
| 89 | PR001612 VL CDR1 (Chothia) | GGHNIGSKNVH |
| 98 | PR001612 VL FWR2 (Chothia) | WYQQRPGQSPVLVIY |
| 107 | PR001612 VL CDR2 (Chothia) | QDNKRPS |

(continued)

| SEQ ID NO | PR001612 | anti-CD16A CD16A_VH-2 hIgG1(AAG) |
|---|---|---|
| 114 | PR001612 VL FWR3 (Chothia) | GIPERFSGSNSGNTATLTISGTQAMDEADYYC |
| 125 | PR001612 VL CDR3 (Chothia) | QVWDNYSVL |
| 127 | PR001612 VL FWR4 (Chothia) | FGGGTKLTVL |
| SEQ ID NO | PR000628 | anti-4-1BB urelumab hIgG4 |
| 162 | PR000628 Heavy Chain Sequence | QVQLQQWGAGLLKPSETLSLTCAVYGGSFSGYYWSWIRQSPEKGLEWIGEINHGGYVTYNPSLESRVTISVDTSKNQFSLKLSSVTAADTAVYYCARDYGPGNYDWYFDLWGRGTLVTVSSASTKGPSVFPLAPCSRSTSESTAALGCLVKDYFPEPVTVSWNSGALTSGVHTFPAVLQSSGLYSLSSVVTVPSSSLGTKTYTCNVDHKPSNTKVDKRVESKYGPPCPPCPAPEFLGGPSVFLFPPKPKDTLMISRTPEVTCVVVDVSQEDPEVQFNWYVDGVEVHNAKTKPREEQFNSTYRVVSVLTVLHQDWLNGKEYKCKVSNKGLPSSIEKTISKAKGQPREPQVYTLPPSQEEMTKNQVSLTCLVKGFYPSDIAVEWESNGQPENNYKTTPPVLDSDGSFFLYSRLTVDKSRWQEGNVFSCSVMHEALHNHYTQKSLSLSLGK |
| 139 | PR000628 VH Sequence | QVQLQQWGAGLLKPSETLSLTCAVYGGSFSGYYWSWIRQSPEKGLEWIGEINHGGYVTYNPSLESRVTISVDTSKNQFSLKLSSVTAADTAVYYCARDYGPGNYDWYFDLWGRGTLVTVSS |
| 9 | PR000628 VH FWR1 (Chothia) | QVQLQQWGAGLLKPSETLSLTCAVY |
| 19 | PR000628 VH CDR1 (Chothia) | GGSFSGY |
| 30 | PR000628 VH FWR2 (Chothia) | YWSWIRQSPEKGLEWIGEI |
| 42 | PR000628 VH CDR2 (Chothia) | NHGGY |
| 54 | PR000628 VH FWR3 (Chothia) | VTYNPSLESRVTISVDTSKNQFSLKLSSVTAADTAVYYCAR |
| 65 | PR000628 VH CDR3 (Chothia) | DYGPGNYDWYFDL |

(continued)

| SEQ ID NO | PR000628 | anti-4-1BB urelumab hIgG4 |
|---|---|---|
| 71 | PR000628 VH FWR4 (Chothia) | WGRGTLVTVSS |
| 175 | PR000628 Light Chain Sequence | EIVLTQSPATLSLSPGERATLSCRASQSVSSYLAWYQQKPGQA PRLLIYDASNRATGIPARFSGSGSGTDFTLTISSLEPEDFAVYY CQQRSNWPPALTFGGGTKVEIKRTVAAPSVFIFPPSDEQLKSG TASVVCLLNNFYPREAKVQWKVDNALQSGNSQESVTEQDSK DSTYSLSSTLTLSKADYEKHKVYACEVTHQGLSSPVTKSFNR GEC |
| 152 | PR000628 VL Sequence | EIVLTQSPATLSLSPGERATLSCRASQSVSSYLAWYQQKPGQA PRLLIYDASNRATGIPARFSGSGSGTDFTLTISSLEPEDFAVYY CQQRSNWPPALTFGGGTKVEIK |
| 79 | PR000628 VL FWR1 (Chothia) | EIVLTQSPATLSLSPGERATLSC |
| 88 | PR000628 VL CDR1 (Chothia) | RASQSVSSYLA |
| 97 | PR000628 VL FWR2 (Chothia) | WYQQKPGQAPRLLIY |
| 106 | PR000628 VL CDR2 (Chothia) | DASNRAT |
| 116 | PR000628 VL FWR3 (Chothia) | GIPARFSGSGSGTDFTLTISSLEPEDFAVYYC |
| 124 | PR000628 VL CDR3 (Chothia) | QQRSNWPPALT |
| 130 | PR000628 VL FWR4 (Chothia) | FGGGTKVEIK |

(continued)

| SEQ ID NO | PR000483 | anti-4-1BB utomilumab hIgG2 |
|---|---|---|
| 160 | PR000483 Heavy Chain Sequence | EVQLVQSGAEVKKPGESLRISCKGSGYSFSTYWISWVRQMPGKGLEWMGKIYPGDSYTNYSPSFQGQVTISADKSISTAYLQWSSLKASDTAMYYCARGYGIFDYWGQGTLVTVSSASTKGPSVFPLAPCSRSTSESTAALGCLVKDYFPEPVTVSWNSGALTSGVHTFPAVLQSSGLYSLSSVVTVPSSNFGTQTYTCNVDHKPSNTKVDKTVERKCCVECPPCPAPPVAGPSVFLFPPKPKDTLMISRTPEVTCVVVDVSHEDPEVQFNWYVDGVEVHNAKTKPREEQFNSTFRVVSVLTVVHQDWLNGKEYKCKVSNKGLPAPIEKTISKTKGQPREPQVYTLPPSREEMTKNQVSLTCLVKGFYPSDIAVEWESNGQPENNYKTTPPMLDSDGSFFLYSKLTVDKSRWQQGNVFSCSVMHEALHNHYTQKSLSLSPGK |
| 137 | PR000483 VH Sequence | EVQLVQSGAEVKKPGESLRISCKGSGYSFSTYWISWVRQMPGKGLEWMGKIYPGDSYTNYSPSFQGQVTISADKSISTAYLQWSSLKASDTAMYYCARGYGIFDYWGQGTLVTVSS |
| 7 | PR000483 VH FWR1 (Chothia) | EVQLVQSGAEVKKPGESLRISCKGS |
| 18 | PR000483 VH CDR1 (Chothia) | GYSFSTY |
| 28 | PR000483 VH FWR2 (Chothia) | WISWVRQMPGKGLEWMGKI |
| 41 | PR000483 VH CDR2 (Chothia) | YPGDSY |
| 52 | PR000483 VH FWR3 (Chothia) | TNYSPSFQGQVTISADKSISTAYLQWSSLKASDTAMYYCAR |
| 64 | PR000483 VH CDR3 (Chothia) | GYGIFDY |
| 68 | PR000483 VH FWR4 (Chothia) | WGQGTLVTVSS |
| 173 | PR000483 Light Chain Se- quence | SYELTQPPSVSVSPGQTASITCSGDNIGDQYAHWYQQKPGQSPVLVIYQDKNRPSGIPERFSGSNSGNTATLTISGTQAMDEADYYCATYTGFGSLAVFGGGTKLTVLGQPKAAPSVTLFPPSSEELQANKATLVCLISDFYPGAVTVAWKADSSPVKAGVETTTPSKQSNNKYAASSYLSLTPEQWKSHRSYSCQVTHEGSTVEKTVAPTEC |

(continued)

| SEQ ID NO | PR000483 | anti-4-1BB utomilumab hIgG2 |
|---|---|---|
| | | S |
| 150 | PR000483 VL Sequence | SYELTQPPSVSVSPGQTASITCSGDNIGDQYAHWYQQKPGQSP VLVIYQDKNRPSGIPERFSGSNSGNTATLTISGTQAMDEADYY CATYTGFGSLAVFGGGTKLTVL |
| 77 | PR000483 VL FWR1 (Chothia) | SYELTQPPSVSVSPGQTASITC |
| 87 | PR000483 VL CDR1 (Chothia) | SGDNIGDQYAH |
| 95 | PR000483 VL FWR2 (Chothia) | WYQQKPGQSPVLVIY |
| 105 | PR000483 VL CDR2 (Chothia) | QDKNRPS |
| 114 | PR000483 VL FWR3 (Chothia) | GIPERFSGSNSGNTATLTISGTQAMDEADYYC |
| 123 | PR000483 VL CDR3 (Chothia) | ATYTGFGSLAV |
| 127 | PR000483 VL FWR4 (Chothia) | FGGGTKLTVL |
| SEQ ID NO | PR000929 | anti-HSA Alb23-his |
| 163 | PR000929 Heavy Chain Sequence | EVQLLESGGGLVQPGGSLRLSCAASGFTFRSFGMSWVRQAPG KGPEWVSSISGSGSDTLYADSVKGRFTISRDNSKNTLYLQMN SLRPEDTAVYYCTIGGSLSRSSQGTLVTVSSASHHHHHH |
| 140 | PR000929 VH Sequence | EVQLLESGGGLVQPGGSLRLSCAASGFTFRSFGMSWVRQAPG KGPEWVSSISGSGSDTLYADSVKGRFTISRDNSKNTLYLQMN SLRPEDTAVYYCTIGGSLSRSSQGTLVTVSS |
| 10 | PR000929 VH FWR1 (Chothia) | EVQLLESGGGLVQPGGSLRLSCAAS |
| 20 | PR000929 VH CDR1 (Chothia) | GFTFRSF |
| 31 | PR000929 VH FWR2 (Chothia) | GMSWVRQAPGKGPEWVSSI |
| 43 | PR000929 VH CDR2 (Chothia) | SGSGSD |

(continued)

| SEQ ID NO | PR000929 | anti-HSA Alb23-his |
|---|---|---|
| 55 | PR000929 VH FWR3 (Chothia) | TLYADSVKGRFTISRDNSKNTLYLQMNSLRPEDTAVYYCTI |
| 66 | PR000929 VH CDR3 (Chothia) | GGSLSR |
| 72 | PR000929 VH FWR4 (Chothia) | SSQGTLVTVSS |

**Table 9: Sequence number and corresponding amino acid sequences of the polypeptide chains of the "κ/λ" Fab-Fab antibody constructed in the present application**

| SEQ ID NO | PR000317 | Anti-B7H4xCD3 1D11-SP34 Fab-Fab |
|---|---|---|
| 177 | Polypeptide chain-1 | EVQLVQSGAEVKKPGASVKVSCKASGYTFTSYWIGWVRQAPGQGLEWIGDIYPGGGYTNYNEKFKGRVTITRDTSTSTAYLELSSLRSEDTAVYYCARLAGSSYRGAMDSWGQGTLVTVSSASTKGPSVFPLAPSSKSTSGGTAALGCLVKDYFPEPVTVSWNSGALTSGVHTFPAVLQSSGLYSLSSVVTVPSSSLGTQTYICNVNHKPSNTKVDKKVEPKSC |
| 178 | Polypeptide chain-2 | DIQMTQSPSSLSASVGDRVTITCKASQGFNKYVAWYQQKPGKAPKLLIYYTSTLQPGVPSRFSGSGSGRDYTLTISSLQPEDFATYYCLQYGDLLYAFGQGTKVEIKRTVAAPSVFIFPPSDEQLKSGTASVVCLLNNFYPREAKVQWKVDNALQSGNSQESVTEQDSKDSTYSLSSTLTLSKADYEKHKVYACEVTHQGLSSPVTKSFNRGECEVQLVESGGGLVQPKGSLKLSCAASGFTFNTYAMNWVRQAPGKGLEWVARIRSKYNNYATYYADSVKDRFTISRDDSQSILYLQMNNLKTEDTAMYYCVRHGNFGNSYVSWFAYWGQGTLVTVSAASTKGPSVFPLAPSSKSTSGGTAALGCLVKDYFPEPVTVSWNSGALTSGVHTFPAVLQSSGLYSLSSVVTVPSSSLGTQTYICNVNHKPSNTKVDKKVEPKSC |

| SEQ ID NO | PR000317 | Anti-B7H4xCD3 1D11-SP34 Fab-Fab |
| --- | --- | --- |
| 168 | Polypeptide chain-3 | QAVVTQESALTTSPGETVTLTCRSTGAVTTSNYANWVQEKP DHLFTGLIGGTNKRAPGVPARFSGSLIGDKAALTITGAQTEDE AIYFCALWYSNLWVFGGGTKLTVLGQPKAAPSVTLFPPSSEEL QANKATLVCLISDFYPGAVTVAWKADSSPVKAGVETTTPSKQ SNNKYAASSYLSLTPEQWKSHRSYSCQVTHEGSTVEKTVAPT ECS |
| | | |
| SEQ ID NO | PR001325 | anti-B7H4xCD3 1D11C2-hzSP34_PR512 Fab-Fab-(G4S)2-Alb23 |
| 177 | Polypeptide chain-1 | EVQLVQSGAEVKKPGASVKVSCKASGYTFTSYWIGWVRQAP GQGLEWIGDIYPGGGYTNYNEKFKGRVTITRDTSTSTAYLELS SLRSEDTAVYYCARLAGSSYRGAMDSWGQGTLVTVSSASTK GPSVFPLAPSSKSTSGGTAALGCLVKDYFPEPVTVSWNSGALT SGVHTFPAVLQSSGLYSLSSVVTVPSSSLGTQTYICNVNHKPS NTKVDKKVEPKSC |
| 187 | Polypeptide chain-2 | DIQMTQSPSSLSASVGDRVTITCKASQGFNKYVAWYQQKPGK APKLLIYYTSTLQPGVPSRFSGSGSGRDYTLTISSLQPEDFATY YCLQYGDLLYAFGQGTKVEIKRTVAAPSVFIFPPSDEQLKSGT ASVVCLLNNFYPREAKVQWKVDNALQSGNSQESVTEQDSKD STYSLSSTLTLSKADYEKHKVYACEVTHQGLSSPVTKSFNRGE CEVQLVESGGGLVQPGGSLKLSCAASGFTFNTYAMNWVRQA SGKGLEWVGRIRSKYNNYATYYADSVKDRFTISRDDSKSTLY LQMNSLKTEDTAVYYCVRHGNFGNSYVSWFAYWGQGTLVT VSSASTKGPSVFPLAPSSKSTSGGTAALGCLVKDYFPEPVTVS WNSGALTSGVHTFPAVLQSSGLYSLSSVVTVPSSSLGTQTYIC NVNHKPSNTKVDKKVEPKSCGGGGSGGGGSEVQLLESGGGL VQPGGSLRLSCAASGFTFRSFGMSWVRQAPGKGPEWVSSISG SGSDTLYADSVKGRFTISRDNSKNTLYLQMNSLRPEDTAVYY CTIGGSLSRSSQGTLVTVSS |

(continued)

| SEQ ID NO | PR001325 | anti-B7H4xCD3 1D11C2-hzSP34_PR512 Fab-Fab-(G4S)2-Alb23 |
|---|---|---|
| 174 | Polypeptide chain-3 | QAVVTQEPSLTVSPGGTVTLTCRSSTGAVTTSNYANWVQQKP GQAPRGLIGGTNKRAPWTPARFSGSLLGDKAALTLLGAQPED EAEYFCALWYSNLWVFGGGTKLTVLGQPKAAPSVTLFPPSSE ELQANKATLVCLISDFYPGAVTVAWKADSSPVKAGVETTTPS KQSNNKYAASSYLSLTPEQWKSHRSYSCQVTHEGSTVEKTVA PTECS |
| | | |
| SEQ ID NO | PR001197 | anti-PSMAxCD3 pasotuxizumab-hzSP34_PR512 Fab-Fab |
| 179 | Polypeptide chain-1 | QVQLVESGGGLVKPGESLRLSCAASGFTFSDYYMYWVRQAP GKGLEWVAIISDGGYYTYYSDIIKGRFTISRDNAKNSLYLQMN SLKAEDTAVYYCARGFPLLRHGAMDYWGQGTLVTVSSASTK GPSVFPLAPSSKSTSGGTAALGCLVKDYFPEPVTVSWNSGALT SGVHTFPAVLQSSGLYSLSSVVTVPSSSLGTQTYICNVNHKPS NTKVDKKVEPKSC |
| 180 | Polypeptide chain-2 | DIQMTQSPSSLSASVGDRVTITCKASQNVDTNVAWYQQKPGQ APKSLIYSASYRYSDVPSRFSGSASGTDFTLTISSVQSEDFATY YCQQYDSYPYTFGGGTKLEIKRTVAAPSVFIFPPSDEQLKSGT ASVVCLLNNFYPREAKVQWKVDNALQSGNSQESVTEQDSKD STYSLSSTLTLSKADYEKHKVYACEVTHQGLSSPVTKSFNRGE CEVQLVESGGGLVQPGGSLKLSCAASGFTFNTYAMNWVRQA SGKGLEWVGRIRSKYNNYATYYADSVKDRFTISRDDSKSTLY LQMNSLKTEDTAVYYCVRHGNFGNSYVSWFAYWGQGTLVT VSSASTKGPSVFPLAPSSKSTSGGTAALGCLVKDYFPEPVTVS WNSGALTSGVHTFPAVLQSSGLYSLSSVVTVPSSSLGTQTYIC NVNHKPSNTKVDKKVEPKSC |

(continued)

| SEQ ID NO | PR001197 | anti-PSMAxCD3 pasotuxizumab-hzSP34_PR512 Fab-Fab |
|---|---|---|
| 174 | Polypeptide chain-3 | QAVVTQEPSLTVSPGGTVTLTCRSSTGAVTTSNYANWVQQKPGQAPRGLIGGTNKRAPWTPARFSGSLLGDKAALTLLGAQPEDEAEYFCALWYSNLWVFGGGTKLTVLGQPKAAPSVTLFPPSSEELQANKATLVCLISDFYPGAVTVAWKADSSPVKAGVETTTPSKQSNNKYAASSYLSLTPEQWKSHRSYSCQVTHEGSTVEKTVAPTECS |
| | | |

| SEQ ID NO | PR001198 | anti-BCMAxCD3 belantamab-hzSP34_PR512 Fab-Fab |
|---|---|---|
| 181 | Polypeptide chain-1 | QVQLVQSGAEVKKPGSSVKVSCKASGGTFSNYWMHWVRQAPGQGLEWMGATYRGHSDTYYNQKFKGRVTITADKSTSTAYMELSSLRSEDTAVYYCARGAIYDGYDVLDNWGQGTLVTVSSASTKGPSVFPLAPSSKSTSGGTAALGCLVKDYFPEPVTVSWNSGALTSGVHTFPAVLQSSGLYSLSSVVTVPSSSLGTQTYICNVNHKPSNTKVDKKVEPKSC |
| 182 | Polypeptide chain-2 | DIQMTQSPSSLSASVGDRVTITCSASQDISNYLNWYQQKPGKAPKLLIYYTSNLHSGVPSRFSGSGSGTDFTLTISSLQPEDFATYYCQQYRKLPWTFGQGTKLEIKRTVAAPSVFIFPPSDEQLKSGTASVVCLLNNFYPREAKVQWKVDNALQSGNSQESVTEQDSKDSTYSLSSTLTLSKADYEKHKVYACEVTHQGLSSPVTKSFNRGECEVQLVESGGGLVQPGGSLKLSCAASGFTFNTYAMNWVRQASGKGLEWVGRIRSKYNNYATYYADSVKDRFTISRDDSKSTLYLQMNSLKTEDTAVYYCVRHGNFGNSYVSWFAYWGQGTLVTVSSASTKGPSVFPLAPSSKSTSGGTAALGCLVKDYFPEPVTVSWNSGALTSGVHTFPAVLQSSGLYSLSSVVTVPSSSLGTQTYICNVNHKPSNTKVDKKVEPKSC |
| 174 | Polypeptide chain-3 | QAVVTQEPSLTVSPGGTVTLTCRSSTGAVTTSNYANWVQQKPGQAPRGLIGGTNKRAPWTPARFSGSLLGDKAALTLLGAQPEDEAEYFCALWYSNLWVFGGGTKLTVLGQPKAAPSVTLFPPSSEELQANKATLVCLISDFYPGAVTVAWKADSSPVKAGVETTTPSKQSNNKYAASSYLSLTPEQWKSHRSYSCQVTHEGSTVEKTVAPTECS |

(continued)

| SEQ ID NO | PR001198 | anti-BCMAxCD3 belantamab-hzSP34_PR512 Fab-Fab |
|---|---|---|
| | | |
| SEQ ID NO | PR001200 | anti-ROR1xCD3 R11H18L9-hzSP34_PR512 Fab-Fab |
| 183 | Polypeptide chain-1 | EVQLVESGGGLVQPGRSLRLSCTASGSDINDYPITWVRQAPGQGLEWIGFINSGGSTWYASWVKGRFTISRDDSKSIAYLQMNSLKTEDTAVYYCARGYSTYYRDFNIWGQGTLVTVSSASTKGPSVFPLAPSSKSTSGGTAALGCLVKDYFPEPVTVSWNSGALTSGVHTFPAVLQSSGLYSLSSVVTVPSSSLGTQTYICNVNHKPSNTKVDKKVEPKSC |
| 184 | Polypeptide chain-2 | DIQMTQSPSSLSASVGDRVTINCQASQSIDSNLAWFQQKPGQPPKLLIYRASNLASGVPDRFSGSGSGTDFTLTISSLEAEDVATYYCLGGVGAVSYRTSFGGGTKVEIKRTVAAPSVFIFPPSDEQLKSGTASVVCLLNNFYPREAKVQWKVDNALQSGNSQESVTEQDSKDSTYSLSSTLTLSKADYEKHKVYACEVTHQGLSSPVTKSFNRGECEVQLVESGGGLVQPGGSLKLSCAASGFTFNTYAMNWVRQASGKGLEWVGRIRSKYNNYATYYADSVKDRFTISRDDSKSTLYLQMNSLKTEDTAVYYCVRHGNFGNSYVSWFAYWGQGTLVTVSSASTKGPSVFPLAPSSKSTSGGTAALGCLVKDYFPEPVTVSWNSGALTSGVHTFPAVLQSSGLYSLSSVVTVPSSSLGTQTYICNVNHKPSNTKVDKKVEPKSC<br><br>QAVVTQEPSLTVSPGGTVTLTCRSSTGAVTTSNYANWVQQKPGQAPRGLIGGTNKRAPWTPARFSGSLLGDKAALTLLGAQPEDEAEYFCALWYSNLWVFGGGTKLTVLGQPKAAPSVTLFPPSSEELQANKATLVCLISDFYPGAVTVAWKADSSPVKAGVETTTPSKQSNNKYAASSYLSLTPEQWKSHRSYSCQVTHEGSTVEKTVAPTECS |

| SEQ ID NO | PR001200 | anti-ROR1xCD3 R11H18L9-hzSP34_PR512 Fab-Fab |
|---|---|---|
| 174 | Polypeptide chain-3 | QAVVTQEPSLTVSPGGTVTLTCRSSTGAVTTSNYANWVQQKPGQAPRGLIGGTNKRAPWTPARFSGSLLGDKAALTLLGAQPEDEAEYFCALWYSNLWVFGGGTKLTVLGQPKAAPSVTLFPPSSEELQANKATLVCLISDFYPGAVTVAWKADSSPVKAGVETTTPSKQSNNKYAASSYLSLTPEQWKSHRSYSCQVTHEGSTVEKTVAPTECS |
| | | |
| SEQ ID NO | PR001310 | anti-HELxCD3 HYHEL5-hzSP34_PR512 Fab-Fab |
| 185 | Polypeptide chain-1 | EVQLQQSGAELMKPGASVKISCKASGYTFSDYWIEWVKQRPGHGLEWIGEILPGSGSTNYHERFKGKATFTADTSSSTAYMQLNSLTSEDSGVYYCLHGNYDFDGWGQGTTLTVSSASTKGPSVFPLAPSSKSTSGGTAALGCLVKDYFPEPVTVSWNSGALTSGVHTFPAVLQSSGLYSLSSVVTVPSSSLGTQTYICNVNHKPSNTKVDKKVEPKSC |
| 186 | Polypeptide chain-2 | DIVLTQSPAIMSASPGEKVTMTCSASSSVNYMYWYQQKSGTSPKRWIYDTSKLASGVPVRFSGSGSGTSYSLTISSMETEDAAEYYCQQWGRNPTFGGGTKLEIKRTVAAPSVFIFPPSDEQLKSGTASVVCLLNNFYPREAKVQWKVDNALQSGNSQESVTEQDSKDSTYSLSSTLTLSKADYEKHKVYACEVTHQGLSSPVTKSFNRGECEVQLVESGGGLVQPGGSLKLSCAASGFTFNTYAMNWVRQASGKGLEWVGRIRSKYNNYATYYADSVKDRFTISRDDSKSTLYLQMNSLKTEDTAVYYCVRHGNFGNSYVSWFAYWGQGTLVTVSSASTKGPSVFPLAPSSKSTSGGTAALGCLVKDYFPEPVTVSWNSGALTSGVHTFPAVLQSSGLYSLSSVVTVPSSSLGTQTYICNVNHKPSNTKVDKKVEPKSC |
| 174 | Polypeptide chain-3 | QAVVTQEPSLTVSPGGTVTLTCRSSTGAVTTSNYANWVQQKPGQAPRGLIGGTNKRAPWTPARFSGSLLGDKAALTLLGAQPEDEAEYFCALWYSNLWVFGGGTKLTVLGQPKAAPSVTLFPPSSEELQANKATLVCLISDFYPGAVTVAWKADSSPVKAGVETTTPSKQSNNKYAASSYLSLTPEQWKSHRSYSCQVTHEGSTVEKTVAPTECS |

(continued)

| SEQ ID NO | PR001310 | anti-HELxCD3 HYHEL5-hzSP34_PR512 Fab-Fab |
|---|---|---|
| | | |
| SEQ ID NO | PR001613 | anti-B7H4xCD16 1D11C2-CD16A-1 Fab-Fab |
| 177 | Polypeptide chain-1 | EVQLVQSGAEVKKPGASVKVSCKASGYTFTSYWIGWVRQAPGQGLEWIGDIYPGGGYTNYNEKFKGRVTITRDTSTSTAYLELSSLRSEDTAVYYCARLAGSSYRGAMDSWGQGTLVTVSSASTKGPSVFPLAPSSKSTSGGTAALGCLVKDYFPEPVTVSWNSGALTSGVHTFPAVLQSSGLYSLSSVVTVPSSSLGTQTYICNVNHKPSNTKVDKKVEPKSC |
| 188 | Polypeptide chain-2 | DIQMTQSPSSLSASVGDRVTITCKASQGFNKYVAWYQQKPGKAPKLLIYYTSTLQPGVPSRFSGSGSGRDYTLTISSLQPEDFATYYCLQYGDLLYAFGQGTKVEIKRTVAAPSVFIFPPSDEQLKSGTASVVCLLNNFYPREAKVQWKVDNALQSGNSQESVTEQDSKDSTYSLSSTLTLSKADYEKHKVYACEVTHQGLSSPVTKSFNRGECQVQLVQSGAEVKKPGESLKVSCKASGYTFTSYYMHWVRQAPGQGLEWMGAIEPMYGSTSYAQKFQGRVTMTRDTSTSTVYMELSSLRSEDTAVYYCARGSAYYYDFADYWGQGTLVTVSSASTKGPSVFPLAPSSKSTSGGTAALGCLVKDYFPEPVTVSWNSGALTSGVHTFPAVLQSSGLYSLSSVVTVPSSSLGTQTYICNVNHKPSNTKVDKKVEPKSC |
| 176 | Polypeptide chain-3 | SYVLTQPSSVSVAPGQTATISCGGHNIGSKNVHWYQQRPGQSPVLVIYQDNKRPSGIPERFSGSNSGNTATLTISGTQAMDEADYYCQVWDNYSVLFGGGTKLTVLGQPKAAPSVTLFPPSSEELQANKATLVCLISDFYPGAVTVAWKADSSPVKAGVETTTPSKQSNNKYAASSYLSLTPEQWKSHRSYSCQVTHEGSTVEKTVAPTECS |
| | | |

(continued)

| SEQ ID NO | PR001614 | anti-B7H4xCD16 1D11C2-CD16A-2 Fab-Fab |
|---|---|---|
| 177 | Polypeptide chain-1 | EVQLVQSGAEVKKPGASVKVSCKASGYTFTSYWIGWVRQAPGQGLEWIGDIYPGGGYTNYNEKFKGRVTITRDTSTSTAYLELSSLRSEDTAVYYCARLAGSSYRGAMDSWGQGTLVTVSSASTKGPSVFPLAPSSKSTSGGTAALGCLVKDYFPEPVTVSWNSGALTSGVHTFPAVLQSSGLYSLSSVVTVPSSSLGTQTYICNVNHKPSNTKVDKKVEPKSC |
| 189 | Polypeptide chain-2 | DIQMTQSPSSLSASVGDRVTITCKASQGFNKYVAWYQQKPGKAPKLLIYYTSTLQPGVPSRFSGSGSGRDYTLTISSLQPEDFATYYCLQYGDLLYAFGQGTKVEIKRTVAAPSVFIFPPSDEQLKSGTASVVCLLNNFYPREAKVQWKVDNALQSGNSQESVTEQDSKDSTYSLSSTLTLSKADYEKHKVYACEVTHQGLSSPVTKSFNRGECQVQLVQSGAEVKKPGESLKVSCKASGYTFTSYYMHWVRQAPGQGLEWMGIINPSGGSTSYAQKFQGRVTMTRDTSTSTVYMELSSLRSEDTAVYYCARGSAYYYDFADYWGQGTLVTVSSASTKGPSVFPLAPSSKSTSGGTAALGCLVKDYFPEPVTVSWNSGALTSGVHTFPAVLQSSGLYSLSSVVTVPSSSLGTQTYICNVNHKPSNTKVDKKVEPKSC |
| 176 | Polypeptide chain-3 | SYVLTQPSSVSVAPGQTATISCGGHNIGSKNVHWYQQRPGQSPVLVIYQDNKRPSGIPERFSGSNSGNTATLTISGTQAMDEADYYCQVWDNYSVLFGGGTKLTVLGQPKAAPSVTLFPPSSEELQANKATLVCLISDFYPGAVTVAWKADSSPVKAGVETTTPSKQSNNKYAASSYLSLTPEQWKSHRSYSCQVTHEGSTVEKTVAPTECS |
|  |  |  |

| SEQ ID NO | PR001615 | anti-BCMAxCD16 belantamab-CD16A-1 Fab-Fab |
|---|---|---|
| 181 | Polypeptide chain-1 | QVQLVQSGAEVKKPGSSVKVSCKASGGTFSNYWMHWVRQAPGQGLEWMGATYRGHSDTYYNQKFKGRVTITADKSTSTAYMELSSLRSEDTAVYYCARGAIYDGYDVLDNWGQGTLVTVSSASTKGPSVFPLAPSSKSTSGGTAALGCLVKDYFPEPVTVSWNSGALTSGVHTFPAVLQSSGLYSLSSVVTVPSSSLGTQTYICNVNHKPSNTKVDKKVEPKSC |
| 190 | Polypeptide chain-2 | DIQMTQSPSSLSASVGDRVTITCSASQDISNYLNWYQQKPGKAPKLLIYYTSNLHSGVPSRFSGSGSGTDFTLTISSLQPEDFATYYCQQYRKLPWTFGQGTKLEIKRTVAAPSVFIFPPSDEQLKSGTASVVCLLNNFYPREAKVQWKVDNALQSGNSQESVTEQDSKDSTYSLSSTLTLSKADYEKHKVYACEVTHQGLSSPVTKSFNRGECQVQLVQSGAEVKKPGESLKVSCKASGYTFTSYYMHWVRQAPGQGLEWMGAIEPMYGSTSYAQKFQGRVTMTRDTSTSTVYMELSSLRSEDTAVYYCARGSAYYYDFADYWGQGTLVTVSSASTKGPSVFPLAPSSKSTSGGTAALGCLVKDYFPEPVTVSWNSGALTSGVHTFPAVLQSSGLYSLSSVVTVPSSSLGTQTYICNVNHKPSNTKVDKKVEPKSC |
| 176 | Polypeptide chain-3 | SYVLTQPSSVSVAPGQTATISCGGHNIGSKNVHWYQQRPGQSPVLVIYQDNKRPSGIPERFSGSNSGNTATLTISGTQAMDEADYYCQVWDNYSVLFGGGTKLTVLGQPKAAPSVTLFPPSSEELQANKATLVCLISDFYPGAVTVAWKADSSPVKAGVETTTPSKQSNNKYAASSYLSLTPEQWKSHRSYSCQVTHEGSTVEKTVAPTECS |
|  |  |  |
| SEQ ID NO | PR001616 | anti-BCMAxCD16 belantamab-CD16A-2 Fab-Fab |
| 181 | Polypeptide chain-1 | QVQLVQSGAEVKKPGSSVKVSCKASGGTFSNYWMHWVRQAPGQGLEWMGATYRGHSDTYYNQKFKGRVTITADKSTSTAYMELSSLRSEDTAVYYCARGAIYDGYDVLDNWGQGTLVTVSSASTKGPSVFPLAPSSKSTSGGTAALGCLVKDYFPEPVTVSWNSGALTSGVHTFPAVLQSSGLYSLSSVVTVPSSSLGTQTYICNVNHKPSNTKVDKKVEPKSC |

(continued)

| SEQ ID NO | PR001616 | anti-BCMAxCD16 belantamab-CD16A-2 Fab-Fab |
|---|---|---|
| 191 | Polypeptide chain-2 | DIQMTQSPSSLSASVGDRVTITCSASQDISNYLNWYQQKPGKA PKLLIYYTSNLHSGVPSRFSGSGSGTDFTLTISSLQPEDFATYYC QQYRKLPWTFGQGTKLEIKRTVAAPSVFIFPPSDEQLKSGTAS VVCLLNNFYPREAKVQWKVDNALQSGNSQESVTEQDSKDST YSLSSTLTLSKADYEKHKVYACEVTHQGLSSPVTKSFNRGEC QVQLVQSGAEVKKPGESLKVSCKASGYTFTSYYMHWVRQAP GQGLEWMGIINPSGGSTSYAQKFQGRVTMTRDTSTSTVYMEL SSLRSEDTAVYYCARGSAYYYDFADYWGQGTLVTVSSASTK GPSVFPLAPSSKSTSGGTAALGCLVKDYFPEPVTVSWNSGALT SGVHTFPAVLQSSGLYSLSSVVTVPSSSLGTQTYICNVNHKPS NTKVDKKVEPKSC |
| 176 | Polypeptide chain-3 | SYVLTQPSSVSVAPGQTATISCGGHNIGSKNVHWYQQRPGQS PVLVIYQDNKRPSGIPERFSGSNSGNTATLTISGTQAMDEADY YCQVWDNYSVLFGGGTKLTVLGQPKAAPSVTLFPPSSEELQA NKATLVCLISDFYPGAVTVAWKADSSPVKAGVETTTPSKQSN NKYAASSYLSLTPEQWKSHRSYSCQVTHEGSTVEKTVAPTEC S |
|  |  |  |

| SEQ ID NO | PR001941 | anti-HELxCD137 HYHEL5-urelumab Fab-Fab |
|---|---|---|
| 185 | Polypeptide chain-1 | EVQLQQSGAELMKPGASVKISCKASGYTFSDYWIEWVKQRPG HGLEWIGEILPGSGSTNYHERFKGKATFTADTSSSTAYMQLNS LTSEDSGVYYCLHGNYDFDGWGQGTTLTVSSASTKGPSVFPL APSSKSTSGGTAALGCLVKDYFPEPVTVSWNSGALTSGVHTFP AVLQSSGLYSLSSVVTVPSSSLGTQTYICNVNHKPSNTKVDKK VEPKSC |

| SEQ ID NO | PR001941 | anti-HELxCD137 HYHEL5-urelumab Fab-Fab |
|---|---|---|
| 193 | Polypeptide chain-2 | DIVLTQSPAIMSASPGEKVTMTCSASSSVNYMYWYQQKSGTS PKRWIYDTSKLASGVPVRFSGSGSGTSYSLTISSMETEDAAEY YCQQWGRNPTFGGGTKLEIKRTVAAPSVFIFPPSDEQLKSGTA SVVCLLNNFYPREAKVQWKVDNALQSGNSQESVTEQDSKDS TYSLSSTLTLSKADYEKHKVYACEVTHQGLSSPVTKSFNRGEC QVQLQQWGAGLLKPSETLSLTCAVYGGSFSGYYWSWIRQSPE KGLEWIGEINHGGYVTYNPSLESRVTISVDTSKNQFSLKLSSVT AADTAVYYCARDYGPGNYDWYFDLWGRGTLVTVSSASTKG PSVFPLAPSSKSTSGGTAALGCLVKDYFPEPVTVSWNSGALTS GVHTFPAVLQSSGLYSLSSVVTVPSSSLGTQTYICNVNHKPSN TKVDKKVEPKSC |
| 192 | Polypeptide chain-3 | EIVLTQSPATLSLSPGERATLSCRASQSVSSYLAWYQQKPGQA PRLLIYDASNRATGIPARFSGSGSGTDFTLTISSLEPEDFAVYYC QQRSNWPPALTFGGGTKVTVLGQPKAAPSVTLFPPSSEELQA NKATLVCLISDFYPGAVTVAWKADSSPVKAGVETTTPSKQSN NKYAASSYLSLTPEQWKSHRSYSCQVTHEGSTVEKTVAPTEC S |
|  |  |  |

| SEQ ID NO | PR001942 | anti-HELxCD137 HYHEL5-utomilumab Fab-Fab |
|---|---|---|
| 185 | Polypeptide chain-1 | EVQLQQSGAELMKPGASVKISCKASGYTFSDYWIEWVKQRPG HGLEWIGEILPGSGSTNYHERFKGKATFTADTSSSTAYMQLNS LTSEDSGVYYCLHGNYDFDGWGQGTTLTVSSASTKGPSVFPL APSSKSTSGGTAALGCLVKDYFPEPVTVSWNSGALTSGVHTFP AVLQSSGLYSLSSVVTVPSSSLGTQTYICNVNHKPSNTKVDKK VEPKSC |

(continued)

| SEQ ID NO | PR001942 | anti-HELxCD137 HYHEL5-utomilumab Fab-Fab |
|---|---|---|
| 194 | Polypeptide chain-2 | DIVLTQSPAIMSASPGEKVTMTCSASSSVNYMYWYQQKSGTSPKRWIYDTSKLASGVPVRFSGSGSGTSYSLTISSMETEDAAEYYCQQWGRNPTFGGGTKLEIKRTVAAPSVFIFPPSDEQLKSGTASVVCLLNNFYPREAKVQWKVDNALQSGNSQESVTEQDSKDSTYSLSSTLTLSKADYEKHKVYACEVTHQGLSSPVTKSFNRGECEVQLVQSGAEVKKPGESLRISCKGSGYSFSTYWISWVRQMPGKGLEWMGKIYPGDSYTNYSPSFQGQVTISADKSISTAYLQWSSLKASDTAMYYCARGYGIFDYWGQGTLVTVSSASTKGPSVFPLAPSSKSTSGGTAALGCLVKDYFPEPVTVSWNSGALTSGVHTFPAVLQSSGLYSLSSVVTVPSSSLGTQTYICNVNHKPSNTKVDKKVEPKSC |
| 173 | Polypeptide chain-3 | SYELTQPPSVSVSPGQTASITCSGDNIGDQYAHWYQQKPGQSPVLVIYQDKNRPSGIPERFSGSNSGNTATLTISGTQAMDEADYYCATYTGFGSLAVFGGGTKLTVLGQPKAAPSVTLFPPSSEELQANKATLVCLISDFYPGAVTVAWKADSSPVKAGVETTTPSKQSNNKYAASSYLSLTPEQWKSHRSYSCQVTHEGSTVEKTVAPTECS |
| | | |

| SEQ ID NO | PR001944 | anti-B7H4xCD137 1D11C2-urelumab Fab-Fab |
|---|---|---|
| 177 | Polypeptide chain-1 | EVQLVQSGAEVKKPGASVKVSCKASGYTFTSYWIGWVRQAPGQGLEWIGDIYPGGGYTNYNEKFKGRVTITRDTSTSTAYLELSSLRSEDTAVYYCARLAGSSYRGAMDSWGQGTLVTVSSASTKGPSVFPLAPSSKSTSGGTAALGCLVKDYFPEPVTVSWNSGALTSGVHTFPAVLQSSGLYSLSSVVTVPSSSLGTQTYICNVNHKPSNTKVDKKVEPKSC |

| SEQ ID NO | PR001944 | anti-B7H4xCD137 1D11C2-urelumab Fab-Fab |
|---|---|---|
| 195 | Polypeptide chain-2 | DIQMTQSPSSLSASVGDRVTITCKASQGFNKYVAWYQQKPGK APKLLIYYTSTLQPGVPSRFSGSGSGRDYTLTISSLQPEDFATY YCLQYGDLLYAFGQGTKVEIKRTVAAPSVFIFPPSDEQLKSGT ASVVCLLNNFYPREAKVQWKVDNALQSGNSQESVTEQDSKD STYSLSSTLTLSKADYEKHKVYACEVTHQGLSSPVTKSFNRGE CQVQLQQWGAGLLKPSETLSLTCAVYGGSFSGYYWSWIRQSP EKGLEWIGEINHGGYVTYNPSLESRVTISVDTSKNQFSLKLSSV TAADTAVYYCARDYGPGNYDWYFDLWGRGTLVTVSSASTK GPSVFPLAPSSKSTSGGTAALGCLVKDYFPEPVTVSWNSGALT SGVHTFPAVLQSSGLYSLSSVVTVPSSSLGTQTYICNVNHKPS NTKVDKKVEPKSC |
| 192 | Polypeptide chain-3 | EIVLTQSPATLSLSPGERATLSCRASQSVSSYLAWYQQKPGQA PRLLIYDASNRATGIPARFSGSGSGTDFTLTISSLEPEDFAVYYC QQRSNWPPALTFGGGTKVTVLGQPKAAPSVTLFPPSSEELQA NKATLVCLISDFYPGAVTVAWKADSSPVKAGVETTTPSKQSN NKYAASSYLSLTPEQWKSHRSYSCQVTHEGSTVEKTVAPTEC S |
| | | |
| SEQ ID NO | PR001945 | anti-B7H4xCD137 1D11C2-utomilumab Fab-Fab |
| 177 | Polypeptide chain-1 | EVQLVQSGAEVKKPGASVKVSCKASGYTFTSYWIGWVRQAP GQGLEWIGDIYPGGGYTNYNEKFKGRVTITRDTSTSTAYLELS SLRSEDTAVYYCARLAGSSYRGAMDSWGQGTLVTVSSASTK GPSVFPLAPSSKSTSGGTAALGCLVKDYFPEPVTVSWNSGALT SGVHTFPAVLQSSGLYSLSSVVTVPSSSLGTQTYICNVNHKPS NTKVDKKVEPKSC |
| 196 | Polypeptide chain-2 | DIQMTQSPSSLSASVGDRVTITCKASQGFNKYVAWYQQKPGK APKLLIYYTSTLQPGVPSRFSGSGSGRDYTLTISSLQPEDFATY YCLQYGDLLYAFGQGTKVEIKRTVAAPSVFIFPPSDEQLKSGT |

(continued)

| SEQ ID NO | PR001945 | anti-B7H4xCD137 1D11C2-utomilumab Fab-Fab |
|---|---|---|
| | | ASVVCLLNNFYPREAKVQWKVDNALQSGNSQESVTEQDSKD STYSLSSTLTLSKADYEKHKVYACEVTHQGLSSPVTKSFNRGE CEVQLVQSGAEVKKPGESLRISCKGSGYSFSTYWISWVRQMP GKGLEWMGKIYPGDSYTNYSPSFQGQVTISADKSISTAYLQW SSLKASDTAMYYCARGYGIFDYWGQGTLVTVSSASTKGPSVF PLAPSSKSTSGGTAALGCLVKDYFPEPVTVSWNSGALTSGVH TFPAVLQSSGLYSLSSVVTVPSSSLGTQTYICNVNHKPSNTKV DKKVEPKSC |
| 173 | Polypeptide chain-3 | SYELTQPPSVSVSPGQTASITCSGDNIGDQYAHWYQQKPGQSP VLVIYQDKNRPSGIPERFSGSNSGNTATLTISGTQAMDEADYY CATYTGFGSLAVFGGGTKLTVLGQPKAAPSVTLFPPSSEELQA NKATLVCLISDFYPGAVTVAWKADSSPVKAGVETTTPSKQSN NKYAASSYLSLTPEQWKSHRSYSCQVTHEGSTVEKTVAPTEC S |

**Example 5 Binding of "κ/λ" Fab-Fab antigen binding proteins to target cell surface antigen molecules**

B7H4xCD3 "κ/λ" Fab-Fab antibodies (PR000317 and PR001325) were used in this Example. The binding of the N-terminal Fab and C-terminal Fab of the antigen-binding protein with a "κ/λ" Fab-Fab structure to their respective cell surface target molecules, as well as the binding ability to the two targets simultaneously, were studied by Flow cytometry (FACS). The structure of PR000317 is shown in Fig. 10(A), and the structure of PR001325 is shown in Fig. 10(C). Their sequences are summarized in Table 9. In the structures of PR000317 and PR001325, the N-terminal Fab was a Fab binding to B7H4, and the C-terminal Fab was a Fab binding to CD3.

**Example 5.1 Test of the binding ability of the N-terminal Fab**

FACS was used to test the binding ability of the antigen-binding protein to the tumor cell line SK-BR-3 (ATCC, #HTB-30) with high B7H4 expression. In brief, the cell suspension was collected, and SK-BR-3 cells were seeded at a density of $5 \times 10^4$ cells in 50 μL per well into a 96-well V-bottom plate (Corning, #3894). Then, 50 μL per well of the antigen-binding protein that had been 3-fold serially diluted to twice the final concentration was added and mixed evenly. hIgG1 iso (CrownBio, #C0001) was used as an isotype control. The cells were placed at 4°C and incubated in the dark for 1 hour. Then, 100 μL/well of pre-cooled PBS buffer was added to rinse the cells for two times, and the supernatant was discarded after centrifugation. Subsequently, 100 μL/well of fluorescent secondary antibody was added and incubated at 4°C in the dark for 40 minutes. A buffer solution was added to rinse the cells twice, and then the supernatant was discarded after centrifugation. Finally, the cells were resuspended by adding 200 μL/well of pre-cooled PBS buffer containing 0.5% BSA. Fluorescence luminescence signal values were read using an ACEA NovoCyte flow cytometer or a BD FACS CANTOII flow cytometer, and the data were processed and analyzed using software FlowJo v10 (FlowJo, LLC). The software GraphPad Prism 8 was used for data processing and graphing analysis. Through four-parameter nonlinear fitting, the parameters such as binding curve and EC50 values of the antibody to target cells were obtained.

The results were shown in Fig. 14 and Table 10. It was demonstrated that both PR000317 and PR001325 can bind to SK-BR-3 cells in a concentration-dependent manner.

### Table 10: Binding activity of to SK-BR-3

| Antibody ID | EC50 (nM) | Maximum value of MFI |
|-------------|-----------|----------------------|
| PR001325 | 13.16 | 216897 |
| PR000317 | 21.98 | 178583 |

### Example 5.2 Test of the binding ability of the C-terminal Fab

FACS was used to test the binding ability of the antigen-binding protein to human T cells. The experimental method was similar to that described in Example 5.1, except that the target cells were replaced. The results were shown in Fig. 15. It was demonstrated that both PR000317 and PR001325 can bind to T cells in a concentration-dependent manner.

### Example 5.3 Test of the ability to bind two types of cells simultaneously

FACS was used to study whether the antigen-binding protein molecule can simultaneously bind to CHO-K1 cells with high human B7H4 expression, i.e., CHO-K1/hB7H4 (self-made by Harbour Biomed) and human T cells.

In the first step, 5 $\mu$M CFSE dye (Thermo, #C34554) and 1 $\mu$M Far red dye (Thermo, #C34564) were added to CHO-K1/hB7H4 cells and human T cells for fluorescent labeling, respectively, and incubated at 37°C for 10 minutes with shaking from time to time; and a 5-fold amount of medium was added to terminate the staining. After that, the cells were centrifuged at 4 °C for 5 minutes, the supernatant was discarded, and then PBS was added to resuspend the cells. The density of the labeled cells was adjusted to $1 \times 10^6$/ml.

In the second step, four times the final concentration of the antigen-binding protein sample to be tested was diluted by a three-fold concentration gradient.

In the third step, 25 $\mu$l of labeled CHO-K1/hB7H4 cells and 50 $\mu$l of labeled T cells were mixed evenly with 25 $\mu$l of pre-gradient diluted antigen-binding protein to be tested, and the mixture was added to a 96-well plate with a total of 8 concentrations. hIgG1 iso (CrownBio, #C0001) was used as an isotype control. Then, the samples were incubated at 4 °C for 1 hour in the dark.

In the fourth step, 100 $\mu$l of 2% paraformaldehyde (PFA) per well was added to fix cells for 10 minutes.

Finally, the fluorescence signal values were read using an ACEA NovoCyte flow cytometer and the data were processed and analyzed using software FlowJo v10 (FlowJo, LLC). The number of cells in different quadrants was counted according to different fluorescent labels. Q1 was Far-red-labeled T cells (T+); Q3 was CFSE-labeled CHO-K1/hB7H4 cells (B7H4+); and Q2 contained a population of cells with two labels (B7H4 + & T +) formed by the simultaneous binding of the bispecific antibody molecule to both cells. The proportion of cell population positive for two labels (B7H4+ & T+) relative to all labeled B7H4+ cells (Q3+Q2) was calculated, reflecting the ability of the bispecific antibody molecule to bind both cells simultaneously. The software GraphPad Prism 8 was used for data processing and graphing analysis. Through four-parameter nonlinear fitting, the curve reflecting the simultaneous binding of the antibody to the two target cells was obtained.

The results were shown in Fig. 16. It was demonstrated that only B7H4xCD3 "$\kappa/\lambda$" Fab-Fab antibodies PR000317 and PR001325 could bind both cell types simultaneously. In contrast, neither the corresponding B7H4 mAb PR000157 nor CD3 mAb PR000260 could bind both cell types simultaneously.

This experiment demonstrated that the "$\kappa/\lambda$" Fab-Fab antigen binding protein could effectively retain the binding specificity of each antigen binding domain (N-terminal Fab and C-terminal Fab), and could bind to two targets simultaneously.

### Example 6 "$\kappa/\lambda$" Fab-Fab antigen-binding protein mediated killing of target cells by effector cells

In this Example, the LDH method was used to investigate the activation of effector cells mediated by the "$\kappa/\lambda$" Fab-Fab bispecific antibody containing a CD3-binding domain and the killing of target cells.

In this Example, the "$\kappa/\lambda$" Fab-Fab bispecific antibodies used include B7H4xCD3 "$\kappa/\lambda$" Fab-Fab antibody (PR000317), PSMAxCD3 "$\kappa/\lambda$" Fab-Fab antibody (PR001197), BCMAxCD3 "$\kappa/\lambda$" Fab-Fab antibody (PR001198), ROR1xCD3 "$\kappa/\lambda$" Fab-Fab antibody (PR001200), and HELxCD3 "$\kappa/\lambda$" Fab-Fab antibody (PR001310). The effector cells may be human peripheral blood mononuclear cells (PBMC) or T cells isolated from PBMC. The target cells may be SK-BR-3 cells with high B7H4 expression (ATCC, #HTB-30), or LNCaP cells with high PSMA expression (Nanjing cobioer, #CBP60346), or NCI-H929 cells with high BCMA expression (ATCC, #CRL-9068), or HEK293 cells with high ROR1 expression (HEK293/hROR1, Kyinno Biotechnology, #KC-0615). Among them, PR001310 was used as a Fab-Fab structural control molecule. One terminus of PR001310 recognizes CD3, and the other terminus binds to hen egg white lysozyme, without binding to the listed target cells. The sequences of the said molecules are summarized in Table 9.

Briefly, effector cells (PBMC or T cells) and specific target cells were adjusted to an appropriate cell density with RPMI1640 medium containing 5% FBS, depending on the desired effector-to-target ratio (E: T). Then, both cell suspensions were seeded at 90 μL cells/well each onto a 96-well plate (Corning, #3799). Subsequently, a pre-gradient diluted antibody to be tested (gradient dilution 10 times the final concentration) was added at 20 μL/well. Samples were added in duplicate. The wells added with the antibody to be tested were recorded as ER (experimental wells, containing the antibody sample to be tested, effector cells, and target cells). And different control groups were set in the plate according to the parameters in the formula below, which were recorded as ESR (effector cell natural release wells, containing only effector cells and culture medium); TSR (target cell natural release wells, containing only target cells and culture medium); CMB (culture medium reference wells, containing only culture medium); TMR (target cell maximum release wells, containing only target cells and culture medium); VCC (volume reference wells, containing only culture medium). The 96-well plate was placed and incubated in a carbon dioxide incubator at 37°C for 24 hours. Then, 10 μL of lysate was added to the TMR wells and VCC wells, and incubation was continued for 30 minutes. After that, 50 μL/well of the supernatant was added to a 96-well plate (Corning, #:3599) and the cytotoxicity assay reagent (CytoTox 96® Non-Radioactive Cytotoxicity Assay Kit, Promega, #G1780) was added at 50 μL/well. After 30 minutes of incubation at room temperature, the reaction was stopped by adding 50 μL of reaction stopper. Finally, the absorption value was read at 490 nM using an Enspire multifunctional plate reader (Perkin Elmer, Inc.), and the killing rate of target cells was calculated according to the following formula. The software GraphPad Prism 8 was used for data processing and graphing analysis.

Killing rate = ( ( ER - CMB ) - ( ESR - CMB ) - ( TSR - CMB ) ) / ( TMR - VCC ) x 100%

Wherein:

$$ER = \text{experimental wells, sample + effector cells + target cells}$$

ESR = effector cell natural release wells, effector cells + culture medium

$$TSR = \text{target cell natural release wells, target cells + culture medium}$$

TMR = target cell maximum release wells, target cells + culture medium + lysis buffer

$$VCC = \text{volume control wells, medium + lysis buffer}$$

CMB = culture medium reference wells, culture medium

More specifically, in SK-BR-3 cell killing test, SK-BR-3 cells and human T cells were seeded in 96-well plates at $2 \times 10^4$ cells/well and $2 \times 10^5$ cells/well, respectively, and the antibody to be tested was diluted in a 5-fold gradient from the highest final concentration of 1 nM. In LNCaP cell killing test, LNCaP cells and human T cells were seeded in 96-well plates at $2 \times 10^4$ cells/well and $8 \times 10^4$ cells/well, respectively, and the antibody to be tested was diluted in a 4-fold gradient from the highest final concentration of 30 nM. In HEK293/hROR1 cell killing test, HEK293/hROR1 cells and human T cells were seeded in 96-well plates at $2 \times 10^4$ cells/well and $8 \times 10^4$ cells/well, respectively, and the antibody to be tested was diluted in a 4-fold gradient from the highest final concentration of 30 nM. In NCI-H929 cell killing test, NCI-H929 cells and human PBMC cells were seeded in 96-well plates at a ratio of 1:10, and the antibody to be tested was diluted in a 4-fold gradient from the highest final concentration of 15 nM.

The results of SK-BR-3 cell killing test were shown in Fig. 17. B7H4xCD3 bispecific antibody PR000317 could effectively kill SK-BR-3 cells with a cell killing activity EC50 of about 1.8 pM and a maximum killing rate of about 34% (Table 11). In contrast, the corresponding B7H4 monoclonal antibody PR000157 and CD3 monoclonal antibody PR000260 did not cause significant cell killing.

The results of LNCaP cell killing test were shown in Fig. 21. PSMAxCD3 bispecific antibody PR001197 could effectively kill LNCaP cells with a cell killing activity EC50 of about 0.14 nM and a maximum killing rate of about 52% (Table 11). In contrast, the corresponding PSMA monoclonal antibody PR000327, CD3 monoclonal antibody PR001310, and control bispecific antibody PR001310 did not cause significant cell killing.

The results of HEK293/hROR1 cell killing test were shown in Fig. 22. ROR1xCD3 bispecific antibody PR001200 could effectively kill HEK293/hROR1 cells with a cell killing activity EC50 of about 1.16 nM and a maximum killing rate of about 55% (Table 11). In contrast, the corresponding ROR1 monoclonal antibody PR000374, CD3 monoclonal antibody PR000512, and control bispecific antibody PR001310 did not cause significant cell killing.

The results of NCI-H929 cell killing test were shown in Fig. 23. BCMAxCD3 bispecific antibody PR001198 could effectively kill NCI-H929 cells with a cell killing activity EC50 of about 0.02 nM and a maximum killing rate of about 61% (Table 11). In contrast, the control bispecific antibody PR001310 did not cause significant cell killing because it did not bind to NCI-H929 cells.

**Table 11: Activity of "κ/λ" Fab-Fab bispecific antibodies in mediating the killing of target cells by effector cells**

| Antibody ID | Target cell (T) | Effector cells (E) | Effector-to-target cell ratio (E:T) | Cell killing EC50 (nM) | Maximum killing rate (%) |
|---|---|---|---|---|---|
| PR000317 | SK-BR-3 | Human T cells | 10 : 1 | 0.0018 | 33.9 |
| PR001197 | LNCaP | Human T cells | 4 : 1 | 0.1432 | 52.4 |
| PR001200 | HEK293/hROR1 | Human T cells | 4 : 1 | 1.158 | 54.8 |
| PR001198 | NCI-H929 | Human PBMC | 10 : 1 | 0.0178 | 61.3 |

This experiment demonstrated that the "κ/λ" Fab-Fab antigen-binding protein containing a CD3-binding domain can effectively mediate the activation of effector cells and the killing of target cells. Moreover, this structure exhibits the flexibility of "plug-and-play". T cell adaptor bispecific antibodies targeting different tumors can be prepared by replacing the antigen-binding domain Fab specific to different tumor-associated antigens.

**Example 7 Pharmacokinetic study of the "κ/λ" Fab-Fab structure and its derived structures**

This Example studied the *in vivo* pharmacokinetics of B7H4xCD3 "κ/λ" Fab-Fab antibodies PR000317 and PR001325 in mice. PR001325 was made by the addition of a serum albumin (ALB) binding domain based on PR000317. ALB was a VH derived from a single domain antibody (PR000929). Also, humanized variant sequences were involved in the CD3-binding Fab of PR001325. The structure of PR000317 is shown in Fig. 10(A), and the structure of PR001325 is shown in Fig. 10(C). Their sequences are summarized in Table 9. As shown in Fig. 19, PR001325 could bind to human serum albumin (Sino Biological, #10968-HNAY) with an activity comparable to that of PR000929.

The procedure was as follows: Six female C57BL/6 mice of appropriate body weight were selected and the tested drug was administered by intravenous injection at a dose of 5 mg/kg. Whole blood was collected before administration and at 5 minutes, 0.5 hours, 2 hours, 4 hours, 8 hours, 24 hours (1 day), on Days 2, 4, 7, 10, and 14 after administration. The blood samples were allowed to stand for 30 minutes to coagulate, then centrifuged at 2,000 rpm at 4 °C for 5 minutes, and isolated serum samples were frozen at -80°C until analysis. Drug concentrations in serum were quantified by ELISA, and the procedure was as follows: B7H4 recombinant protein (Human B7-H4 Protein, His Tag, ACRO Biosystems, #B74-H5222) was coated on 96-well plates at 1μg/ml, 50 μl/well to capture test molecules containing the Fab-terminus of anti-B7H4 in serum, followed by the addition of HRP-labeled goat anti-human IgG (Fab-specific) secondary antibody (Sigma-Aldrich, #A0293) for detection. Phoenix WinNonlin software version 8.2 was used to analyze pharmacokinetic parameters using a non-compartmental model (NCA).

Table 12: *In vivo* **pharmacokinetic parameters** of PR000317 and PR001325 in mice

| PK parameters | Unit | PR000317 | PR001325 |
|---|---|---|---|
| CL | mL/day/kg | 380 | 53.0 |
| $V_{ss}$ | mL/kg | 40.8 | 66.9 |
| $T_{1/2}$ | Hours | 2.3 | 23.1 |
| $AUC_{last}$ | day*ug/mL | 11.3 | 81.3 |
| $AUC_{INF}$ | day*ug/mL | 11.3 | 81.3 |

The results were shown in Table 12 and Fig. 20. The serum half-life of PR000317 in mice was short (about 2.3 h), due to the lack of Fc, while the serum half-life of PR001325 was significantly prolonged to 23 hours, due to the addition of an ALB binding domain based on PR000317.

This experiment demonstrated that the serum half-life of antigen-binding proteins with the "κ/λ" Fab-Fab structure could be conveniently adjusted by introducing a "half-life extension module" (such as serum albumin binding domain) to the "κ/λ" Fab-Fab structure, and the "plug-and-play" flexibility of the "κ/λ" Fab-Fab structure was retained.

**Example 8 Anti-tumor efficacy of B7H4xCD3 "κ/λ" Fab-Fab antigen-binding protein**

In this Example, the anti-tumor effect of the B7H4xCD3 "κ/λ" Fab-Fab antibody PR000317 in the MDA-MB-468 tumor model with NCG mice reconstituted with the human PBMC immune system was investigated.

The specific procedure was as follows: on the day of cell inoculation, each NCG mouse was subcutaneously inoculated with $1 \times 10^7$ MDA-MB-468 tumor cells (ATCC, #HTB-132). The cells were resuspended in a mixture of PBS and Matrigel (1:1), and subcutaneously inoculated at 0.1 mL/mouse. When the average tumor volume of mice reached 80 - 100 $mm^3$, they were divided into groups, with 5 mice in each group. Then, each mouse was inoculated intravenously with $2 \times 10^6$ human PBMCs (0.1 mL/mouse). Two hours after PBMC inoculation, PBS was intravenously injected in the vehicle control group, and PR000317 at a dose of 2 mg/kg was intravenously administered in the treatment group. The dosing cycle was twice a week for a total of 6 doses of intravenous administration. After the start of administration, the body weight and tumor volume were measured twice a week, and tumor volume was calculated as: tumor volume $(mm^3)$ = 0.5 x tumor long diameter x (tumor short diameter)$^2$. The experimental observation was completed on Day 22 after administration, and then all mice were euthanized.

As shown in Fig. 18, on Day 22 post-administration, the vehicle control mice had an average tumor volume of 369 $mm^3$, while the average tumor volume of the tested drug PR000317 (2 mg/kg) treatment group was 33 $mm^3$. Therefore, PR000317 exhibited a significant anti-tumor effect.

**Claims**

1. An antigen-binding protein comprising an antigen-binding region A and an antigen-binding region B binding to different antigens or different epitopes of a same antigen, wherein the antigen-binding protein comprises three polypeptide chains: a first polypeptide chain, a second polypeptide chain, and a third polypeptide chain, and wherein:

   the first polypeptide chain comprises VH_A-CH1 from the amino terminus (N-terminus) to the carboxyl terminus (C-terminus);
   the second polypeptide chain comprises VL_A-CL-L-VH_B-CH1 or VH_B-CH1-L-VL_A-CL from the N-terminus to the C-terminus, and
   the third polypeptide chain comprises VL_B-CL from the N-terminus to the C-terminus,
   and wherein: VH_A and VL_A are respectively the heavy chain variable region and the light chain variable region derived from antibody A, which constitute the antigen-binding region A; VH_B and VL_B are respectively the heavy chain variable region and the light chain variable region derived from antibody B, which constitute the antigen-binding region B; CL is a light chain constant region domain, wherein the CLs in the second polypeptide chain and the third polypeptide chain comprise constant regions derived from different light chain types; CH1 is the first domain of a heavy chain constant region, wherein the CH1s in the first polypeptide chain and the second polypeptide chain may be the same or different; and L is an optional linker peptide.

2. The antigen-binding protein according to claim 1, wherein the CLs in the second polypeptide chain and the third polypeptide chain each independently comprise a constant region derived from a κ light chain or a λ light chain (Cκ or Cλ).

3. The antigen-binding protein according to claim 2, wherein the CL in the second polypeptide chain comprises the constant region Cκ derived from a κ light chain, and the CL in the third polypeptide chain comprises the constant region Cλ derived from a λ light chain.

4. The antigen-binding protein according to claim 2, wherein the CL in the second polypeptide chain comprises the constant region Cλ derived from a λ light chain, and the CL in the third polypeptide chain comprises the constant region Cκ derived from a κ light chain.

5. The antigen-binding protein according to any one of claims 1-4, wherein the VL_A and VL_B each independently comprise a variable region derived from a κ light chain or a λ light chain (Vκ or Vλ).

6. The antigen-binding protein according to claim 5, wherein the VL_A comprises a variable region which is derived from the same type of light chain with the light chain constant region comprised in the CL of the second polypeptide chain, and/or the VL_B comprises a variable region which is derived from the same type of light chain with the light chain constant region comprised in the CL of the third polypeptide chain.

7. The antigen-binding protein according to any one of claims 1-6, wherein the CH1s each independently comprise a CH1 region derived from an IgG isotype, such as selected from the CH1 region of IgG1, IgG2, and IgG4, for example selected from the CH1 region of human IgG1, IgG2, and IgG4.

8. The antigen-binding protein according to any one of claims 1-7, wherein:

(a) the first polypeptide chain comprises VH_A-CH1 from the N-terminus to the C-terminus, the second polypeptide chain comprises VL_A-Cκ-L-VH_B-CH1 from the N-terminus to the C-terminus, and the third polypeptide chain comprises VL_B-Cλ from the N-terminus to the C-terminus;

(b) the first polypeptide chain comprises VH_A-CH1 from the N-terminus to the C-terminus, the second polypeptide chain comprises VL_A-Cλ-L-VH_B-CH1 from the N-terminus to the C-terminus, and the third polypeptide chain comprises VL_B-Cκ from the N-terminus to the C-terminus;

(c) the first polypeptide chain comprises VH_A-CH1 from the N-terminus to the C-terminus, the second polypeptide chain comprises VH_B-CH1-L-VL_A-Cκ from the N-terminus to the C-terminus, and the third polypeptide chain comprises VL _B-Cλ from the N-terminus to the C-terminus;

(d) the first polypeptide chain comprises VH_A-CH1 from the N-terminus to the C-terminus, the second polypeptide chain comprises VH_B-CH1-L-VL_A-Cλ from the N-terminus to the C-terminus, and the third polypeptide chain comprises VL_B-Cκ from the N-terminus to the C-terminus;

wherein, the VL_A and VL_B each independently comprise a variable region derived from a κ light chain or a λ light chain (Vκ or Vλ), and L is an optional linker peptide.

9. The antigen-binding protein according to any one of claims 1-8, wherein:

(a) the first polypeptide chain comprises VH_A-CH1 from the N-terminus to the C-terminus, the second polypeptide chain comprises Vκ_A-Cκ-L-VH_B-CH1 from the N-terminus to the C-terminus, and the third polypeptide chain comprises Vλ_B-Cλ from the N-terminus to the C-terminus;

(b) the first polypeptide chain comprises VH_A-CH1 from the N-terminus to the C-terminus, the second polypeptide chain comprises Vκ_A-Cλ-L-VH_B-CH1 from the N-terminus to the C-terminus, and the third polypeptide chain comprises Vλ_B-Cκ from the N-terminus to the C-terminus;

(c) the first polypeptide chain comprises VH_A-CH1 from the N-terminus to the C-terminus, the second polypeptide chain comprises VH_B-CH1-L-Vκ_A-Cκ from the N-terminus to the C-terminus, and the third polypeptide chain comprises Vλ_B-Cλ from the N-terminus to the C-terminus;

(d) the first polypeptide chain comprises VH_A-CH1 from the N-terminus to the C-terminus, the second polypeptide chain comprises VH_B-CH1-L-Vκ_A-Cλ from the N-terminus to the C-terminus, and the third polypeptide chain comprises Vλ_B-Cκ from the N-terminus to the C-terminus;

(e) the first polypeptide chain comprises VH_A-CH1 from the N-terminus to the C-terminus, the second polypeptide chain comprises Vκ_A-Cκ-L-VH_B-CH1 from the N-terminus to the C-terminus, and the third polypeptide chain comprises Vκ_B-Cλ from the N-terminus to the C-terminus;

(f) the first polypeptide chain comprises VH_A-CH1 from the N-terminus to the C-terminus, the second polypeptide chain comprises Vκ_A-Cλ-L-VH_B-CH1 from the N-terminus to the C-terminus, and the third polypeptide chain comprises Vκ_B-Cκ from the N-terminus to the C-terminus;

(g) the first polypeptide chain comprises VH_A-CH1 from the N-terminus to the C-terminus, the second polypeptide chain comprises VH_B-CH1-L-Vκ_A-Cκ from the N-terminus to the C-terminus, and the third polypeptide chain comprises Vκ_B-Cλ from the N-terminus to the C-terminus;

(h) the first polypeptide chain comprises VH_A-CH1 from the N-terminus to the C-terminus, the second polypeptide chain comprises VH_B-CH1-L-Vκ_A-Cλ from the N-terminus to the C-terminus, and the third polypeptide chain comprises Vκ_B-Cκ from the N-terminus to the C-terminus;

(i) the first polypeptide chain comprises VH_A-CH1 from the N-terminus to the C-terminus, the second polypeptide chain comprises Vλ_A-Cκ-L-VH_B-CH1 from the N-terminus to the C-terminus, and the third polypeptide chain comprises Vλ_B-Cλ from the N-terminus to the C-terminus;

(j) the first polypeptide chain comprises VH_A-CH1 from the N-terminus to the C-terminus, the second polypeptide chain comprises Vλ_A-Cλ-L-VH_B-CH1 from the N-terminus to the C-terminus, and the third polypeptide chain comprises Vλ_B-Cκ from the N-terminus to the C-terminus;

(k) the first polypeptide chain comprises VH_A-CH1 from the N-terminus to the C-terminus, the second polypeptide chain comprises VH_B-CH1-L-Vλ_A-Cκ from the N-terminus to the C-terminus, and the third polypeptide chain comprises Vλ_B-Cλ from the N-terminus to the C-terminus;

(l) the first polypeptide chain comprises VH_A-CH1 from the N-terminus to the C-terminus, the second polypeptide chain comprises VH_B-CH1-L-Vλ_A-Cλ from the N-terminus to the C-terminus, and the third polypeptide chain comprises Vλ_B-Cκ from the N-terminus to the C-terminus.

10. The antigen-binding protein according to any one of claims 1-9, wherein the linker peptide comprises (G4S)n, where the n is an integer selected from 1-5.

11. The antigen-binding protein according to any one of claims 1-10, wherein the antigen-binding region A and the antigen-binding region B each independently bind to an antigen selected from the group consisting of: a tumor-associated antigen, an immune cell antigen such as a T cell antigen and/or an NK cell antigen, and an immune checkpoint molecule.

12. The antigen-binding protein according to claim 11, wherein the antigen-binding region A and the antigen-binding region B bind to two different tumor-associated antigens, or different epitopes on a same tumor-associated antigen.

13. The antigen-binding protein according to claim 11, wherein one of the antigen-binding region A and the antigen-binding region B binds to a tumor-associated antigen, and the other binds to an immune cell antigen, such as a T cell antigen and/or an NK cell antigen.

14. The antigen-binding protein according to any one of claims 11-13, wherein the tumor-associated antigen is selected from CD19, BCMA, TSHR, CD171, CS-1, CLL-1, GD3, Tn Ag, FLT3, CD38, CD123, CD44v6, B7H3, B7H4, KIT, IL-13Ra2, IL-11Ra, PSCA, PSMA, PRSS21, VEGFR2, LewisY, CD24, PDGFR-beta, SSEA-4, MUC1, EGFR, NCAM, CAIX, LMP2, EphA2, fucosyl GM1, sLe, GM3, TGS5, HMWMAA, GD2, FOLR1, FOLR2, TEM1/CD248, TEM7R, CLDN6, CLDN18.2, GPRC5D, CXORF61, CD97, CD179a, ALK, polysialic acid, PLAC1, GloboH, NY-BR-1, UPK2, HAVCR1, ADRB3, PANX3, GPR20, LY6K, OR51E2, TAARP, WT1, ETV6-AML, SPA17, XAGE1, Tie 2, MAD-CT-1, MAD-CT-2, FOSL1, hTERT, ML-IAP, ERG, NA17, PAX3, AR, cyclin B1, MYCN, RhoC, CYP1B1, BORIS, SART3, PAX5, OY-TES1, LCK, AKAP-4, SSX2, CD79a, CD79b, CD72, LAIR1, FCAR, LILRA2, CD300LF, CLEC12A, BST2, EMR2, LY75, GPC3, FCRL5, IGLL1, CD20, CD30, HER2, ROR1, FLT3, TAAG72, CD22, CD33, GD2, gp100Tn, FAP, tyrosinase, EPCAM, CEA, IGF-1R, EphB2, mesothelin, cadherin 17, CD32b, EGFRvIII, GPNMB, GPR64, HER3, LRP6, LYPD8, NKG2D, SLC34A2, SLC39A6, SLITRK6, GUCY2C, or TACSTD2; preferably B7H3, B7H4, PSMA, CLDN18.2, GPC3, HER2, ROR1, CD22, EPCAM, mesothelin, EGFRvIII or TACSTD2; preferably, the tumor-associated antigen is selected from B7H4, PSMA, ROR1 and BCMA.

15. The antigen-binding protein according to any one of claims 11-13, wherein the immune cell antigen is selected from T cell receptor (TCR), CD3, CD28, 4-1BB, OX40, GITR, CD27, ICOS, CD2, CD40, CD226, CD16, NKp30, NKp44, NKp46, NKG2D and 2B4, preferably, the immune cell antigen is CD3, CD16, or CD137.

16. The antigen-binding protein according to any one of claims 1-15, further comprising an antigen-binding region C binding to a different antigen or a different epitope on the same antigen from that of the antigen-binding region A and the antigen-binding region B.

17. The antigen-binding protein according to claim 16, wherein the antigen-binding region C binds to serum albumin.

18. The antigen-binding protein according to any one of claims 1-10, wherein

the VL_A comprises LCDR1, LCDR2, and LCDR3 having the amino acid sequences as shown in SEQ ID NO: 81, 99, and 117, respectively; the VH_A comprises HCDR1, HCDR2, and HCDR3 having the amino acid sequences as shown in SEQ ID NO: 12, 35, and 58, respectively; and the VL_B comprises LCDR1, LCDR2, and LCDR3 having the amino acid sequences as shown in SEQ ID NO: 82, 100, and 118, respectively; the VH_B comprises HCDR1, HCDR2, and HCDR3 having the amino acid sequences as shown in SEQ ID NO: 13, 36, and 59, respectively;
the VL_A comprises LCDR1, LCDR2, and LCDR3 having the amino acid sequences as shown in SEQ ID NO: 85, 103, and 121, respectively; the VH_A comprises HCDR1, HCDR2, and HCDR3 having the amino acid sequences as shown in SEQ ID NO: 16, 39, and 62, respectively; and the VL_B comprises LCDR1, LCDR2, and LCDR3 having the amino acid sequences as shown in SEQ ID NO: 82, 100, and 118, respectively; the VH_B comprises HCDR1, HCDR2, and HCDR3 having the amino acid sequences as shown in SEQ ID NO: 13, 36, and 59, respectively;
the VL_A comprises LCDR1, LCDR2, and LCDR3 having the amino acid sequences as shown in SEQ ID NO: 83, 101, and 119, respectively; the VH_A comprises HCDR1, HCDR2, and HCDR3 having the amino acid sequences as shown in SEQ ID NO: 14, 37, and 60, respectively; and the VL_B comprises LCDR1, LCDR2, and LCDR3 having the amino acid sequences as shown in SEQ ID NO: 82, 100, and 118, respectively; the VH_B comprises HCDR1, HCDR2, and HCDR3 having the amino acid sequences as shown in SEQ ID NO: 13, 36, and 59, respectively;

the VL_A comprises LCDR1, LCDR2, and LCDR3 having the amino acid sequences as shown in SEQ ID NO: 86, 104, and 122, respectively; the VH_A comprises HCDR1, HCDR2, and HCDR3 having the amino acid sequences as shown in SEQ ID NO: 17, 40, and 63, respectively; and the VL_B comprises LCDR1, LCDR2, and LCDR3 having the amino acid sequences as shown in SEQ ID NO: 82, 100, and 118, respectively; the VH_B comprises HCDR1, HCDR2, and HCDR3 having the amino acid sequences as shown in SEQ ID NO: 13, 36, and 59, respectively;

the VL_A comprises LCDR1, LCDR2, and LCDR3 having the amino acid sequences as shown in SEQ ID NO: 84, 102, and 120, respectively; the VH_A comprises HCDR1, HCDR2, and HCDR3 having the amino acid sequences as shown in SEQ ID NO: 15, 38, and 61, respectively; and the VL_B comprises LCDR1, LCDR2, and LCDR3 having the amino acid sequences as shown in SEQ ID NO: 82, 100, and 118, respectively; the VH_B comprises HCDR1, HCDR2, and HCDR3 having the amino acid sequences as shown in SEQ ID NO: 13, 36, and 59, respectively;

the VL_A comprises LCDR1, LCDR2, and LCDR3 having the amino acid sequences as shown in SEQ ID NO: 81, 99, and 117, respectively; the VH_A comprises HCDR1, HCDR2, and HCDR3 having the amino acid sequences as shown in SEQ ID NO: 12, 35, and 58, respectively; and the VL_B comprises LCDR1, LCDR2, and LCDR3 having the amino acid sequences as shown in SEQ ID NO: 89, 107, and 125, respectively; the VH_B comprises HCDR1, HCDR2, and HCDR3 having the amino acid sequences as shown in SEQ ID NO: 12, 44, and 67, respectively;

the VL_A comprises LCDR1, LCDR2, and LCDR3 having the amino acid sequences as shown in SEQ ID NO: 81, 99, and 117, respectively; the VH_A comprises HCDR1, HCDR2, and HCDR3 having the amino acid sequences as shown in SEQ ID NO: 12, 35, and 58, respectively; and the VL_B comprises LCDR1, LCDR2, and LCDR3 having the amino acid sequences as shown in SEQ ID NO: 89, 107, and 125, respectively; the VH_B comprises HCDR1, HCDR2, and HCDR3 having the amino acid sequences as shown in SEQ ID NO: 12, 45, and 67, respectively;

the VL_A comprises LCDR1, LCDR2, and LCDR3 having the amino acid sequences as shown in SEQ ID NO: 83, 101, and 119, respectively; the VH_A comprises HCDR1, HCDR2, and HCDR3 having the amino acid sequences as shown in SEQ ID NO: 14, 37, and 60, respectively; and the VL_B comprises LCDR1, LCDR2, and LCDR3 having the amino acid sequences as shown in SEQ ID NO: 89, 107, and 125, respectively; the VH_B comprises HCDR1, HCDR2, and HCDR3 having the amino acid sequences as shown in SEQ ID NO: 12, 44, and 67, respectively;

the VL_A comprises LCDR1, LCDR2, and LCDR3 having the amino acid sequences as shown in SEQ ID NO: 83, 101, and 119, respectively; the VH_A comprises HCDR1, HCDR2, and HCDR3 having the amino acid sequences as shown in SEQ ID NO: 14, 37, and 60, respectively; and the VL_B comprises LCDR1, LCDR2, and LCDR3 having the amino acid sequences as shown in SEQ ID NO: 89, 107, and 125, respectively; the VH_B comprises HCDR1, HCDR2, and HCDR3 having the amino acid sequences as shown in SEQ ID NO: 12, 45, and 67, respectively;

the VL_A comprises LCDR1, LCDR2, and LCDR3 having the amino acid sequences as shown in SEQ ID NO: 84, 102, and 120, respectively; the VH_A comprises HCDR1, HCDR2, and HCDR3 having the amino acid sequences as shown in SEQ ID NO: 15, 38, and 61, respectively; and the VL_B comprises LCDR1, LCDR2, and LCDR3 having the amino acid sequences as shown in SEQ ID NO: 88, 106, and 124, respectively; the VH_B comprises HCDR1, HCDR2, and HCDR3 having the amino acid sequences as shown in SEQ ID NO: 19, 42, and 65, respectively;

the VL_A comprises LCDR1, LCDR2, and LCDR3 having the amino acid sequences as shown in SEQ ID NO: 84, 102, and 120, respectively; the VH_A comprises HCDR1, HCDR2, and HCDR3 having the amino acid sequences as shown in SEQ ID NO: 15, 38, and 61, respectively; and the VL_B comprises LCDR1, LCDR2, and LCDR3 having the amino acid sequences as shown in SEQ ID NO: 87, 105, and 123, respectively; the VH_B comprises HCDR1, HCDR2, and HCDR3 having the amino acid sequences as shown in SEQ ID NO: 18, 41, and 64, respectively;

the VL_A comprises LCDR1, LCDR2, and LCDR3 having the amino acid sequences as shown in SEQ ID NO: 81, 99, and 117, respectively; the VH_A comprises HCDR1, HCDR2, and HCDR3 having the amino acid sequences as shown in SEQ ID NO: 12, 35, and 58, respectively; and the VL_B comprises LCDR1, LCDR2, and LCDR3 having the amino acid sequences as shown in SEQ ID NO: 88, 106, and 124, respectively; the VH_B comprises HCDR1, HCDR2, and HCDR3 having the amino acid sequences as shown in SEQ ID NO: 19, 42, and 65, respectively; or

the VL_A comprises LCDR1, LCDR2, and LCDR3 having the amino acid sequences as shown in SEQ ID NO: 81, 99, and 117, respectively; the VH_A comprises HCDR1, HCDR2, and HCDR3 having the amino acid sequences as shown in SEQ ID NO: 12, 35, and 58, respectively; and the VL_B comprises LCDR1, LCDR2, and LCDR3 having the amino acid sequences as shown in SEQ ID NO: 87, 105, and 123, respectively; the VH_B comprises HCDR1, HCDR2, and HCDR3 having the amino acid sequences as shown in SEQ ID NO: 18, 41, and 64, respectively,

wherein the CDR sequences are defined according to Chothia numbering system.

**19.** The antigen-binding protein according to claim 18, wherein

the VL_A comprises the amino acid sequence as shown in SEQ ID NO: 144; the VH_A comprises the amino acid sequence as shown in SEQ ID NO: 131; the VL_B comprises the amino acid sequence as shown in SEQ ID NO: 145; and the VH_B comprises the amino acid sequence as shown in SEQ ID NO: 132;

the VL_A comprises the amino acid sequence as shown in SEQ ID NO: 144; the VH_A comprises the amino acid sequence as shown in SEQ ID NO: 131; the VL_B comprises the amino acid sequence as shown in SEQ ID NO: 151; and the VH_B comprises the amino acid sequence as shown in SEQ ID NO: 138;

the VL_A comprises the amino acid sequence as shown in SEQ ID NO: 148; the VH_A comprises the amino acid sequence as shown in SEQ ID NO: 135; the VL_B comprises the amino acid sequence as shown in SEQ ID NO: 151; and the VH_B comprises the amino acid sequence as shown in SEQ ID NO: 138;

the VL_A comprises the amino acid sequence as shown in SEQ ID NO: 146; the VH_A comprises the amino acid sequence as shown in SEQ ID NO: 133; the VL_B comprises the amino acid sequence as shown in SEQ ID NO: 151; and the VH_B comprises the amino acid sequence as shown in SEQ ID NO: 138;

the VL_A comprises the amino acid sequence as shown in SEQ ID NO: 149; the VH_A comprises the amino acid sequence as shown in SEQ ID NO: 136; the VL_B comprises the amino acid sequence as shown in SEQ ID NO: 151; and the VH_B comprises the amino acid sequence as shown in SEQ ID NO: 138;

the VL_A comprises the amino acid sequence as shown in SEQ ID NO: 147; the VH_A comprises the amino acid sequence as shown in SEQ ID NO: 134; the VL_B comprises the amino acid sequence as shown in SEQ ID NO: 151; and the VH_B comprises the amino acid sequence as shown in SEQ ID NO: 138;

the VL_A comprises the amino acid sequence as shown in SEQ ID NO: 144; the VH_A comprises the amino acid sequence as shown in SEQ ID NO: 131; the VL_B comprises the amino acid sequence as shown in SEQ ID NO: 153; and the VH_B comprises the amino acid sequence as shown in SEQ ID NO: 141;

the VL_A comprises the amino acid sequence as shown in SEQ ID NO: 144; the VH_A comprises the amino acid sequence as shown in SEQ ID NO: 131; the VL_B comprises the amino acid sequence as shown in SEQ ID NO: 153; and the VH_B comprises the amino acid sequence as shown in SEQ ID NO: 142;

the VL_A comprises the amino acid sequence as shown in SEQ ID NO: 146; the VH_A comprises the amino acid sequence as shown in SEQ ID NO: 133; the VL_B comprises the amino acid sequence as shown in SEQ ID NO: 153; and the VH_B comprises the amino acid sequence as shown in SEQ ID NO: 141;

the VL_A comprises the amino acid sequence as shown in SEQ ID NO: 146; the VH_A comprises the amino acid sequence as shown in SEQ ID NO: 133; the VL_B comprises the amino acid sequence as shown in SEQ ID NO: 153; and the VH_B comprises the amino acid sequence as shown in SEQ ID NO: 142;

the VL_A comprises the amino acid sequence as shown in SEQ ID NO: 147; the VH_A comprises the amino acid sequence as shown in SEQ ID NO: 134; the VL_B comprises the amino acid sequence as shown in SEQ ID NO: 152; and the VH_B comprises the amino acid sequence as shown in SEQ ID NO: 139;

the VL_A comprises the amino acid sequence as shown in SEQ ID NO: 147; the VH_A comprises the amino acid sequence as shown in SEQ ID NO: 134; the VL_B comprises the amino acid sequence as shown in SEQ ID NO: 150; and the VH_B comprises the amino acid sequence as shown in SEQ ID NO: 137;

the VL_A comprises the amino acid sequence as shown in SEQ ID NO: 144; the VH_A comprises the amino acid sequence as shown in SEQ ID NO: 131; the VL_B comprises the amino acid sequence as shown in SEQ ID NO: 152; and the VH_B comprises the amino acid sequence as shown in SEQ ID NO: 139; or

the VL_A comprises the amino acid sequence as shown in SEQ ID NO: 144; the VH_A comprises the amino acid sequence as shown in SEQ ID NO: 131; the VL_B comprises the amino acid sequence as shown in SEQ ID NO: 150; and the VH_B comprises the amino acid sequence as shown in SEQ ID NO: 137.

**20.** The antigen-binding protein according to claim 1, wherein

the first polypeptide chain comprises the amino acid sequence as shown in SEQ ID NO: 177; the second polypeptide chain comprises the amino acid sequence as shown in SEQ ID NO: 178; and the third polypeptide chain comprises the amino acid sequence as shown in SEQ ID NO: 168;

the first polypeptide chain comprises the amino acid sequence as shown in SEQ ID NO: 177; the second polypeptide chain comprises the amino acid sequence as shown in SEQ ID NO: 187; and the third polypeptide chain comprises the amino acid sequence as shown in SEQ ID NO: 174;

the first polypeptide chain comprises the amino acid sequence as shown in SEQ ID NO: 179; the second polypeptide chain comprises the amino acid sequence as shown in SEQ ID NO: 180; and the third polypeptide chain comprises the amino acid sequence as shown in SEQ ID NO: 174;

the first polypeptide chain comprises the amino acid sequence as shown in SEQ ID NO: 181; the second polypeptide chain comprises the amino acid sequence as shown in SEQ ID NO: 182; and the third polypeptide chain comprises the amino acid sequence as shown in SEQ ID NO: 174;

the first polypeptide chain comprises the amino acid sequence as shown in SEQ ID NO: 183; the second polypeptide chain comprises the amino acid sequence as shown in SEQ ID NO: 184; and the third polypeptide chain comprises the amino acid sequence as shown in SEQ ID NO: 174;

the first polypeptide chain comprises the amino acid sequence as shown in SEQ ID NO: 185; the second polypeptide chain comprises the amino acid sequence as shown in SEQ ID NO: 186; and the third polypeptide chain comprises the amino acid sequence as shown in SEQ ID NO: 174;

the first polypeptide chain comprises the amino acid sequence as shown in SEQ ID NO: 177; the second polypeptide chain comprises the amino acid sequence as shown in SEQ ID NO: 188; and the third polypeptide chain comprises the amino acid sequence as shown in SEQ ID NO: 176;

the first polypeptide chain comprises the amino acid sequence as shown in SEQ ID NO: 177; the second polypeptide chain comprises the amino acid sequence as shown in SEQ ID NO: 189; and the third polypeptide chain comprises the amino acid sequence as shown in SEQ ID NO: 176;

the first polypeptide chain comprises the amino acid sequence as shown in SEQ ID NO: 181; the second polypeptide chain comprises the amino acid sequence as shown in SEQ ID NO: 190; and the third polypeptide chain comprises the amino acid sequence as shown in SEQ ID NO: 176;

the first polypeptide chain comprises the amino acid sequence as shown in SEQ ID NO: 181; the second polypeptide chain comprises the amino acid sequence as shown in SEQ ID NO: 191; and the third polypeptide chain comprises the amino acid sequence as shown in SEQ ID NO: 176;

the first polypeptide chain comprises the amino acid sequence as shown in SEQ ID NO: 185; the second polypeptide chain comprises the amino acid sequence as shown in SEQ ID NO: 193; and the third polypeptide chain comprises the amino acid sequence as shown in SEQ ID NO: 192;

the first polypeptide chain comprises the amino acid sequence as shown in SEQ ID NO: 185; the second polypeptide chain comprises the amino acid sequence as shown in SEQ ID NO: 194; and the third polypeptide chain comprises the amino acid sequence as shown in SEQ ID NO: 173;

the first polypeptide chain comprises the amino acid sequence as shown in SEQ ID NO: 177; the second polypeptide chain comprises the amino acid sequence as shown in SEQ ID NO: 195; and the third polypeptide chain comprises the amino acid sequence as shown in SEQ ID NO: 192; or

the first polypeptide chain comprises the amino acid sequence as shown in SEQ ID NO: 177; the second polypeptide chain comprises the amino acid sequence as shown in SEQ ID NO: 196; and the third polypeptide chain comprises the amino acid sequence as shown in SEQ ID NO: 173.

21. The antigen-binding protein according to any one of claims 1-20, wherein the antigen-binding protein does not comprise an antibody Fc region.

22. An isolated nucleic acid encoding the antigen-binding protein according to any one of claims 1-21.

23. A recombinant expression vector comprising the isolated nucleic acid according to claim 22;
preferably, the expression vector comprises a eukaryotic cell expression vector and/or a prokaryotic cell expression vector.

24. A host cell comprising the isolated nucleic acid according to claim 22 or the recombinant expression vector according to claim 23; preferably, the host cell is a prokaryotic cell and/or a eukaryotic cell, preferably, the prokaryotic cell is an *E. coli* cell, such as TG1 or BL21 cell, and preferably, the eukaryotic cell is an HEK293 cell or a CHO cell.

25. A preparation method for an antigen-binding protein, comprising culturing the host cell according to claim 24, and obtaining the antigen-binding protein from the culture.

26. A pharmaceutical composition **characterized in that** the pharmaceutical composition comprises the antigen-binding protein according to any one of claims 1-21, and a pharmaceutically acceptable carrier;

preferably, the pharmaceutical composition further comprises one or more additional anti-tumor agents as active ingredients.

27. A method for preventing and/or treating a disease in a subject in need thereof, comprising a step of administering to the subject an effective amount of the antigen-binding protein according to any one of claims 1-21 or the pharmaceutical composition according to claim 26.

28. The method according to claim 27, wherein the disease is cancer.

29. A preparation method for the antigen-binding protein according to any one of claims 1-21, comprising:

(a) expressing the first polypeptide chain, the second polypeptide chain, and the third polypeptide chain of the antigen-binding protein in a host cell to obtain a protein mixture assembled by the first polypeptide chain, the second polypeptide chain, and the third polypeptide chain;
(b) based on the type of the light chain constant region comprised in the second polypeptide chain of the antigen-binding protein, affinity-capturing the second polypeptide chain with a corresponding affinity chromatography method, so as to obtain the antigen-binding protein composed of the first polypeptide chain and the third polypeptide chain interacting with the second polypeptide chain, and to remove the mismatched products not containing the second polypeptide.

30. The preparation method according to claim 29, wherein the second polypeptide chain contains a Cκ region, and the antigen-binding protein is obtained with a κ-chain specific affinity chromatography method.

31. The preparation method according to claim 29, wherein the second polypeptide chain contains a Cλ region, and the antigen-binding protein is obtained with a λ-chain specific affinity chromatography method.

IgG A          IgG B

+

Fab-A          Fab-B
(N-terminal Fab)    (C-terminal Fab)

Fig. 1

First polypeptide chain

Second polypeptide chain

Fab-A    First linker
(N-terminal Fab)  peptide    (C-terminal Fab)

Second linker
peptide

Fig. 2

**(A)**

Fab-A
(N-terminal Fab)

First polypeptide chain — N — | VL_A | CL | — C

Linker peptide

Second polypeptide chain — N — | VH_A | CH1 | L | VH_B | CH1 | — C

Third polypeptide chain — N — | VL_B | CL | — C

Fab-B
(C-terminal Fab)

**(B)**

(Main by-product)

i.

Mismatched
N-terminal Fab — N — | VL_A | CL | — C

N — | VH_A | CH1 | L | VH_B | CH1 | — C

N — | VL_B | CL | — C

Mismatched
C-terminal Fab

Fig. 3

**(A)**

Fab-A
(N-terminal Fab)

First polypeptide chain   N ⊢ VH_A | CH1 ⊣ C   Linker peptide

Second polypeptide chain   N ⊢ VL_A | CL | L | VH_B | CH1 ⊣ C

Third polypeptide chain   N ⊢ VL_B | CL ⊣ C

Fab-B
(C-terminal Fab)

**(B)**

(Main by-product)

i.

Mismatched Fab

N ⊢ VH_A | CH1 ⊣ C
N ⊢ VL_B | CL ⊣ C

Fig. 4

**(A)**

(Encoding three polypeptide chains)

First polypeptide chain N ─[ VH_A | CH1 ]─ C

Second polypeptide chain N ─[ VL_A | Cκ | L | VH_B | CH1 ]─ C

Third polypeptide chain N ─[ VL_B | Cλ ]─ C

↓ κ-chain specific affinity capture

**(B)**

(Main product is captured)

Fab-A
(N-terminal Fab)

First polypeptide chain N ─[ VH_A | CH1 ]─ C

Linker peptide

Second polypeptide chain N ─[ VL_A | Cκ | L | VH_B | CH1 ]─ C

Third polypeptide chain N ─[ VL_B | Cλ ]─ C

Fab-B
(C-terminal Fab)

**(C)**

(By-product flows through)

i. **Mismatched Fab**

N ─[ VH_A | CH1 ]─ C
N ─[ VL_B | Cλ ]─ C

ii. **Light chain dimer**

N ─[ VL_B | Cλ ]─ C
N ─[ VL_B | Cλ ]─ C

iii. **Fd dimer**

N ─[ VH_A | CH1 ]─ C
N ─[ VH_A | CH1 ]─ C

Fig. 5

**(A)**

Configuration-I: κ-chain specific affinity capture is adopted

**Fab-A**
**(N-terminal Fab)**

First polypeptide chain

Second polypeptide chain
(long chain)

Third polypeptide chain

Linker peptide

**Fab-B**
**(C-terminal Fab)**

**(B)**

Configuration-II: λ-chain specific affinity capture is adopted

**Fab-A**
**(N-terminal Fab)**

First polypeptide chain

Second polypeptide chain
(long chain)

Third polypeptide chain

Linker peptide

**Fab-B**
**(C-terminal Fab)**

Fig. 6

**(C)**

Configuration-III: κ-chain specific affinity capture is adopted

**Fab-A**
**(C-terminal Fab)**

First polypeptide chain     N —[ VH_A | CH1 ]— c

Second polypeptide chain   N —[ VH_B | CH1 ][L] [ VL_A | Cκ ]— c
(long chain)

Third polypeptide chain     N —[ VL_B | Cλ ]— c   Linker peptide

**Fab-B**
**(N-terminal Fab)**

**(D)**

Configuration-IV: λ-chain specific affinity capture is adopted

**Fab-A**
**(C-terminal Fab)**

First polypeptide chain     N —[ VH_A | CH1 ]— c

Second polypeptide chain   N —[ VH_B | CH1 ][L] [ VL_A | Cλ ]— c
(long chain)

Third polypeptide chain     N —[ VL_B | Cκ ]— c   Linker peptide

**Fab-B**
**(N-terminal Fab)**

Fig. 6 (continued)

Fig. 7

Fig. 8

Fig. 9

**(A)**

**(B)**

**(C)**

Fig. 10

**(A)**

**(B)**

Fig. 11

**(A)**

**(B)**

Fig. 12

**(A)**

**(B)**

Fig. 13

## Binding to SK-BR-3

Fig. 14

## Binding to T cells

Fig. 15

**Binding to CHO-K1/hB7H4 and
pan T Cells Simultaneously**

Fig. 16

Fig. 17

**(A)**

**(B)**

Fig. 18

**Binding to human serum
albumin (pH 6.0)**

Fig. 19

Fig. 20

**LNCaP + Pan T, [LDH]**

Fig. 21

**HEK293/hROR1 + Pan T, [LDH]**

Fig. 22

Fig. 23

## INTERNATIONAL SEARCH REPORT

| International application No. |
|---|
| **PCT/CN2023/139544** |

### A. CLASSIFICATION OF SUBJECT MATTER

C07K16/00(2006.01)i

According to International Patent Classification (IPC) or to both national classification and IPC

### B. FIELDS SEARCHED

Minimum documentation searched (classification system followed by classification symbols)

IPC: C07K

Documentation searched other than minimum documentation to the extent that such documents are included in the fields searched

Electronic data base consulted during the international search (name of data base and, where practicable, search terms used)

NCBI, CNTXT, VEN, ENTXT, CNKI, 万方数据库, WANFANG DATABASE, PUBMED, ISI: 序列检索, sequence search, 发明人, inventor, Fab, FAB-FAB, 串联, tandem, 多特异性抗体, 双特异性抗体, BsAb, kappa, lambda, 轻链, 非对称, asymmetric, bispecific antibodies, 3 polypeptide chains, purification, kappaselect

### C. DOCUMENTS CONSIDERED TO BE RELEVANT

| Category* | Citation of document, with indication, where appropriate, of the relevant passages | Relevant to claim No. |
|---|---|---|
| Y | CN 106459182 A (EPIMAB BIOTHERAPEUTICS INC.) 22 February 2017 (2017-02-22) abstract, drawings, claims 1-3, 8-21, 23, 28 and 28-57, and description, paragraphs 100 and 151 | 1-31 |
| Y | WO 2022002038 A1 (HARBOUR BIOMED (SHANGHAI) CO., LTD.) 06 January 2022 (2022-01-06) figure 1(8), and claims 1-3 | 1-31 |
| Y | WO 2022002006 A1 (HARBOUR BIOMED (SHANGHAI) CO., LTD.) 06 January 2022 (2022-01-06) figure 1(5), and embodiment 1.2.1 | 1-31 |
| Y | CN 109476732 A (SANOFI SA) 15 March 2019 (2019-03-15) claims 1, 4-15, 56, 83-84 and 86, and description, paragraphs 702 and 1176-1183 | 1-31 |
| A | CN 114728065 A (SHANGHAI EPIMAB BIOTHERAPEUTICS CO., LTD.) 08 July 2022 (2022-07-08) claims 1-21 | 1-31 |

☑ Further documents are listed in the continuation of Box C.  ☑ See patent family annex.

| * | Special categories of cited documents: | |
|---|---|---|
| "A" | document defining the general state of the art which is not considered to be of particular relevance | "T" later document published after the international filing date or priority date and not in conflict with the application but cited to understand the principle or theory underlying the invention |
| "D" | document cited by the applicant in the international application | "X" document of particular relevance; the claimed invention cannot be considered novel or cannot be considered to involve an inventive step when the document is taken alone |
| "E" | earlier application or patent but published on or after the international filing date | |
| "L" | document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" | document referring to an oral disclosure, use, exhibition or other means | "&" document member of the same patent family |
| "P" | document published prior to the international filing date but later than the priority date claimed | |

| Date of the actual completion of the international search | Date of mailing of the international search report |
|---|---|
| **11 March 2024** | **18 March 2024** |

| Name and mailing address of the ISA/CN | Authorized officer |
|---|---|
| **China National Intellectual Property Administration (ISA/CN)** **China No. 6, Xitucheng Road, Jimenqiao, Haidian District, Beijing 100088** | |
| | Telephone No. |

Form PCT/ISA/210 (second sheet) (July 2022)

**EP 4 640 700 A1**

## INTERNATIONAL SEARCH REPORT

<table>
<tr><td colspan="3">International application No.<br><br>**PCT/CN2023/139544**</td></tr>
</table>

**C.    DOCUMENTS CONSIDERED TO BE RELEVANT**

| Category* | Citation of document, with indication, where appropriate, of the relevant passages | Relevant to claim No. |
|---|---|---|
| Y | CN 106804108 A (GENENTECH, INC.) 06 June 2017 (2017-06-06)<br>      SEQ ID NOs: 126-127 | 18-31 |
| Y | CN 107207595 A (GLENMARK PHARMACEUTICALS S.A.) 26 September 2017 (2017-09-26)<br>      SEQ ID NOs: 60-61 | 18-31 |
| Y | FISCHER, N. et al. "Exploiting Light Chains for the Scalable Generation and Platform Purification of Native Human Bispecific IgG"<br>*Nature Communications,* Vol. vol. 6, 12 February 2015 (2015-02-12),<br>      abstract, and figures 1 and 3 | 1-31 |
| Y | WU, X.F. et al. "Fab-based Bispecific Antibody Formats with Robust Biophysical Properties and Biological Activity"<br>*mAbs,* Vol. 7, No. (3), 30 June 2015 (2015-06-30),<br>      abstract, and figure 4 | 21-31 |
| Y | BRINKMANN, U. et al. "The Making of Bispecific Antibodies"<br>*mAbs,* Vol. 9, No. (2), 31 December 2017 (2017-12-31),<br>      abstract, figures 1 and 2, and pages 186-187 | 21-31 |

Form PCT/ISA/210 (second sheet) (July 2022)

**INTERNATIONAL SEARCH REPORT**

| International application No. |
| --- |
| **PCT/CN2023/139544** |

| Box No. I | Nucleotide and/or amino acid sequence(s) (Continuation of item 1.c of the first sheet) |
| --- | --- |

1. With regard to any nucleotide and/or amino acid sequence disclosed in the international application, the international search was carried out on the basis of a sequence listing:

    a. ☑ forming part of the international application as filed.

    b. ☐ furnished subsequent to the international filing date for the purposes of international search (Rule 13*ter*.1(a)),

        ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

**INTERNATIONAL SEARCH REPORT**

International application No.

**PCT/CN2023/139544**

| **Box No. II** | **Observations where certain claims were found unsearchable (Continuation of item 2 of first sheet)** |

This international search report has not been established in respect of certain claims under Article 17(2)(a) for the following reasons:

1. ☑ Claims Nos.: **27-28**
   because they relate to subject matter not required to be searched by this Authority, namely:

   Claims 27-28 relate to a method for preventing and/or treating diseases, that is, relate to the subject matter for which no search is required by the International Searching Authority as defined in PCT Rule 39.1: (4) methods for treatment of the human or animal body by surgery or therapy, as well as diagnostic methods. The international search is conducted on the basis of the use of the antigen-binding protein of any one of claims 1-21 or the pharmaceutical composition of claim 26 in the preparation of a drug for preventing and/or treating diseases.

2. ☐ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

Form PCT/ISA/210 (continuation of first sheet) (July 2022)

# EP 4 640 700 A1

| INTERNATIONAL SEARCH REPORT | International application No. |
|---|---|
| Information on patent family members | PCT/CN2023/139544 |

| Patent document cited in search report | | | Publication date (day/month/year) | Patent family member(s) | | | Publication date (day/month/year) |
|---|---|---|---|---|---|---|---|
| CN | 106459182 | A | 22 February 2017 | JP | 2020075932 | A | 21 May 2020 |
| | | | | US | 2019248924 | A1 | 15 August 2019 |
| | | | | US | 2023132403 | A9 | 27 April 2023 |
| | | | | JP | 2017506215 | A | 02 March 2017 |
| | | | | JP | 6706578 | B2 | 10 June 2020 |
| | | | | NZ | 720353 | A | 20 December 2019 |
| | | | | CA | 2931641 | A1 | 09 July 2015 |
| | | | | CA | 2931641 | C | 10 May 2022 |
| | | | | ES | 2923641 | T3 | 29 September 2022 |
| | | | | US | 2016289343 | A1 | 06 October 2016 |
| | | | | US | 10266608 | B2 | 23 April 2019 |
| | | | | EP | 3089994 | A1 | 09 November 2016 |
| | | | | EP | 3089994 | A4 | 06 September 2017 |
| | | | | EP | 3089994 | B1 | 04 May 2022 |
| | | | | WO | 2015103072 | A1 | 09 July 2015 |
| | | | | AR | 098979 | A1 | 22 June 2016 |
| | | | | KR | 20160103109 | A | 31 August 2016 |
| | | | | KR | 102056963 | B1 | 17 December 2019 |
| | | | | DK | 3089994 | T3 | 04 July 2022 |
| | | | | EP | 4071177 | A1 | 12 October 2022 |
| | | | | BR | 112016015140 | A2 | 23 January 2018 |
| | | | | US | 2020157249 | A1 | 21 May 2020 |
| | | | | MX | 2016008667 | A | 02 February 2017 |
| | | | | CU | 20160101 | A7 | 29 November 2016 |
| | | | | TW | 201613962 | A | 16 April 2016 |
| | | | | TWI | 672316 | B | 21 September 2019 |
| | | | | IL | 246287 | A0 | 31 July 2016 |
| | | | | IL | 246287 | B | 31 January 2021 |
| | | | | RU | 2016129959 | A | 02 February 2018 |
| | | | | RU | 2016129959 | A3 | 30 July 2018 |
| | | | | AU | 2014374055 | A1 | 16 June 2016 |
| | | | | AU | 2014374055 | B2 | 08 November 2018 |
| | | | | RU | 2020142965 | A | 10 February 2021 |
| | | | | RU | 2020142965 | A3 | 02 July 2021 |
| | | | | RU | 2769133 | C2 | 28 March 2022 |
| | | | | US | 2016289341 | A1 | 06 October 2016 |
| | | | | US | 10519251 | B2 | 31 December 2019 |
| WO | 2022002038 | A1 | 06 January 2022 | | None | | |
| WO | 2022002006 | A1 | 06 January 2022 | | None | | |
| CN | 109476732 | A | 15 March 2019 | KR | 20180134378 | A | 18 December 2018 |
| | | | | US | 2021061925 | A1 | 04 March 2021 |
| | | | | US | 11192960 | B2 | 07 December 2021 |
| | | | | PH | 12018502182 | A1 | 04 November 2019 |
| | | | | JP | 2019522459 | A | 15 August 2019 |
| | | | | JP | 7195929 | B2 | 26 December 2022 |
| | | | | SG | 11201808911 | SA | 29 November 2018 |
| | | | | CO | 2018012107 | A2 | 22 November 2018 |
| | | | | MX | 2018012566 | A | 05 August 2019 |
| | | | | MA | 44670 | A | 20 February 2019 |
| | | | | MA | 44670 | B1 | 30 November 2023 |

Form PCT/ISA/210 (patent family annex) (July 2022)

**INTERNATIONAL SEARCH REPORT**
Information on patent family members

| | International application No. |
|---|---|
| | PCT/CN2023/139544 |

| Patent document cited in search report | | | Publication date (day/month/year) | Patent family member(s) | | | Publication date (day/month/year) |
|---|---|---|---|---|---|---|---|
| | | | | UY | 37206 | A | 30 November 2017 |
| | | | | EP | 3443006 | A2 | 20 February 2019 |
| | | | | EP | 3443006 | B1 | 02 August 2023 |
| | | | | CL | 2018002883 | A1 | 18 January 2019 |
| | | | | DOP | 2018000225 | A | 31 January 2019 |
| | | | | US | 2017320967 | A1 | 09 November 2017 |
| | | | | US | 10882922 | B2 | 05 January 2021 |
| | | | | EA | 201892312 | A1 | 30 April 2019 |
| | | | | IL | 262241 | A | 29 November 2018 |
| | | | | IL | 262241 | B1 | 01 January 2024 |
| | | | | CR | 20180539 | A | 15 February 2019 |
| | | | | PE | 20190128 | A1 | 17 January 2019 |
| | | | | AU | 2017248671 | A1 | 29 November 2018 |
| | | | | PT | 3443006 | T | 26 October 2023 |
| | | | | TW | 201803899 | A | 01 February 2018 |
| | | | | TWI | 788286 | B | 01 January 2023 |
| | | | | CA | 3020633 | A1 | 19 October 2017 |
| | | | | BR | 112018070998 | A2 | 26 February 2019 |
| | | | | WO | 2017180913 | A2 | 19 October 2017 |
| | | | | WO | 2017180913 | A9 | 23 November 2017 |
| | | | | WO | 2017180913 | A3 | 08 February 2018 |
| CN | 114728065 | A | 08 July 2022 | KR | 20220104783 | A | 26 July 2022 |
| | | | | WO | 2021104371 | A1 | 03 June 2021 |
| | | | | CA | 3160163 | A1 | 03 June 2021 |
| | | | | MX | 2022006230 | A | 22 June 2022 |
| | | | | EP | 4065164 | A1 | 05 October 2022 |
| | | | | US | 2023002489 | A1 | 05 January 2023 |
| | | | | JP | 2023504016 | A | 01 February 2023 |
| | | | | TW | 202132347 | A | 01 September 2021 |
| | | | | TWI | 774137 | B | 11 August 2022 |
| | | | | AU | 2020390288 | A1 | 26 May 2022 |
| | | | | IL | 293138 | A | 01 July 2022 |
| CN | 106804108 | A | 06 June 2017 | US | 2016159910 | A1 | 09 June 2016 |
| | | | | US | 10059768 | B2 | 28 August 2018 |
| | | | | WO | 2016040724 | A1 | 17 March 2016 |
| | | | | EP | 3191518 | A1 | 19 July 2017 |
| | | | | EP | 3191518 | B1 | 15 January 2020 |
| | | | | TW | 201625689 | A | 16 July 2016 |
| | | | | JP | 2017530700 | A | 19 October 2017 |
| | | | | JP | 6943760 | B2 | 06 October 2021 |
| | | | | AR | 101848 | A1 | 18 January 2017 |
| | | | | US | 2022251213 | A1 | 11 August 2022 |
| | | | | US | 2019062432 | A1 | 28 February 2019 |
| | | | | US | 11286302 | B2 | 29 March 2022 |
| | | | | JP | 2022002515 | A | 11 January 2022 |
| CN | 107207595 | A | 26 September 2017 | EA | 201690803 | A1 | 30 September 2016 |
| | | | | EA | 036577 | B1 | 25 November 2020 |
| | | | | HK | 1244013 | A1 | 27 July 2018 |
| | | | | MY | 176522 | A | 13 August 2020 |
| | | | | KR | 20170092562 | A | 11 August 2017 |

Form PCT/ISA/210 (patent family annex) (July 2022)

**INTERNATIONAL SEARCH REPORT**
Information on patent family members

International application No.

**PCT/CN2023/139544**

| Patent document cited in search report | Publication date (day/month/year) | Patent family member(s) | | | Publication date (day/month/year) |
|---|---|---|---|---|---|
| | | JP | 2016538275 | A | 08 December 2016 |
| | | PE | 20160724 | A1 | 04 August 2016 |
| | | US | 2020102403 | A1 | 02 April 2020 |
| | | US | 2017145115 | A1 | 25 May 2017 |
| | | US | 2022213227 | A1 | 07 July 2022 |
| | | ZA | 201603433 | B | 27 March 2019 |
| | | JP | 2017536412 | A | 07 December 2017 |
| | | JP | 6994942 | B2 | 04 February 2022 |
| | | BR | 112016009919 | A2 | 05 December 2017 |
| | | CA | 2966124 | A1 | 12 May 2016 |
| | | CA | 2966124 | C | 03 January 2023 |
| | | US | 2018112011 | A1 | 26 April 2018 |
| | | IL | 251850 | A0 | 29 June 2017 |
| | | WO | 2015063339 | A1 | 07 May 2015 |
| | | SG | 11201603244 | VA | 30 May 2016 |
| | | AU | 2014343636 | A1 | 02 June 2016 |
| | | EP | 3215540 | A1 | 13 September 2017 |
| | | KR | 20160090308 | A | 29 July 2016 |
| | | US | 2015133640 | A1 | 14 May 2015 |
| | | US | 9493563 | B2 | 15 November 2016 |
| | | PH | 12016500823 | A1 | 13 June 2016 |
| | | IL | 245381 | A0 | 30 June 2016 |
| | | IL | 245381 | B | 30 September 2021 |
| | | EP | 3066133 | A1 | 14 September 2016 |
| | | AP | 201609222 | A0 | 31 May 2016 |
| | | CL | 2016001068 | A1 | 18 November 2016 |
| | | BR | 112017009264 | A2 | 30 January 2018 |
| | | EP | 3176185 | A1 | 07 June 2017 |
| | | US | 2018355064 | A1 | 13 December 2018 |
| | | US | 11851502 | B2 | 26 December 2023 |
| | | MX | 2016005781 | A | 18 July 2016 |
| | | CA | 2929256 | A1 | 07 May 2015 |
| | | CA | 2929256 | C | 26 April 2022 |
| | | SG | 10201909806 | SA | 28 November 2019 |
| | | AU | 2019257534 | A1 | 28 November 2019 |
| | | AU | 2019257534 | B2 | 09 December 2021 |
| | | US | 2021171661 | A1 | 10 June 2021 |
| | | US | 11891454 | B2 | 06 February 2024 |
| | | JP | 2020023516 | A | 13 February 2020 |
| | | JP | 7068251 | B2 | 16 May 2022 |
| | | AU | 2015342169 | A1 | 08 June 2017 |
| | | WO | 2016071004 | A1 | 12 May 2016 |
| | | MA | 40889 | A | 12 September 2017 |
| | | NZ | 720161 | A | 29 July 2022 |

Form PCT/ISA/210 (patent family annex) (July 2022)

**REFERENCES CITED IN THE DESCRIPTION**

**Patent documents cited in the description**

- CN 202211633462
- WO 2016040724 A
- WO 2016094873 A
- WO 2021063330 A
- WO 2018158349 A
- WO 2012175400 A
- WO 2016071004 A

**Non-patent literature cited in the description**

- **KABAT et al.** Sequences of Proteins of Immunological Interest. National Institutes of Health (U.S.), 1991
- Chothia scheme based on the location of the structural loop regions. *J Mol Biol*, 1997, vol. 273, 927-48